(19) Europäisches Patentamt European Patent Office Office européen des brevets

(11) **EP 1 577 823 B1**

(12) **EUROPEAN PATENT SPECIFICATION**

(45) Date of publication and mention of the grant of the patent:
**21.01.2009 Bulletin 2009/04**

(51) Int Cl.:
***G06F 19/00*** *(2006.01)*          ***G01N 33/68*** *(2006.01)*

(21) Application number: **04290716.2**

(22) Date of filing: **16.03.2004**

(54) **A method for identifying unknown proteins  by a  two-dimensional separation step and the use of markers**

Methode zur Identifizierung von unbekannten Proteinen mittels zwei-dimensionaler Auftrennung  und Verwendung von Markern

Méthode pour identifier des protéines inconnues  par une séparation bi-dimensionelle e l'utilisation de marqueurs

(84) Designated Contracting States:
**AT BE BG CH CY CZ DE DK EE ES FI FR GB GR HU IE IT LI LU MC NL PL PT RO SE SI SK TR**

(43) Date of publication of application:
**21.09.2005   Bulletin 2005/38**

(73) Proprietor: **CENTRE NATIONAL DE LA RECHERCHE SCIENTIFIQUE (CNRS) 75794 Paris Cedex 16 (FR)**

(72) Inventors:
• **Lamb, Ned Jeremy Charles 34820 Assas (FR)**
• **Fernandez, Anne Claude 34820 Assas (FR)**

(74) Representative: **Michelet, Alain Cabinet Harlé et Phélip 7, rue de Madrid 75008 Paris (FR)**

(56) References cited:
WO-A-01/07920          WO-A-02/077016
US-A- 5 073 963

• **KIM JEONGKWON ET AL: "Electrophoretic mobility for peptides with post-translational modifications in capillary electrophoresis." ELECTROPHORESIS, vol. 24, no. 5, March 2003 (2003-03), pages 782-793, XP002296770 ISSN: 0173-0835**
• **WAGNER K ET AL: "Protein mapping by two-dimensional high performance liquid chromatography" JOURNAL OF CHROMATOGRAPHY A, vol. 893, no. 2, 6 October 2000 (2000-10-06), pages 293-305, XP002296771 ISSN: 0021-9673**

**Description**

## FIELD OF THE INVENTION

The present invention relates to a method for identifying unknown proteins which are contained in a sample to be analysed. This method is based on the characterisation of the experimental migrating properties of a set of peptide fragments which are generated from the unknown proteins, and subsequently on a comparison of these characterised migrating properties with the expected migrating properties of known proteins which would be subjected to the same experimental conditions.

In addition to characterizing unknown proteins consisting of proteins of an unknown sequence, the method of the invention also enables the characterization of the occurrence of chemical modifications undergone by one or several amino acid residues contained in the protein under analysis, such as post-translational modifications like phosphorylation, whether this protein under analysis is of a known or an unknown sequence.

## BACKGROUND OF THE INVENTION

Recent advances in genetics, including the sequencing of the human and mouse genomes, have given rise to the possibilities for rapidly identifying the entire protein content of cells involved in all living processes. These advances have highlighted the need for the identification of proteins, more particularly the state of post-translational modification of a protein which can change dynamically, and thus dramatically alter protein function.

Strategies for target-driven drug discovery and rational drug design require the identification of key cellular proteins that are causally related to disease processes and the use of such proteins as targets for therapeutic intervention. However, present methods for protein identification, in particular the identification of post-translational modifications of proteins, are both expensive and time-consuming and additionally suffer from deficiencies in detection and analysis.

Although the genomic approach has advanced the general understanding of the genetic basis of biological processes, it has significant limitations. Firstly, the functions of the protein products encoded by genes, in particular partial cDNA sequences, are frequently unknown. Secondly, little information about the post-translational modifications of proteins can be deduced from the knowledge of the primary DNA sequence. It is now clear that a considerable proportion of proteins undergo changes in post-translational modification (including phosphorylation, glycosylation, acetylation, myristilation and methylation) that can significantly influence their biological properties. Thirdly, protein expression is frequently subject to post-translational control such that the cellular levels of mRNA do not correlate with the cellular content of the expression product. Fourthly, whereas the genetic content of an individual is identical in each cell type in an individual (with the exception of the gonads), the protein content and post-translational state varies dynamically in a manner which is directly related to perturbations of clinical significance.

The currently known two major methods for determining the identity of an unknown protein rely on, respectively, chemical degradation and mass-spectrometry.

According to the first method above, known as a modified version of the Edman degradation method which is limited by the requirement for pico-molar amounts of peptides, two computer programs are used to identify unknown proteins : FastA and FastXY. These programs are freely available and are simply sequence comparison programs. FastA searches databases of known protein sequences for peptides, which align with the unknown sequence. FastXY is a derivative from FastA in which the only significant difference lies in the input sequence. With FastA, the sequence of letters is assumed to be a single string of letters. With FastXY, groups of letters representing the amino acids cleaved in each cycle are separated such that amino acids in cycle 1 are separated from those from cycle 2 etc. The search program scans known peptides for subsequently reconstructing every peptide combination.

The second method, mass spectrometry, involves the analysis of peptides after enzymatic digestion of unknown proteins. Two types of analysis are currently used. According to the first type of analysis, a database of known protein sequences has been constructed which contains the potential mass spectrometry profiles calculated empirically. The amino acid composition of each peptide is known and profiles are compared against this database. According to the second type of analysis, peptides are physically sequenced by mass spectrometry and searched against the public databases by either FastA or Blastp. Methods for implementing the various steps required for performing mass spectrometry analysis of unknown proteins, including steps of detection of protein spots in a two-dimensional array, are notably described in the PCT applications n° WO 01/55721, WO 01/07920 and n° WO 98/23950 as well as in the United States patent n° US-6,064754 and the United States patent application n° 2002/0098595.

Such a program for peptide sequencing is the mass spectrometry program peptide search (cf www.ebi.ac.uk) owned by Protana Inc (www.protana.com). it compares empirical peptide profiles to those observed.

However, methods that include a step of sequencing peptides of interest by mass spectrometry, at a nano-molar scale, still require important technical and cost-effective means. Moreover, such methods have a low resolution in the femto-molar range.

One of the key difficulties in obtaining an acceptable resolution of the prior art methods, when using starting protein samples containing protein concentrations of less than one nanomole/L, is the numerous artefacts caused by the working environment and cross contamination. The purification and visualization of peptides the atto-molar scale require a logarithmic increase in detector sensitivity level, which is not attainable today.

Also, prior art methods that include a step of analysing the pattern of protein spots generated from a protein-containing biological sample in a two-dimensional electrophoresis are already known. Some of these methods include steps wherein two experimental protein spot patterns can be compared, such as disclosed in the PCT application n° WO 01/07920.

Methods for automatically matching two-dimensional spot patterns which are usable indifferently in the fields of medicine, astronomy, chemistry and biology are also known in the art, for example from the disclosure of the United States patent n° US-5,073,963.

However, these methods are at most useful to detect protein spot changes, i.e. protein expression changes, between two or more protein patterns, without simultaneously providing any technical means which would allow an identification or a characterization of the proteins that are shown to be differentially expressed.

There is a need in the art to supplement genomic data by analyzing patterns of protein expression, particularly the state of protein post-translational modification in biological processes and in relation to disease through the direct analysis of protein expression and post-translational modification.

More specifically, there is a need in the art for methods which would allow the characterisation or the identification of proteins contained in a biological sample, e.g. proteins contained in a protein cell extract, which methods should preferably be both rapid, accurate and sensitive and which preferably should not require the use of highly expensive peptide analysis devices for the final protein identification, such as mass-spectrometry devices.

## SUMMARY OF THE INVENTION

The present invention now provides a new method for identification of unknown proteins in an experimental sample. The method is based on a comparison between the corrected mobility values characterising a specific set of peptides generated from each of these unknown proteins and theoretical mobility values of peptides from known proteins, so as to assign the identity of each of the unknown proteins contained in the said experimental sample as being the protein for which there is an identity, or at least a close similarity, with the theoretical mobility values of the peptides from a protein already known in the art.

A first object of the invention thus consists of a method for identifying one unknown protein contained in a sample, based on the migrating properties on a substrate of peptides generated from the said unknown protein, which method comprises the steps of:

(a) subjecting the said sample containing the said unknown protein to an enzymatic or a chemical cleavage whereby a pool of protein-specific peptide fragments is obtained;

(b) incorporating in the said pool of protein-specific peptide fragments at least two marker peptide compounds of known amino acid sequence, whereby an assay sample is obtained;

(c) subjecting the said assay sample to a two-dimensional separation step on the surface area of a separation substrate, wherein the said two-dimensional separation step consists of :

(i) a first separation step whereby the protein-specific peptide fragments and the at least two marker compounds migrate along the X-axis on the surface area of a first separation substrate; and
(ii) a second separation step whereby the protein-specific peptide fragments and the said marker compounds migrate along the Y-axis on the surface area of a second separation substrate,

wherein (a) the first and second separation substrates are identical or (b) the first and second separation substrates are distinct, whereby a two-dimensional array of peptide spots is formed from the pool of peptide fragments and the marker compounds which were initially contained in the assay sample;

(d) determining the experimental localisation value for each peptide spot contained in the two-dimensional array, wherein the said experimental localisation value consists of the X and Y co-ordinates for each peptide spot contained in the two-dimensional array, respective to a unique predetermined point of origin in the array;

(e) comparing the experimental localisation value determined at step (d) for each marker compound with a predetermined value for the theoretical localisation value of the said marker compound on the two-dimensional array, and then calculating two correction factors, respectively :

(i) a first correction factor "Ce" which is representative of the distance, along the X-axis of the two-dimensional array, between the experimental and theoretical X co-ordinates determined for the said marker compound; and
(ii) a second correction factor "CrF" which is representative of the distance, along the Y-axis of the two-dimensional array, between the experimental and theoretical Y co-ordinates determined for the said marker compound;

(f) calculating, for each peptide spot corresponding to the peptide fragments generated by the cleavage of the said unknown protein, the corrected X and Y co-ordinates of the said peptide spot on the two-dimensional array by applying to the experimental X and Y co-ordinates of the said peptide spot determined at step (d) the correction factors "Ce" and "CrF" determined at step (e), respectively, whereby a set of corrected localisation values for the set of peptide spots corresponding to the peptide fragments is obtained;

(g) comparing the set of corrected localisation values obtained from the set of peptide spots at the end of step (f), wherein said set of corrected localisation values characterise the unknown protein, with either :

(i) a pre-built ordered collection of sets of theoretical localisation values, wherein each set of theoretical localisation values contained in the said ordered collection characterises the set of peptide fragments which is expected to be generated by subjecting a given protein of a known amino acid sequence to the same enzymatic or chemical cleavage as that experimentally performed on the unknown protein at step (a) of the method; or
(ii) successive sets of theoretical localisation values, wherein each set of theoretical localisation values characterises the set of peptide fragments which is expected to be generated by subjecting a given protein of a known amino acid sequence to the same enzymatic or chemical cleavage as that experimentally performed on the unknown protein at step (a) of the method, and wherein each set of theoretical localisation values is generated immediately before it is compared with the set of corrected localisation values for the set of peptide spots corresponding to the set of peptide fragments which was generated from the unknown protein at step (a);

(h) selecting, among the sets of theoretical localisation values contained in the ordered collection of sets defined at step (g-i), or alternatively the sets of theoretical localisation values defined at step (g-ii), (i) the set(s) which exhibit the closest similarity with-, or (ii) the set(s) which is (are) identical to- the set of corrected localisation values obtained at the end of step (f) for the unknown protein to be identified, whereby the said unknown protein is assigned to consist of the protein(s) corresponding to the said selected set(s).

In a specific embodiment of the method above, the said method allows the characterisation of the occurrence of at least one modified amino acid residue, including a phosphorylated amino acid residue, within a known protein in a sample. In the said specific embodiment of the method, step (g) is performed using, in each set of theoretical values corresponding to a given protein, more than one theoretical value for a given peptide contained in the said protein (i.e. protein-specific peptide), respectively (i) one theoretical localisation value that identifies the said peptide bearing no chemical modification and (ii) at least one theoretical localisation value for the said given peptide bearing a given chemical modification, such as phosphorylation of one or several of the amino acid residues that it contains. According to this specific embodiment, the identification method above consists of performing steps (a) to (f) of the method above, starting from a sample containing the unknown protein, and wherein, in step (g), the set of corrected localisation values that characterises the set of peptide spots generated from the said unknown protein is compared with the theoretical localisation values of the expected set of peptide spots that should be generated from the known protein when no amino acid, or alternatively when one or several amino acid residues contained in its sequence is (are) chemically modified, including by the presence of a radioactive isotope, a fluorescent molecule or a post-translational modification, including phosphorylation.

Thus, the method above allows the identification of one or several amino acid residues contained in an unknown protein whose amino acids have undergone any modification(s), including post-translational modification(s). In one specific embodiment, peptides are generated, starting from a protein which has previously been modified *in vitro* or *in vivo* in the presence of a radioactive isotope or a fluorescent molecule. Thus, after step (f) of the method above, the theoretical localisation values of the modified peptide(s) are compared to those of the modified peptides obtained from the unknown protein after correcting for their change in X-axis and/or Y-axis mobility(ties) that result from such modification (s).

A further object of the invention consists of a computer memory support which is readable by a computer apparatus, wherein the said computer memory support contains digital information consisting of a computer software and wherein the said computer software is loadable in the memory of the said computer apparatus for performing steps e) to h) of the method above.

The present invention also relates to a computer program containing the instructions necessary for performing steps e) to h) of the method, as well as to a computer apparatus, the memory of which is loaded with the said computer program.

## DESCRIPTION OF THE FIGURES

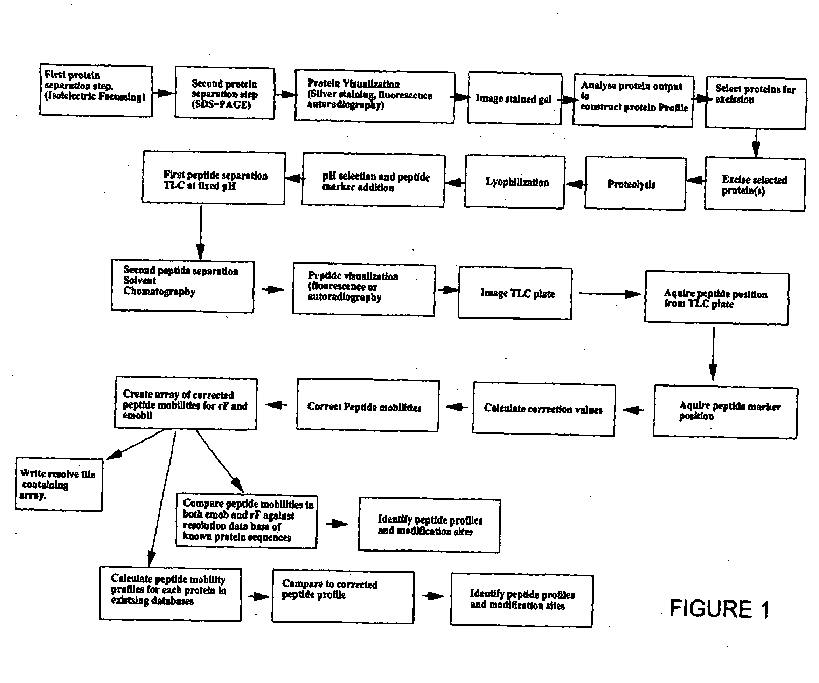

**Figure 1** illustrates a flow-chart of a general embodiment of the protein identification method of the invention. Each box corresponds to a step of the method. The method shown in Figure 1 comprises steps that are not specifically included in the said method, while these additional steps may be part of the method, namely the steps from the first step ("first protein separation step") to the seventh step ("excise selected protein(s)"). These initial seven steps may nevertheless be performed as method steps prior to performing the protein identification method of the invention, which starts with step (a) of "proteolysis" and ends at the end-step of the flow-chart of Figure1. In Figure 1, step (a) of the method according to the invention consists of the step of "proteolysis", whereas step (h) consists of the step "Identify peptide profiles and modification sites".

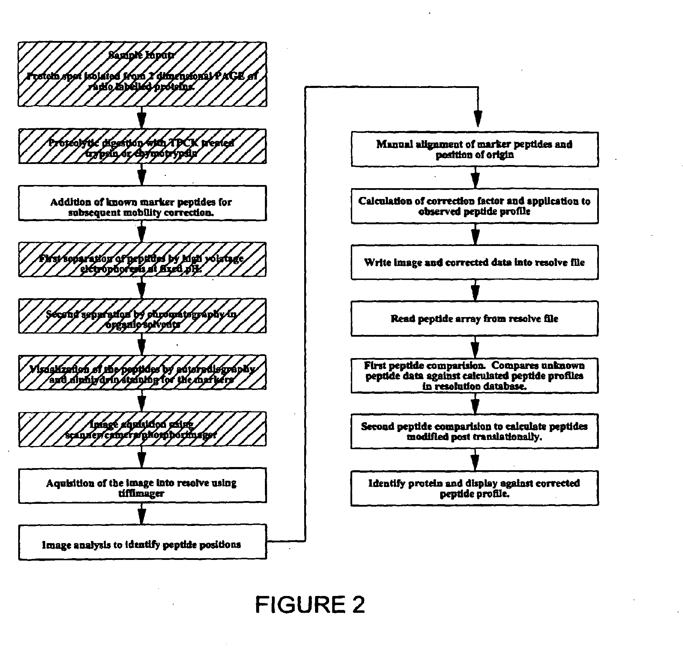

**Figure 2** illustrates another flow-chart describing the protein identification method of the invention, from step (a) ("proteolytic digestion") to step (h) ("identify protein and display against corrected peptide profile"). Hashed boxes designate steps of the method according to the invention that may be already known *per se* in prior art techniques.

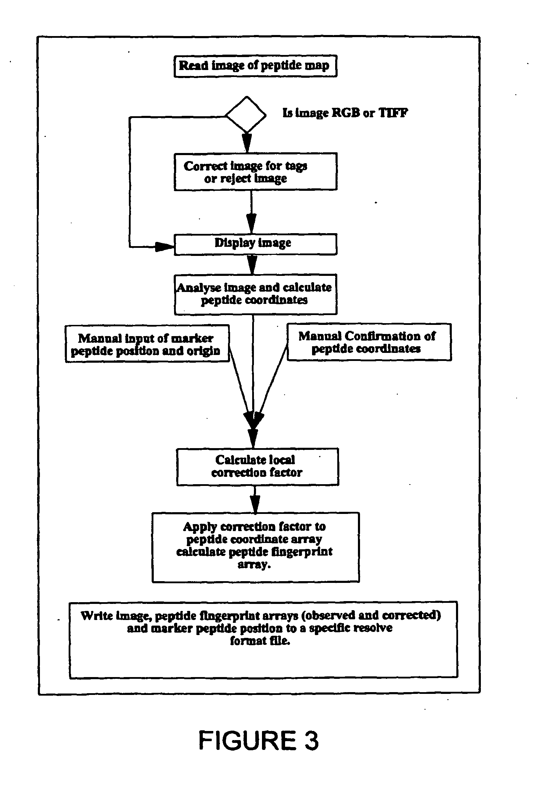

**Figure 3** illustrates a Flow-chart of the process module named "Resolve".
More precisely, figure 3 illustrates a specific embodiment of carrying out steps (d) to (f) of the method according to the invention, wherein this method is implemented through an automated process.
Essentially an image is read into a device so that it can be viewed.
In step (d), the image is processed to determine the position in X and Y directions (axis) of each peptide spot detectable on the image. The position of the peptides used to standardize the image are then translated onto the image by superimposition of the image of the peptide profile with the image having the marker peptide.
In step (e), the position of these marker peptides is then either calculated automatically or manually determine by a user. Based on their observed position and their known mobilities in X and Y directions, a correction factor is calculated from these known peptides.
In step (f), the correction factors (for emobil and for rF) are then applied to the observed distances migrated by each peptide. The corrected mobilities for each peptide are stored for use either for identifying the unknown protein or for identifying the chemical modification of one or more peptides derived thereof.

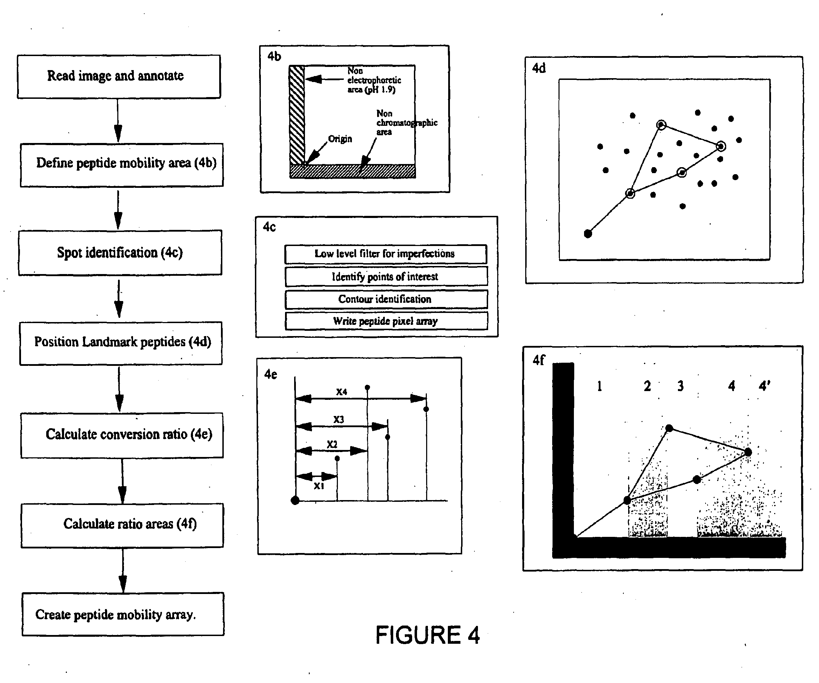

**Figure 4** illustrates another general flow-chart of steps (d) to (f) of the protein identification method of the invention on the left side of the figure (Figure 4a) with specific details given for five steps of the method, respectively :

- "Define peptide mobility array" (Figure 4b). Figure 4b shows the area in which the calculations are performed;
- Spot identification" (Figure 4c). Figure 4c shows an example of the automated process for image analysis;
- "Position Landmark peptides" (Figure 4d). Figure 4d shows the placement on the "landing lights";
- "Calculate conversion ratios" (Figure 4e). Figure 4e shows the calculation of the average distance (here in pixels) to migration ratio in the X axis, the same being also calculated for the Y axis (not shown); and
- "Calculate ratio areas" (Figure 4f). Figure 4f shows a typical example of the correction ratios for the X-axis separation step (here, an electrophoretic step). In each of the areas (grey or white) a peptide found in this area will have its mobility (in X and Y) corrected by a ratio corresponding to the difference observed between the peptide (landmark) mobility observed and that calculated for the theoretical value for this peptide (or other landmark).

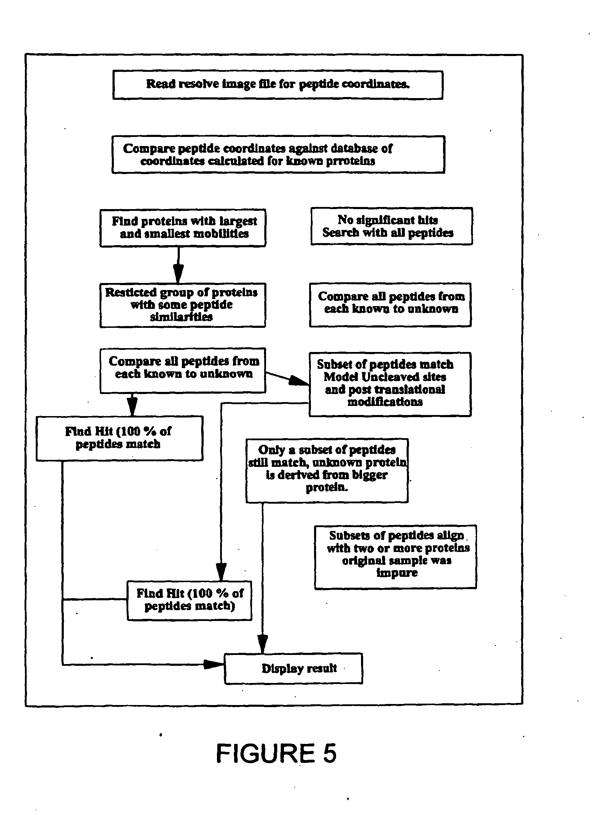

**Figure 5** illustrates a Flow-chart of the process module named "Resolution".
Figure 5 illustrates a specific embodiment of carrying out steps (g) and (h) of the method according to the invention, wherein this method is implemented through an automated process.
An unknown protein can be identified by comparing (i) the peptide coordinates obtained after 2D migration and corrected for their mobility against peptides of known mobility against (ii) a database of peptide mobilities calculated for known proteins.
One embodiment of this method is outlined in Figure 5. The database of proteins consists of one or more peptide sequences.
In the fastest implementation, the extreme coordinates of the unknown protein (largest emobil, largest rF; smallest emobil, largest rF; largest emobil smallest rF; and smallest rF and smallest emobil) are used in the comparison process. Proteins with comparable mobilities are retained as a restricted group. When all sequences in the database have been searched, for each protein in the restricted group, the complete peptide mobility profile is compared to that of the unknown protein.

For those peptides which do not migrate as predicted, the mobility is calculated taking into consideration cleavage failures and phosphorylation events.

Proteins are considered to be the same if greater than 80% of peptides have a mobility similar to that of peptides from the unknown protein.

As an alternative, the complete peptide mobility profile is compared to that calculated for each protein in the database. Proteins displaying the highest level of similarity are selected and as described above for peptides which show aberrant mobility, mobilities are calculated for peptides with cleavage failures or phosphorylation events.

This method can also be used to determine the site of one or more phosphorylation events by comparing the corrected peptide mobility profile against that obtained from the sequence of the known protein. In this case the mobility profile may be only a single or double spots corresponding to radioactively labeled peptides.

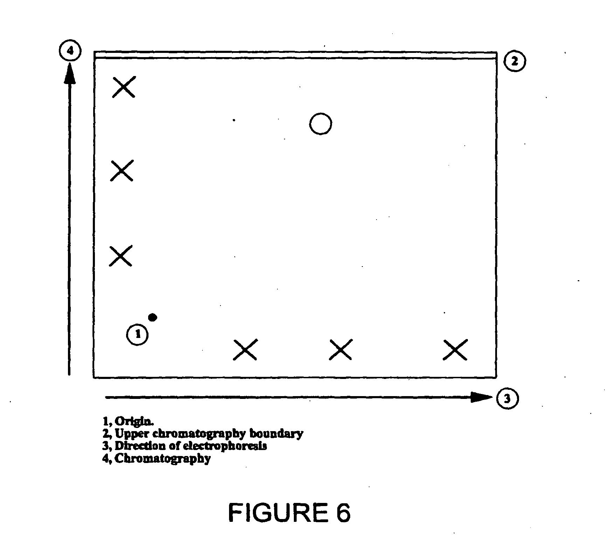

**Figure 6** illustrates a specific embodiment of the method according to the invention, wherein, at step (d) of the method, when calculating the experimental localisation values for each peptide spot contained in the two-dimensional array, the peptide spots formed by the marker peptides are aligned with the peptide spots formed by the protein-specific peptide fragments which are generated at step (a) of the method.

In order to align the marker peptides with the peptides from the sample, the TLC (Thin Layer Chromatography) plate is directly marked with ink containing a radioactive marker.

The example given here shows the type of marking made on the TLC plate when the sample contains radioactive peptides and non-radioactive markers. In this example, the TLC plate is marked with ink to show (i) the origin where the sample is deposited, (ii) the upper line on the chromatographic phase, and (iii) two or more distinct forms outside the area where the peptides resolve.

After staining with ninhydrin to reveal the position of the marker peptides the TLC plate is photographed and then exposed to film to reveal the position of the peptides. Subsequently the two images can be superimposed using the geometric forms for alignment.

In Figure 6, "1" denotes the Origin; "2" denotes the upper chromatography boundary; "3" denotes the direction of electrophoresis and (4) denotes the direction of "chromatography".

**Figure 7** illustrates the calculation of the similarity score between one peptide spot derived from the unknown protein (protein-specific peptide fragment) and a peptide derived from a known protein referenced in a database, which peptide being generated after subjecting the known protein from the database with the same protein cleavage as that performed at step (a) of the method for the unknown protein.

The similarity between the observed corrected mobility of a peptide and that calculated from a known protein sequence is determined by calculating an alignment score.

The alignment score is defined as « 1/distance » where the « distance » is the square root of the sum of the squared differences in mobility between the observed peptide and the calculated peptide in both dimensions.

As such the square of the observed rF for the peptide (y0) subtracted from the predicted rF (y) added to the square of the predicted Emobil value after substraction of the observed Emobil value gives the square of the distance.

After correcting the mobilities of peptides to compensate for discrepancies in mobility, the calculated peptide mobilities of a known protein are compared to those observed after calibration.

Subsequently, the similarities are compared by calculating the distance ratio.

In the present example the mobilities of the observed peptide (grey circle) are compared to those of the other peptide (black and open circles). The spot with the most similarity (black circle) has clearly the closest ratio (represented as distance).

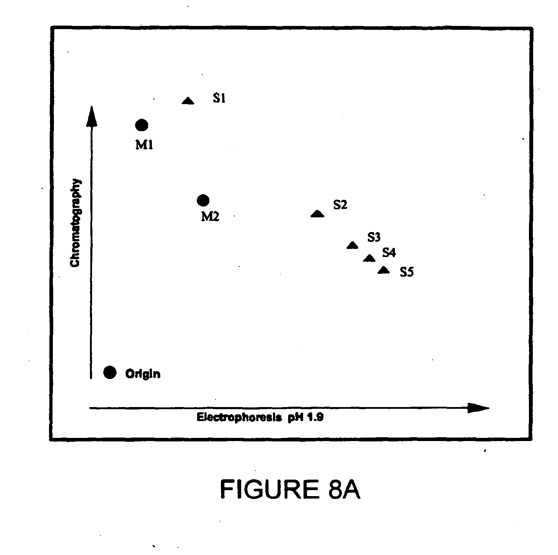

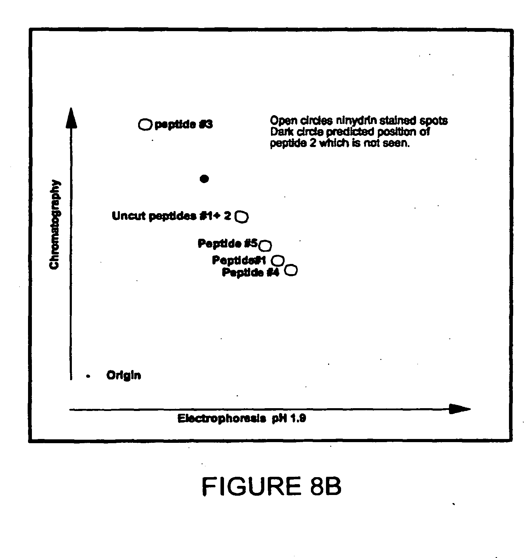

**Figure 8** illustrates the identification of the peptide products for a known peptide sequence after digestion with trypsin.

A short peptide sequence corresponding to the ATP binding site of the human cyclin dependent kinase 1 (cdk1) was synthesized in vitro.

After HPLC purification, the peptide was digested with trypsin. The protein-specific peptide fragments generated through the proteolysis were separated (i) by electrophoresis [X-Axis] and then (ii) by chromatography [Y-axis], in the presence of 2 peptide markers of known sequence.

Peptides were revealed by ninhydrin staining.

Figure **8A** shows the position of the five peptide spots consisting of the five protein-specific peptides (S1 to S5) generated by the protein cleavage, as well as the position of the two marker peptides (M 1 and M2), after the two-dimensional separation.

Figure **8B** shows the image of the TLC plate, the position of the peptides identified on this plate and the identification of the peptides by the method described here. The various parameters that characterise each of the peptides of Figure 8B are found in Table 5.

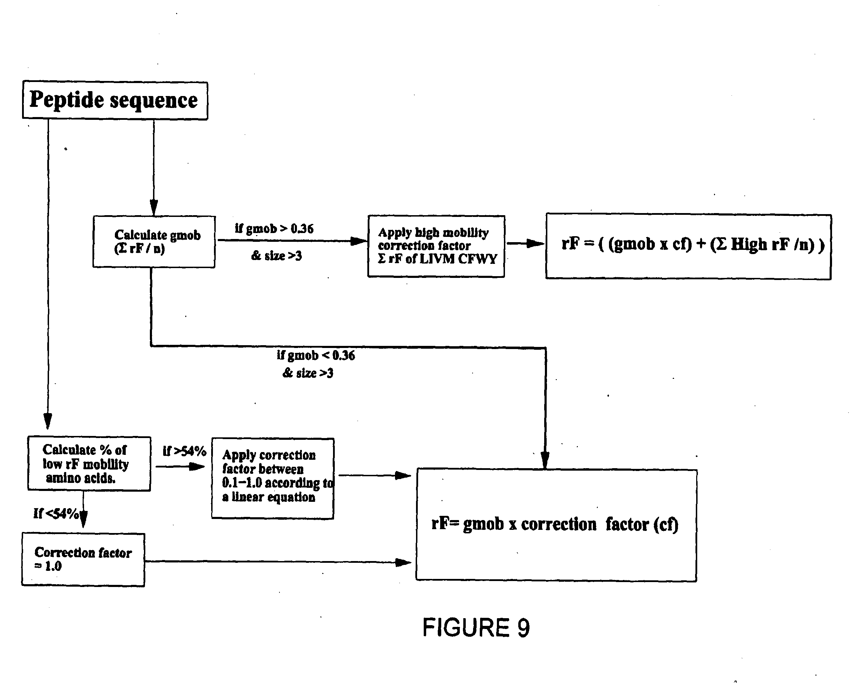

Figure **9** illustrates a schematic diagram showing the decision making process used to calculate the relative chromatographic mobility on the basis of the aminoacid composition of a peptide.

The estimation of chromatographic mobility is calculated on the basis of the aminoacid composition and is in most cases not equivalent to the mean of the sum of the individual amino acid mobilities for the amino acids making up a peptide. In order to compensate for a reduction in mobility when peptides contain low rF amino acids and increased mobility when peptides contain methionine and cysteine, tyrosine, phenylalanine or tryptophan the composition of each peptide is determined and the final mobility determined according to the method outlined in figure 9.

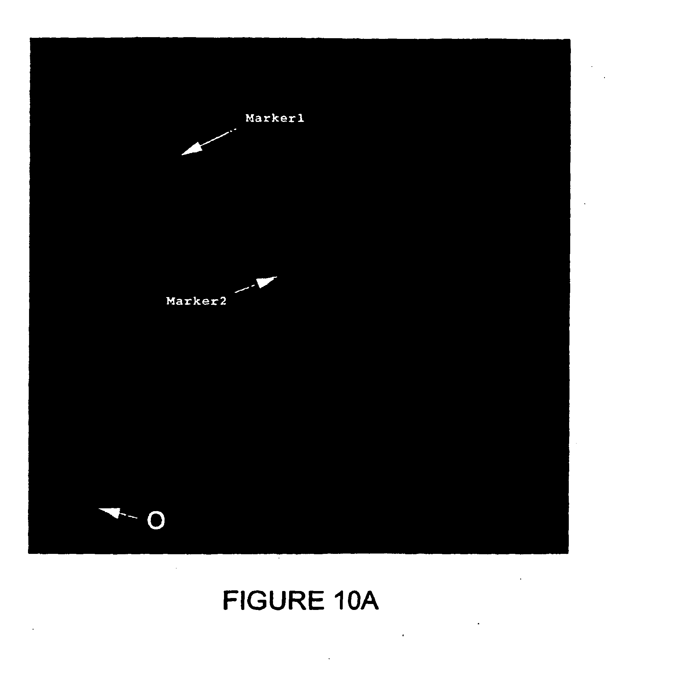

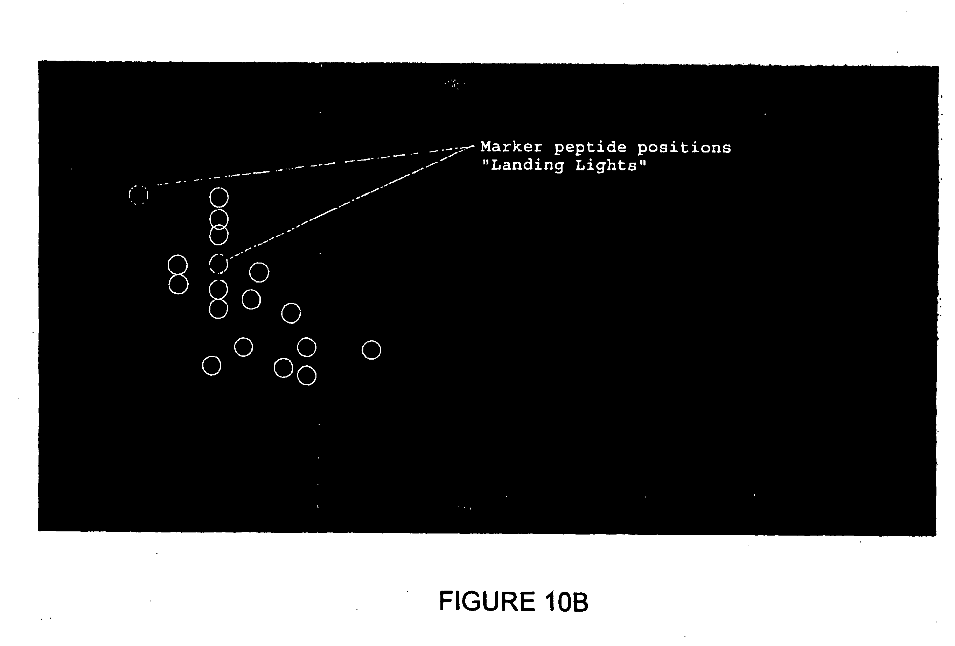

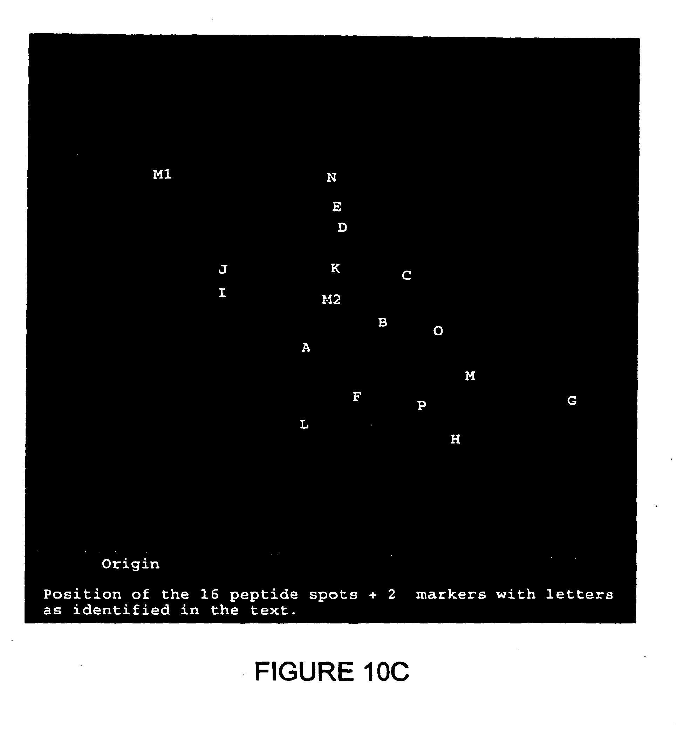

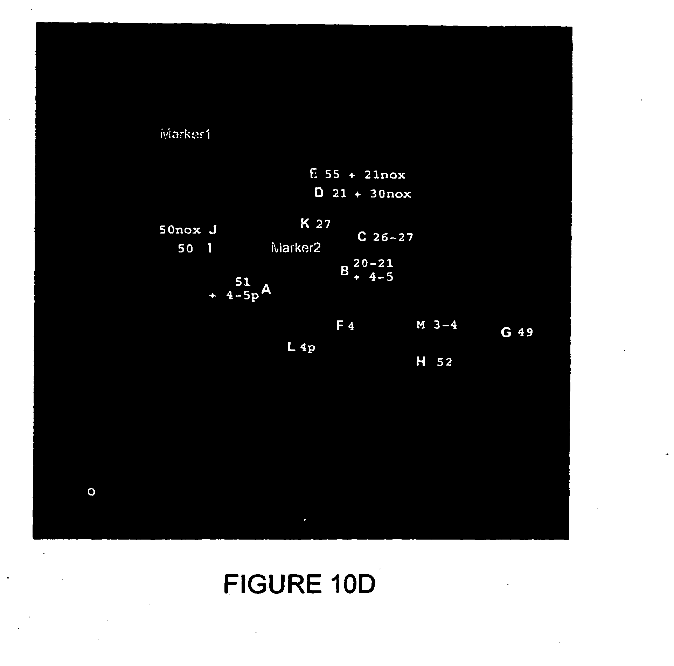

Figure **10** illustrates the Identification of an unknown protein in this example vimentin, by comparison of the peptide profile obtained for methionine labeled vimentin against the Genbank primate database protein database.

Primary human fibroblasts (HS68) were grown to 50% confluence and labeled overnight with [$^{35}$S] methionine.

After extraction, proteins were separated by 2 dimensional PAGE and the band corresponding to vimentin excised under sterile conditions.

After digestion with trypsin for 36 hours, vimentin peptides were separated (i) by electrophoresis and then (ii) by chromatography on TLC plates.

After marking with radiolabeled ink, plates were exposed on film for 24 hours to obtain an image of the peptides.

Plates were then stained with ninhydrin to reveal the marker peptides.

Images were merged and calibrated to obtain the peptide mobility profile.

The profile was then compared against the calculated peptide profiles.

Figure **10A** shows the digitised image obtained after merging (i) the autoradiograph image of the [$^{35}$S] methionine labeled vimentin-specific peptide fragments after the two-dimensional separation and (ii) the image of the ninhydrin-stained marker peptides after the same two-dimensional separation. The two marker peptide spots are indicated by arrows. The open circle at the bottom of the figure indicates the position of the « origin » of the separation substrate, here the TLC plate.

Figure **10B** shows the digitised image of the coordinates of each of the 16 vimentin-specific peptide spots and marker peptide spots. The coordinates of the positions of the vimentin-specific peptides on the original autoradiograph are subsequently corrected with respect to the correction values calculated by comparing the expected and the actual mobility of the marker peptides. Then, the corrected coordinates of the vimentin-specific peptide spots are displayed, here in black circled squares.

The coordinates of the two marker peptides (also termed « landing lights ») are indicated on Figure 10B).

Figure **10C** shows the calibrated digitised image after spot detection. The 16 vimentin-specific peptide spots are designated « A » to « P », respectively. The two marker peptides are designated « M1 » and « M2 », respectively. The most minor peptide spots on the autoradiogram are named N, O and P, respectively. These three minor peptides are mentioned as "not identified" or attributed "in the legend of figure 10D.

After correcting the X and Y mobilities for each peptide the ends and of where compared to those calculed for vimentin. Figure **10D** shows the final output image after identification of the identity of the vimentin-specific peptide spots, at steps (g) and (h) of the method according to the invention. The numerical results corresponding to Figure 10D are shown in Table 6. On Figure 10D and in Table 8, the numbers represent the peptide which have been identified with respect to their position within the amino acid sequence of human vimentin. There are 9 methionine-containing peptides in human vimentin and all 9 peptides are identified. Interestingly, the more strongly labeled spots correspond to more than one peptide, i.e. peptides that migrate closely on the separation substrate. « nox » denotes peptides with non-oxidised methionine ; « p » denotes a phosphorylated peptide ; « 4-5 » denotes non cut peptide containing peptide 4 and peptide 5.

Shown are the corrected peptide profile (Fig 10B), the calibrated image after spot detection (Fig 10C), and the final output image after identification (Fig 10D).

## DETAILED DESCRIPTION OF THE INVENTION

It has been found according to the invention that the mobility values of the peptides, when peptides are generated from a given unknown protein on a two-dimensional array, once accurately determined, could be used to characterise univocally said unknown protein.

Similarly, it has been found according to the invention that the occurrence of chemical modifications, such as post-translational modifications, of one or several amino acid residues in a known protein of interest that is contained in a sample can be detected by comparing (i) the experimental mobility values of a set of peptides generated from the protein of interest contained in the sample with (ii) (a) the expected mobility values of the same set of peptides when these peptides have not undergone any chemical modification and/or (b) the expected mobility values when these peptides have undergone at least one chemical modification, such as a post-translational modification, for example, the phosphorylation or the oxidation of one or several amino acid residues contained therein.

It has also been found according to the invention that a given protein of a known amino acid sequence can be characterised by the calculated theoretical mobility values of the various peptides, also termed "set of peptides", that may be generated by subjecting the said known protein to a specific enzymatic or a chemical cleavage.

It has also been found according to the invention that the experimental mobility values of an unknown protein, the identification of which protein is sought, could be mathematically corrected through a correction factor determined from the comparison between the experimental and the theoretically expected mobility values of at least two marker compounds which are mixed in the same assay sample that also contains the peptides generated from the said unknown protein.

It has also been found according to the invention that comparing the set of mathematically corrected mobility values, corresponding to the set of protein-specific peptides, with each set of theoretical mobility values determined for each protein of a serial (or collection) of proteins of known amino acid sequence, and then selecting the set of theoretical mobility values which is identical to, or which at least possesses the closest similarity with-, the set of mathematically corrected mobility values calculated for the unknown protein, allows to assign, to the unknown protein, the identity of a known protein having the identical or the closest set of theoretical mobility values.

Thus, a first object of the invention consists of a method for identifying one unknown protein contained in a sample, based on the migrating properties on a substrate of peptides generated from the said unknown protein, wherein the said method comprises the steps of :

(a) subjecting the said sample containing the said unknown protein to an enzymatic or a chemical cleavage, whereby a pool of protein-specific peptide fragments is obtained;

(b) incorporating in the said pool of protein-specific peptide fragments at least two marker peptide compounds of known amino acid sequence, whereby an assay sample is obtained;

(c) subjecting the said assay sample to a two-dimensional separation step on the surface area of a separation substrate, wherein the said two-dimensional separation step consists of :

(i) a first separation step whereby the protein-specific peptide fragments and the at least two marker compounds migrate along the X-axis on the surface area of a first separation substrate; and
(ii) a second separation step whereby the protein-specific peptide fragments and the said marker compounds migrate along the Y-axis on the surface area of a second separation substrate,

wherein (1) the first and second separation substrates are identical or (2) the first and second separation substrates are distinct, whereby a two-dimensional array of protein spots is formed from the pool of peptide fragments and the marker compounds which were initially contained in the assay sample;

(d) determining the experimental localisation value for each peptide spot contained in the two-dimensional array, wherein the said experimental localisation value consists of the X and Y co-ordinates for each peptide spot contained in the two-dimensional array, respective to a unique predetermined point of origin in the array;

(e) comparing the experimental localisation value determined at step (d) for each marker compound with a predetermined value for the theoretical localisation value of the said marker compound on the two-dimensional array, and then calculating two correction factors, respectively :

(i) a first correction factor "Ce" which is representative of the distance, along the X-axis of the two-dimensional array, between the experimental and theoretical X co-ordinates determined for the said marker compound; and
(ii) a second correction factor "CrF" which is representative of the distance, along the Y-axis of the two-dimensional array, between the experimental and theoretical Y co-ordinates determined for said marker compound;

(f) calculating, for each peptide spot corresponding to the peptide fragments generated by the cleavage of the said unknown protein, the corrected X and Y co-ordinates of said peptide spot on the two-dimensional array by applying to the experimental X and Y co-ordinates of the said peptide spot determined at step (d) the correction factors "Ce" and "CrF" determined at step (e), respectively, whereby a set of corrected localisation values for the set of peptide spots corresponding to the peptide fragments is obtained;

(g) comparing the set of corrected localisation values obtained from the set of peptide spots at the end of step (f), wherein said set of corrected localisation values characterise the unknown protein, with either :

(i) a pre-built ordered collection of sets of theoretical localisation values, wherein each set of theoretical localisation values contained in the said ordered collection characterises the set of peptide fragments which is expected to be generated by subjecting a given protein of a known amino acid sequence to the same enzymatic or chemical cleavage as that experimentally performed on the unknown protein at step (a) of the method; or
(ii) successive sets of theoretical localisation values, wherein each set of theoretical localisation values characterises the set of peptide fragments which is expected to be generated by subjecting a given protein of a known amino acid sequence to the same enzymatic or chemical cleavage as that experimentally performed on the unknown protein at step (a) of the method, and wherein each set of theoretical localisation values is generated immediately before it is compared with the set of corrected localisation values for the set of protein spots corresponding to the set of peptide fragments which was generated from the unknown protein at step a);

(h) selecting, among the sets of theoretical localisation values contained in the ordered collection of sets defined at step (g-i), or alternatively the sets of theoretical localisation values defined at step (g-ii), (i) the set(s) which exhibit the closest similarity with, or (ii) the set(s) which is (are) identical to, the set of corrected localisation values obtained at the end of step (f) for the unknown protein to be identified, whereby the said unknown protein is assigned to consist of the protein(s) corresponding to said selected set(s).

A first technical advantage of the protein identification method above lies in the fact that its implementation does not require a complex and costly protein or peptide-analysis apparatus for the final characterisation of an unknown protein, in contrast to prior state of the art methods of mass-spectrometry analysis of peptides. Furthermore, the avoidance of such spectrometry analysis steps, which are lengthy and technically complex to perform, makes the method of the invention rapid, easy to perform and inexpensive.

A second technical advantage of the protein identification method of the invention is its high sensitivity. This method does not require an extensive handling of the sample to be analysed, nor analysis steps which would require a large amount of the starting protein material, e.g. nanomolar or micromolar amounts of the starting protein material, but requires in contrast only femtomolar or even attomolar amounts of the said starting material. As described above, the protein identification method of the invention includes primary steps wherein exclusively the localisation of peptide spots on a two-dimensional array is performed. These steps only require that a spot is detected, whereby peptide spot detection is successfully carried out with small amounts of protein material when using a sensitive detector device, such as a photometer or a CCD camera, especially when the protein material to be analysed has been previously labelled with a detectable molecule, such as a radioactive, a luminescent or a fluorescent detectable molecule. Indeed, the use, as the starting material, of a labelled protein or labelled peptides, especially radioactively labelled protein or peptides, further reduces the amount of the said starting material that is required for successfully and accurately performing the identification method of the invention.

A further technical advantage of the protein identification method of the invention consists of the possibility to perform steps (d) to (h) of the method automatically, without having actual access to the protein material which is under characterisation, for example by designing a computer program which performs automatically steps (d) to (h) and then displays the final protein identification results, namely the identity of the unknown protein(s) initially contained in the sample that is under analysis.

As it will be explained in detail further in the present specification, the identification method of the invention can be used for identifying one or several amino acid residues of the protein under analysis. For example a protein of a known sequence under analysis, whose amino acid residue(s) has (have) been chemically modified by a post-translational modification that naturally occurs within the cell wherein the said protein has been produced, or by a post-translational modification carried out *in vitro,* such as post-translational modification encompassing the *in vivo* or *in vitro* phosphorylation of one or several amino acid residues of the said protein, including phosphorylation with a radioactive isotope, like $^{32}$[P]. According to this embodiment of the identification method of the invention, the corrected experimental localisation values of the post-translationally modified peptides are compared to the theoretical localisation values calculated for a set of peptides derived from a protein of a known amino acid sequence wherein, respectively, one or several amino acid residues are phosphorylated, when carrying out step (g) of the method above, before performing step (f). As an illustrative example of the said embodiment of the method, only peptides containing a serine/threonine residue or a tyrosine residue are compared in step (g) and the theoretical localisation values of the phosphorylated peptides are used. According to this embodiment, performing the identification method above in this specific embodiment rapidly leads to the identification of the peptide(s) that have undergone the said post-translational modification.

Thus, the invention relates to a new method for the identification of unknown proteins as well as their state of chemical, e.g. post-translational, modification based on a prediction of the mobility of their component peptide fragments on two distinct separation substrates, and, in a preferred embodiment, on a prediction of the electrophoretic and chromatographic mobility of their component peptide fragments.

According to the latter preferred embodiment, this new method comprises steps essentially involving two components, (i) the inclusion on the electrophoretic plate of specific marker peptides (or proteins) which allow compensation in electrophoretic and chromatographic abnormalities and (ii) method steps for the protein identification process, which are preferably performed through the working of a computer program.

Such a computer program performs six functions: (1) it digitizes into computer memory an image of the observed mobilities of the unknown peptides and the constant marker peptides, generally at least three, preferably four peptides, of known absolute mobility; (2) It calculates correction factors for both electrophoretic ($Ce$) and chromatographic ($CrF$) migration for each of the marker peptides with respect to their predicted mobilities and (3) corrects the relative mobilities (emobile and $rF$) of each peptide from the unknown sample with respect to these correction factors to create an array of idealized values; (3) the program then compares the corrected set of emobile and $rF$ values which characterize the unknown protein against a database of known proteins by calculating idealised peptide arrays (set of emobile and $rF$ values corresponding to a set of peptide fragments) derived for each protein in the database; (5) for proteins which exhibit significant similarity to the corrected array for the unknown protein, the program stringently reanalyses the mobility of aberrant peptides to determine if post-translational modification or proteolysis aberrant cleavage(s) could be responsible for the aberrant mobility; thus, function (5) consists of identifying, for a given protein under analysis, one or several peptides generated therefrom that have been post-translationally modified; (6) finally, the program displays an overlapping image of the corrected mobility plot for the unknown proteins and the aligned image of the existing protein. This image includes a number of parameters which may have affected the mobility map including post-translational modification and failures in the initial unknown protein cleavage.

As used herein, the "unknown" protein which is identified according to the method of the invention consists of either (i) a protein of an unknown sequence the characterisation of which is sought, or (ii) a protein of a known sequence for which eventual chemical modification(s), such as post-translational modification(s) is (are) sought, or (iii) a protein of an unknown sequence for which both the amino acid sequence and the eventual chemical modification(s), such as post-translational modification(s) is (are) sought.

The "sample" wherein the unknown protein is included preferably contains the said protein in a rather purified form. Preferably, the said sample contains only the unknown protein as a protein material, or alternatively it contains the unknown protein which may be associated with very minor amounts, lower than 10% the molar amount of the protein of interest, of one or several distinct proteins, in order to avoid experimental artefacts consisting of detectable peptide spots on the two-dimensional array which are not related to the said unknown protein to be identified.

The unknown protein, to be analysed according to the protein identification method of the invention, can originate from the final step of a former protein separation technique. For example, a cell protein extract is firstly separated into the various distinct proteins it contains, through a conventional two-dimensional protein separation technique, whereby a number of single peptide spots, corresponding to the same number of unknown proteins, are generated in a two-dimensional array. Then, one peptide spot is collected from the two-dimensional array, wherein the said peptide spot, which contains an unknown protein, is suspended in a suitable liquid solution. In this specific case, the "sample" containing the unknown protein, which has to be processed according to the protein identification method of the invention, preferably consists of an aliquot fraction of the liquid solution wherein the said unknown protein has been suspended.

Thus, the protein identification method of the invention is particularly useful for characterising and identifying an unknown protein which is initially present under the form of a peptide spot, as a result of the protein separation of a given cell extract, for example of a cell protein extract obtained after having subjected the said cells to specific culture conditions, i.e. in the presence of a biologically active compound. Then peptide spots which are detectable only when the said specific culture conditions are applied, or alternatively peptide spots which are undetectable only when the said specific culture conditions are applied, are collected and subjected as a series of "samples" to the protein identification method of the invention, in order to determine which protein or proteins synthesis is (are) affected by the specific cell culture conditions which were applied.

In the same way, the protein identification method of the invention is able to easily determine which proteins are specifically produced by various types of tissue-specific cells, e.g. fibroblasts, epithelial cells etc.

By "migrating properties", it is intended herein the ability of a set of peptides, which is generated after the cleavage of the parent unknown protein, to shift along the surface area of a substrate, from an initial location wherein it is deposited on the said substrate, to another location of the surface area of the said substrate, the shift of each peptide of the said set of peptides being caused by the physical or chemical properties of each of the peptides, such as its electric charge, its molecular mass, its acid/basic properties, etc. As it will be easily understood further in the specification, the expression "migrating properties" is not used herein only in its strictly conventional technical sense of the shift of a peptide on the surface area of a two-dimensional substrate such as an electrophoresis gel or a chromatographic substrate.

Instead, the expression "migrating properties" encompasses the behaviour of a given peptide on any separation substrate, such as an HPLC chromatography column or any other separation substrate. Indeed, when the "migration properties" of a given peptide are determined, in the method of the invention, on an HPLC separation substrate, the said migrating properties may be expressed as the retention time of the said given peptide on the said HPLC separation substrate.

As used herein, the term "marker compound" refers to a reference compound which serves as a standard reference having a known theoretical mobility value and which is used in the protein identification method of the invention to calculate mobility correction factors, the said correction factors representing the differences in mobility between the expected theoretical mobility of the said compound in the method and the observed experimental mobility of the said compound during the two-dimensional separation step (c). Throughout the present specification, a marker compound may also be termed "landmark compound" or "landing light" or, when the said marker compound consists of a marker peptide or a marker protein, the said marker compound may also be termed "landmark" peptide or protein. Furthermore, according to the invention, the theoretical mobility properties of a given marker compound can be previously assessed as the mean mobility value determined from a series of separation assays of the said marker compound, in the same conditions as those applied during the two-dimensional separation step (c) of the protein identification method of the invention. In another embodiment, the theoretical mobility properties of a marker compound, when this marker compound consists of a protein or of a peptide, can be calculated as it is specified in detail, further in the present specification. Thus, a marker compound to be used according to the protein identification method of the invention is exclusively characterized by its mobility properties during the two-dimensional separation step (c). It follows that a marker compound encompasses any peptide of a known or of a predetermined migrating mobility. In a preferred embodiment, a marker compound consists of a peptide having a length of at least 10 amino acid residues and having a known amino acid sequence, the said marker peptide being used, according to the protein identification method of the invention, as a reference peptide for which the localisation on the surface area of a substrate can be predicted by calculation. There is not a strict upper limit as regards the molecular mass of a marker peptide. However, generally, a marker peptide does not possess a molecular mass greater than 1000 kDa, and even greater than 500 kDa, because high molecular mass marker peptides will poorly migrate on the surface area of a substrate and their experimental location value on the said substrate will provide poor technical information that can be used when performing the protein identification method of the invention. Generally, the lower molecular mass limit of a marker peptide is of at least 3 kDa in order to be informative when performing the protein identification method of the invention. When more than two marker peptides are incorporated at step (b) of the method, it is ensured that the distinct marker peptides possess distinct molecular mass and/or distinct electric charge status, so as to provide useful distinct known reference peptides which are informative when performing the protein identification method of the invention.

As used herein, a "separation step" consists of a step wherein the peptide fragments and the two or more marker compounds which are contained in the assay sample are deposited on a substrate and wherein the said peptide fragments and marker compounds are put in conditions to migrate along one axis among the respective X-axis and Y-axis of the two-dimensional substrate, according to at least one physical or chemical property of the said peptide fragments and marker compounds, such as their molecular mass, their electric charge, their acid/basic properties, etc. It should be understood that the terms "two-dimensional", "X-axis" and "Y-axis" are not herein employed only under their conventional meanings in the context of a protein separation technique. In the identification method of the invention, step (c) consists of a two-dimensional separation step only because, during the first and the second separation steps respectively, the peptides contained in the assay sample are separated according to distinct migrating properties of the said peptides. Thus, the expression "X-axis" materializes the unit scale used for expressing the migrating properties, i.e. the experimental localisation value, of a given peptide on the first separation substrate, whatever the kind of separation substrate it is. Similarly, the expression "Y-axis" materializes the unit scale used for expressing the migrating properties, i.e. the experimental localisation value, of a given peptide on the second separation substrate, whatever kind of separation substrate it is, provided that either (i) the first and the second separation substrates are distinct or (ii) the first and the second separation substrates are identical but the first and second separation steps are performed using distinct separation conditions. An illustrative example of the latter embodiment consists of the use of the same HPLC substrate during the first and the second separation steps, but with distinct pH conditions in each separation step.

As used herein, a "separation substrate" refers to a substrate having a surface area that is used for performing the separation step which is referred to above. According to the invention, a separation substrate encompasses chromatography substrates, including Thin Layer Chromatography (TLC) substrates, suitable substrates for iso-electrofocusing separation, or any other substrate which allows the separation of peptides or proteins according to their physical and/or chemical properties, such as, for example, any chromatography substrate, including HPLC ("High Performance Liquid Chromatography"), FPLC (Fast Protein Liquid Chromatography"), gel filtration chromatography, ion exchange chromatography, reverse phase chromatography or affinity chromatography. In several embodiments, the same separation substrate may be used for the two separation steps, at step (c) of the protein identification method of the invention, wherein, at each of the two separation steps, the protein-specific peptide fragments and the marker compound(s) migrate along respectively the X-axis and the Y-axis of the said separation substrate, according to distinct physical or chemical properties. However, the first and the second separation steps can be performed using two distinct separation substrates, each separation substrate being specifically suited to effect the peptide/protein separation according to distinct physical or chemical properties of the protein-specific peptide fragments and marker peptides. In the latter case, the marker compounds and the peptide fragments which have been subjected to the first separation step may be transferred from the first separation substrate towards the second separation substrate before performing the second separation step. In a further embodiment wherein the first and the second separation steps are performed using two distinct separation substrates, aliquot volumes of the assay sample obtained at the end of step (c) are subjected separately (i) to the first separation step on the first separation substrate and (ii) to the second separation step on the second separation substrate, whereby the whole two-dimensional separation step (c) is performed with two distinct separation substrates without requiring a transfer of the material from the first separation substrate towards the second separation substrate. A specific technical advantage of this latter embodiment is the possibility to perform simultaneously the first and the second separation step, respectively.

As used herein, the expression "protein-specific peptide fragments" refers to the various peptides that are generated after the enzymatic or the chemical cleavage of the protein which is performed at step (a) of the method. A list of the preferred chemical compounds or proteolytic enzyme used at step (a) of the method is given in Table 3.

As used herein, the term "localisation value" refers to the position, of the protein spot area containing most of the protein material, wherein the said spot area is contained in the considered peptide spot located on the surface area of a separation substrate used according to the method of the invention. The localisation value of the said protein or peptide spot is expressed by the combination of the spot location along the X-axis and along the Y-axis of the said separation substrate, respectively. The kind of measure units which are used to express the spot location on the X-axis and the Y-axis are not an essential feature of the method. These units may be expressed as millimeters or centimeters, or even as a retention time in seconds, if an HPLC separation substrate is used. In the case wherein the surface area of the first or of the second separation substrate is scanned so as to generate an electronic image of the spots before determining the localisation value of each protein/peptide spot, the X-axis co-ordinates and the Y-axis co-ordinate of each spot are preferably expressed as pixel co-ordinates, in reference to the number of pixels per unit of surface area of the said separation substrate.

As used herein, the term "localisation value" of a protein/peptide spot also refers to the X- and Y-co-ordinates of the said protein/peptide spot and also to the "mobility" of the said protein/peptide.

As used herein, the term "mobility" of a peptide or a protein refers to the localisation value of the spot containing the said protein or said peptide on the surface area of a separation substrate. Consequently, the mobility value of a peptide strictly equates the localisation value of the said peptide. It will be found, in the present specification, that the X-axis co-ordinate, i.e. the X-axis localisation value, of a specific protein or peptide on the surface area of a separation substrate, may also be termed "e-mobility" of the said protein or peptide. This is in reference notably to a specific embodiment of the protein identification method of the invention, wherein the first separation step consists of an electrofocusing separation step wherein the said protein or peptide is migrating, along the X-axis of the separation substrate, according to its electrophoretic mobility properties. It will also be found, in the present specification, the Y-axis co-ordinate, i.e. the Y-axis localisation value, of a specific protein or peptide on the surface area of a separation substrate, may also be termed "rF-mobility" of the said protein or peptide. This is in reference notably to a specific embodiment of the protein identification method of the invention, wherein the second separation step consists of a chromatography separation step wherein the said protein or peptide is migrating, along the Y-axis of the separation substrate, according to its molecular mass mobility properties.

As used herein, the expression " experimental localisation value" refers to the location value of a protein/peptide spot, on the surface area of a separation substrate, where the localisation value has been experimentally determined by effective location of the said protein/peptide spot which is actually observed on the surface area of the separation substrate.

As used herein, the expression "theoretical localisation value" refers to the location value that a protein/peptide spot should have had on the surface area of a separation substrate in ideal experimental separation conditions. For example, the marker compounds of a known amino acid sequence which are incorporated at step (b) of the method and which are then subjected to the two-dimensional separation step (c), according to controlled separation conditions, should ideally be located at a "theoretical localisation value" on the surface area of the second separation substrate, at the end of step (c). Generally, the experimental localisation value which is actually observed for the spot of a specific marker compound differs significantly from the theoretical localisation value which is expected for the said spot, simply because the experimental separation conditions are very rarely "ideal", beginning by possible physical or chemical heterogeneity on the surface area of the separation substrates which are used.

At step (e) of the method, the correction factors "Ce" and "Crf" to be applied to the experimental localisation values of the protein-specific peptide fragments can be easily determined by anyone skilled in the art, once both the experimental localisation value and the theoretical localisation value of a given marker compound are known. The correction factor "Ce" corresponds to the value of the distance shift, along the X-axis of the separation substrate, between the expected theoretical localisation value of a given marker compound and its observed actual experimental X-axis localisation value on the separation substrate. The thus calculated X-axis shift value is then applied to the X-axis experimental localisation value of each of the protein-specific peptide fragments which were generated by the cleavage of the unknown protein at step (a) of the method, in order to obtain the "corrected" X-axis localisation value for each protein-specific peptide fragment. In the same way, the correction factor "CrF" corresponds to the distance shift, along the Y-axis of the separation substrate, between the expected theoretical localisation value of the same given marker compound and its observed actual experimental Y-axis localisation value on the separation substrate. The thus calculated Y-axis shift value is then also applied to the Y-axis experimental value of each of the protein-specific peptide fragments which were generated by the cleavage of the unknown protein at step (a) of the method, in order to obtain the "corrected" Y-axis localisation value for each protein-specific peptide fragment.

In a most preferred embodiment of the protein identification method of the invention, the correction factors "Ce" and "CrF" are calculated as detailed hereunder.

A specific value of Ce and Crf can be calculated, for each marker compound X1 to Xn, as it is shown hereunder for the marker compound X1 :

(1) For correction factor Ce (X-axis):

(i) calculating the experimental X-axis localisation value of the marker compound X1.

$$EX1 = x1 - xOrigin,$$

wherein:

- EX1 consists of the experimental localisation value of the said marker compound along the X-axis of the said separation substrate, the said experimental localisation value consisting of the distance between the X-axis origin [xOrigin] of the separation substrate and the center [x1] of the marker compound spot on the said separation substrate.

Indeed, the measure units used to express x1 and Origin are the same, irrespective of whether these measure units are centimeters, millimeters, etc.

Preferably, the distance is measured, or otherwise converted, in pixels, by generating an electronic image of the separation substrate onto which the marker compounds have migrated.

(ii) calculating a X-axis Ce ratio.

Once the experimental X-axis localisation value EX1 has been determined, a ratio is calculated between X1 and the predetermined X-axis theoretical localisation value [TX1] for the same marker peptide X1, as it follows :

$$X\text{-AxisRatio } X1 = EX1 / TX1,$$

wherein,

- EX1 is the X-axis experimental localisation value for marker peptide X1 calculated at step (i); and
- TX1 is the X-axis theoretical localisation value for marker peptide X1 which has been predetermined.

The kind of measure units for expressing EX1 and TX1 are not necessarily the same, since only a ratio value is expected, irrespective of the absolute value of the said ratio. However, generally, TX1 is expressed in pixel units.

(2) For correction factor Crf (Y-axis) :

(i) calculating the experimental Y-axis localisation value of the marker compound X1.

$$EY1 = y1 - yOrigin,$$

wherein:

- EY1 consists of the experimental localisation value of the said marker compound along the Y-axis of the said separation substrate, said experimental localisation value consisting of the distance between the Y-axis origin [yOrigin] of the separation substrate and the center [y1] of the marker compound spot on the said separation substrate.

Indeed, the measure units used to express y1 and yOrigin are the same, irrespective of whether these measure units are centimeters, millimeters, etc.
Preferably, the distance is measured, or otherwise converted, in pixels, by generating an electronic image of the separation substrate onto which the marker compounds have migrated.

(ii) calculating a Y-axis Crf ratio.

Once the experimental X-axis localisation value EY1 has been determined, a ratio is calculated between Y1 and the predetermined Y-axis theoretical localisation value [TY1] for the same marker peptide X1, as it follows :

$$\text{Y-AxisRatio } X1 = EY1 / TY1,$$

wherein

- EY1 is the Y-axis experimental localisation value for marker peptide X1 calculated at step (i); and
- TY1 is the X-axis theoretical localisation value for marker peptide X1 which has been predetermined.

The units of measurement for expressing EY1 and TY1 are not necessarily the same, since only a ratio value is, irrespective expected of the absolute value of the said ratio. Generally, TY1 is expressed in pixel units.
As previously mentioned, the Ce ratio and the Crf ratio, respectively, are calculated for each of the marker compounds X1 to Xn, and then compared, every one to every each other. In normal experimental conditions, the Ce values for each marker compound should be identical or substantially identical, this denotes a high homogeneity of the surface area of the substrate used for the first separation step, along the X-axis. Similarly, the Crf values for each marker compound should be identical or substantially identical, this denotes a high homogeneity of the surface area of the substrate used for the second separation step, along the Y-axis. In the case wherein the Ce values for two marker compounds X1 and X2, respectively, significantly differ from each other, for example when the ratio CeX1/CeX2 is lower than 0.8 or is higher than 1.2, then the first separation step (c)(i) of the method is performed again. Similarly, when the Crf values for two marker compounds X1 and X2, respectively, significantly differ from each other, for example when the ratio CrfX1/CrfX2 is lower than 0.8 or is higher than 1.2, then the second separation step (c)(ii) of the method is performed again.
It follows that a single Ce value and a single Crf value should be obtained for all the marker compounds used, to which correction factors are then applied to the experimental localisation values determined for every protein-specific peptide spot, in step (f) of the method. Typically, the corrected localisation values of each of the peptide fragments generated at step (a) are calculated, at step (f), by multiplying the experimental localisation values of the protein-specific peptide fragments which are determined at step (d) by the correction factors Ce and Crf, respectively.
According to a first preferred embodiment of the protein identification method above, the protein cleavage performed at step (a) consists of an enzyme cleavage using an enzyme selected from the group consisting of trypsin, trypsin [lys blocked], chymotrypsin, clostipain, proline endopeptidase, myxobacter protease, armilaria protease, staphylococcal protease, and endoproteinase Asp-N. The specific conditions wherein an enzymatic protein cleavage can be performed are well known to anyone skilled in the art in Hunter et al (1988). A list of the preferred proteolytic agents is given in Table 3.
According to a second preferred embodiment of the method above, the protein cleavage performed at step (a) involves a chemical cleavage using a compound selected from the group consisting of cyanogen bromide, 2-nitro-5-thiocyanobenzoic acid and an acidic compound suitable for acid hydrolysis at pH 2.5 or similar. The specific conditions wherein a chemical protein cleavage can be performed are well known to anyone skilled in the art in Boyle et al. (1991).
In order to increase the sensitivity of the protein identification method of the invention, and especially for enhancing the sensitivity of step (d) wherein it is determined the X and Y co-ordinates of the peptide spots contained in the two-dimensional array corresponding to the marker compounds, the said marker compounds are labelled with a detectable molecule. The role of the detectable molecule consists of amplifying a detectable signal provided by every marker compound spot included in the array, which could otherwise be provided by a conventional protein staining, especially when the said marker compounds consist of peptide markers.

Thus, in a further preferred embodiment of the protein identification method of the invention, the marker compounds which are used are labelled with a detectable molecule.

Also, in order to increase the sensitivity of the protein identification method of the invention, and especially for enhancing the sensitivity of step (d) wherein it is determined the X and Y co-ordinates of the peptide spot(s) contained in the two-dimensional array corresponding to the unknown protein-specific peptides, the said unknown protein, or the peptide fragments derived thereof by either an enzymatic or a chemical cleavage, are labelled with a detectable molecule. The role of the detectable molecule consists of amplifying a detectable signal provided by every unknown protein-specific peptide spot included in the array, which could otherwise be provided by conventional protein staining.

Thus, in a preferred embodiment of the method above, the unknown protein, or the peptide fragments derived thereof by an enzyme or a chemical cleavage, is (are) labelled with a detectable molecule.

In another preferred embodiment of the method above, both the marker compounds and the unknown protein, or the peptide fragments derived thereof by an enzyme or a chemical cleavage, are labelled with a detectable molecule.

In order to increase the accuracy and the rapidity of the method, when both the marker compounds and the peptide fragments are labelled, the said marker compounds and peptide fragments are differentially labelled with two distinct detectable molecules, so as to allow direct discrimination at step (d) between the experimental localisation values of respectively the marker compounds and the protein-specific peptide fragments.

In a preferred embodiment of the protein identification method of the invention, at step (c), the first separation step consists of a high voltage flat-bed electrophoresis and the second separation step consists of an ascending Chromatography both performed on a thin layer of cellulose.

In another preferred embodiment of the protein identification method of the invention, at step (c), the first separation step consists of a first HPLC separation step at a first pH value and the second separation step consists of a second HPLC separation step at a second pH value.

In a still further embodiment of the protein identification method of the invention, at step (c), the first separation step consists of a first HPLC separation step on a first HPLC separation substrate, e.g. a molecular sieve substrate, and the second separation step consists of a second HPLC separation step on a second HPLC separation substrate, e.g. an anion-exchange substrate.

According to the preferred embodiment of step (c), most preferably, the chromatographic separation step is performed under denaturing conditions, in order to restrict, as much as possible, chromatographic mobility of the peptides to their respective molecular mass, thus minimizing protein migration heterogeneity which would be caused by the three-dimensional conformations of the said peptides. Most preferably, the sample is denatured by the solvents and acid used for performing the chromatographic separation step. Typically, the buffer solution used for chromatography contains either (i) 10% VN acetonitryle in combination with trichloracetic acid, especially for HPLC, or (ii) a mixture of acetic acid, pyridine and butanol, especially for TLC.

Thus, according to this preferred embodiment of the method, the marker compound(s) and the peptide fragments are firstly separated according to their electrophoretic properties (electrophoretic mobility) and then are subsequently separated according to their chromatographic properties (chromatographic mobility).

As an illustration of said embodiment, electrophoresis can be carried out in the pH range from 1.9 to 9.5, by varying the buffer which is used. At pH 1.9, all peptides migrate from the anode to the cathode because they are all positively charged. At pH 9.5, most peptides are negatively charged and migrate to the anode. The electrophoresis step consists of a non-equilibrium process at pH 1.9, in order to ensure that peptide mixtures reproducibly migrate at the same location of the surface area of the electrophoresis substrate, from one assay to another.

In a further preferred embodiment of the method above, two to ten peptide marker compounds of distinct electrophoretic mobility or chromatographic mobility are incorporated in the pool of protein-specific peptide fragments, at step (b) of the method. It has been observed that, when performing successively the two-dimensional separation step (b), local distortions of peptide migration may occur, which may be due to local physical or chemical heterogeneity of the separation substrate surface area, whereby substrate heterogeneity will cause artefacts or discontinuities in the migration of both the protein-specific peptide fragments and the marker compounds. Such artefacts or discontinuities are most preferably taken into account when calculating the correction factors "Ce" and "CrF" at step (e) of the method. Thus, in rare cases, a multiplicity of correction factors "Ce" and "CrF" are calculated respectively for the different surface areas of the separation substrates used at step (c). Then, for the calculation of the experimental corrected X and Y co-ordinates of each peptide spot of experimental corrected the array corresponding to each protein-specific peptide fragment included therein, only the respective correction factors "Ce" and "CrF" are applied and determined for the specific surface area of the separation substrate in the vicinity of which each peptide spot is localised.

Preferably, at step (a) of the method 2 to 10 marker compounds, more preferably 3 to 7 marker compounds are incorporated, and still more preferably 4 or 5 marker compounds are incorporated, although the method can be successfully and accurately performed with only two marker compounds.

It follows that the accuracy of the calculation of the theoretical X and Y co-ordinates of the peptide spots, corresponding to the protein-specific peptide fragments generated by cleavage of the unknown protein, is increased with an increasing number of marker compounds which are incorporated at step (b), marker compounds will migrate where to various locations, and wherein the whole set of locations covers most of the surface area of the separation substrates. This then allows to compensate for variations in plate manufacture and, for example, for discontinuities in electrophoretic and chromatographic running.

For calculating the predetermined value for the theoretical localisation of each marker compound, the physico-chemical properties of each amino acid included in the amino acid sequence of the said marker compound is taken into account as well as the global amino acid composition of the said marker compound, two parameters which influence the theoretical behaviour ( and indeed also the experimental behaviour) of the said marker compound, respectively during the first electrophoresis separation step and the second chromatographic separation step.

As regards the electrophoretic mobility of any peptide (or protein), said electrophoretic mobility is influenced by the net charge of the said peptide, which is caused by the charged status of the carboxy and amino terminal groups of the said peptide as well as by the charged status of the side chains of the amino acid residues contained therein, essentially the side chains of the amino acid residues lysine, arginine, histidine, tyrosine, glutamic acid, aspartic acid, phospho-serine, tyrosine and threonine. Positive net charges are contributed by lysine, arginine, histidine and the peptide amino terminal groups. Negative net charges are contributed by tyrosine, phospho-tyrosine glutamic acid, aspartic acid, phospho-serine, tyrosine, threonine, phospho-threonine and the carboxy terminal group.

Thus, for calculating the predetermined value for the theoretical localisation for each marker compound which is incorporated at step (a) of the method, the net charge of the said marker compound, for example a marker protein, is determined from its known amino acid sequence. The net charge of the said marker compound is calculated by taking into account the $pK_R$ value which has been assigned to each amino acid residue, wherein said $pK_R$ value consists of the pKa value for the positively charged and the negatively charged amino acids, respectively. The $pK_R$ values for the different amino acids are listed in the appended Table 1.

Furthermore, it has been found according to the invention that there exists a contribution of the peptide mass to the electrophoretic mobility of the said peptide. Thus, an accurate calculation of the electrophoretic mobility of a given peptide cannot be restricted to the determination of the mean net charge of the said peptide, based on its known amino acid sequence. Specifically, it has been found according to the invention that the electrophoretic separation properties of particular peptides follow a non-equilibrium factor at all pH values and, as such, the only useful data which is provided, after having performed the electrophoretic separation step, is the relative spatial distribution of the different peptides with respect to one another. It has thus been found according to the invention that the electrophoretic mobility of a peptide is also influenced by its mass, since there is an element of resistance on free peptide mobility during the electrophoresis separation step, where resistance is conveyed by the separation substrate used for electrophoresis.

According to the invention, it has been determined that the extent to which the molecular mass of a given peptide influences its own electrophoretic mobility is a two-thirds (2/3) function of the molecular mass of the said peptide. More specifically, it has been found that an accurate theoretical X-co-ordinate value (X-axis theoretical localisation value), which is also termed the electrophoretic mobility value, or « Emobil », of a given peptide is most preferably determined according to the following formula: Emobil = netcharge/$Mr^{(2.0/3.0)}$, wherein « netcharge » means the mean net charge value of a said peptide, whereby the mean net charge is calculated from the individual net charge of each of the amino acid residues contained in a said peptide, and wherein Mr consists of the molecular mass of a said peptide.

Indeed, the absolute position of a given peptide on the surface area of the electrophoresis separation substrate, with respect to its electrophoretic mobility, greatly depends on the duration of the electrophoretic time period. However, there is no requirement that this latter parameter be taken into account according to the method of the invention, since what is retained for the calculation of the experimental localisation value of a peptide spot of interest are both (i) the position, on the separation substrate, of a marker protein of a known amino acid sequence, relative to a unique predetermined point of origin in the said separation substrate (or array) and (ii) the relative positions, on the said separation substrate, between the said marker protein and the said given peptide of interest.

Thus, what is important for performing the protein identification method of the invention is that the positional differences between individual peptides initially contained in the assay sample are proportionally the same with respect to the unique point of origin which has been formerly predetermined on the separation substrate (or array), wherein the said positional differences are independent in respect to the time period of the electrophoresis separation step.

As regards the chromatographic mobility of any peptide (or protein), the said chromatographic mobility is influenced by the relative chromatographic mobilities of each of the 25 possible kind of amino acid residues included therein, namely: alanine, arginine, asparagine, aspartic acid, cysteine, cyclic cysteine, glutamine, glutamic acid, Glycine, histidine, isoleucine, leucine, lysine, methionine, oxidised methionine, phenylalanine, proline, serine, phospho-serine, threonine, phospho-threonine, tryptophan, tyrosine, phospho-tyrosine and valine.

For the purpose of performing, with the highest possible accuracy, the protein identification method of the invention, specifically in respect to the calculation of the theoretical Y-axis localisation values of a known peptide, also termed theoretical chromatographic mobility of the said peptide, the calculation of the theoretical chromatographic mobility of any known peptide is performed by using an original algorithm which takes into account the differences in mobility experimentally observed by the inventors between aliphatic, aromatic, hydrophobic and hydrophilic amino acid residues, that may be contained in the amino acid sequence of the said peptide.

Thus, according to the invention, a set of chromatographic « Rf » mobility values have been experimentally determined and a specific « Rf » value has been assigned for each amino acid residue, including a specific « Rf » value for each of the chemical forms of a given amino acid residue, (e.g. phosphorylated or oxidized amino acid residue).

Thus, according to the invention, based on the original experimental data obtained by the inventors, a specific Rf electrophoretic value has been assigned to each of the 25 amino acid residues above. All the whole experimentally determined Rf values are shown in Table 2.

The Rf chromatographic mobility values which are listed in Table 2 have been experimentally determined by the inventors when using, as the chromatography separation substrate, a Thin Layer Chromatography (TLC) plate which is marketed by the Company MERCK TLC plates). The Rf values listed in Table 4 have been obtained using the solvent buffer conventionally termed "Phospho-Chromo" the composition of which is described in example 8.The inventors believe that the same Rf values can also be used to calculate the theoretical chromatographic mobility of a given peptide when the Y-axis chromatographic separation step is performed on another type of thin layer chromatographic substrate. However, for an optimal accuracy of the method, anyone skilled in the art may also, for a given type of chromatography separation substrate, perform a *de novo* calibration and then generate a set of specific Rf values corresponding to the individual amino acids, chromatographic mobilities on the said other type of chromatographic separation substrate, preferably according to the calibration method which is described in detail in the present specification.

In a preferred embodiment of step (e), when the marker compound consists of a marker protein, the predetermined value for the theoretical localisation for each marker protein consists of determining respectively the theoretical X and Y co-ordinates for each marker protein and is calculated as follows :

Theoretical X co-ordinate value :

(i) determine the net charge of the marker protein of a known amino acid sequence as follows :

(i-1) calculate the positive charge of the marker protein according to the following formula (1):

$$netplus = ([H^+] / Kd + [H^+]) + ([H^+] / pK1 + [H^+]) ,$$

wherein Kd is $10^{x(-pKr)}$ and pKr is the pKa for the positively charged amino acids and pK1 is the pKa of the amino-terminal group of the marker protein.

(i-2) calculate the negative charge of the marker protein according to the following formula (2) :

$$netminus = 1-( [H^+] / Kd + [H^+] ) + ( 1- ([H^+] / pK2 + [H^+] ) )$$

wherein Kd is $10^{x(-pKr)}$ and pKr is the pKa for the negatively charged amino acids and pK2 is the pKa of the carboxy-terminal group of the marker protein.

(i-3) calculate the net charge of the marker protein according to the following formula (3) :

$$\text{netcharge} = \text{netplus} - \text{netminus}$$

(ii) calculate the electrophoretic mobility of the marker protein according to the following formula (4) :

$$\text{Emobil} = \text{netcharge} / Mr^{(2.0/3.0)},$$

wherein Mr is the molecular weight of the marker protein expressed in daltons.

Theoretical Y co-ordinate value :

(i) calculate the absolute Y-mobility value of said marker protein according to the following formula (5) :

$$\text{AbsMob} = [rf\text{-}aa_1 + rfaa_2 + \ldots + rfaa_n]/n$$

Wherein $rf\text{-}aa_1$, $rfaa_2$, ..., $rfaa_n$ are the respective Y-axis mobility values for each of the amino acid residues contained in a said marker protein of a known amino acid sequence and wherein « n » is the total number of amino acids contained in a said marker protein ;

(iii) Calculate a corrected value for the absolute Y-mobility value of the said marker protein, whereby the corrected value takes into account

(a) the whereby percentage low mobility amino acids and (b) the number of high mobility amino acids in the sequence of the said marker protein, according to the following formula (6) :

$$\text{Rf} = \text{AbsMob} \cdot \text{CF}$$

Wherein CF is the correction factor applied.
According to the invention, the same calculation as above is performed to calculate the theoretical localisation value of any peptide of a known amino acid sequence which is generated after a cleavage of the amino acid sequence of a known protein by the same enzymatic or chemical cleavage as that which has been initially performed on the unknown protein, at step (a) of the method.
It has been found according to the invention that the accuracy of the method allowing the calculation of the predetermined value for the theoretical localisation, for each marker protein that is specified above, could be further enhanced by also taking into account (i) the marker peptide content in low chromatographic mobility amino acid residues and (ii) the marker peptide content in high chromatographic mobility amino acids.
According to the invention the low chromatographic mobility amino acids are, respectively : R, N, D, c, Q, E, G, H, K, S, s, t and y, wherein the said amino acids are represented according to the International Nomenclature and wherein "c" means an oxidised cysteine residue, "s", "t" and "y" mean a phosphorylated serine, threonine or tyrosine residue, respectively.
According to the invention, the high chromatographic mobility amino acids are, respectively : L, V, I, C, Y, F, M and W, wherein the said amino acids are represented according to the International Nomenclature.
It has been found according to the invention that the theoretical chromatographic value, which may be calculated as described above, should be corrected when the marker protein which is used possesses, in its amino acid sequence, more than 54% of amino acids having an rf value of less than 0.36.

It has also been found according to the invention that the theoretical chromatographic value, which may be calculated as described above, should be corrected when the marker peptide which is used possesses, in its amino acid sequence, one or several high chromatographic mobility amino acid residues, such as L, V, I, Y, F, M and C.

Thus, in order to further enhance the precision of the protein identification method of the invention, the corrected value for the absolute Y-mobility (Rf) for the said marker peptide is calculated according to the following conditions:

(a) If 54<%lowRf<100 and gmob >0.36, then:

$$Rf(i,j) = \sum \frac{mob(i)}{size} * \left[ \frac{-0.9(\%lowRf)}{44} + \frac{23.15}{11} \right] + \sum \frac{highRf(j)}{size}$$

(b) if 54<%lowRf<100 and gmob <0.36, then:

$$Rf(i) = \sum \frac{mob(i)}{size} * \left[ \frac{-0.9*(\%lowRf)}{44} + \frac{23.15}{11} \right]$$

(c) if lowRf <54% and gmob >0.36, then :

$$Rf(i,j) = \sum \frac{mob(i)}{size} + \sum \frac{highRf(j)}{size}$$

(d) else :

$$Rf(i) = \sum \frac{mob(i)}{size}$$

wherein :

- Rf (i,j) and Rf(i) both mean the Rf value wherein the distinct conditions above are fulfilled ;
- $\Sigma$ [mob(i)/size] represents AbsMob (or gmob);
- %lowRf is the percentage value of low mobility amino acids contained in the sequence of a said marker which is used for peptide calculating the correction factor which takes into account the percentage of low mobility amino acids and wherein the said correction factor is calculated according to the formula above, depending on the condition which is fulfilled ;
- gmob means the general mobility of the peptide under consideration, which encompasses the absolute mobility [i.e. AbsMob] of the said peptide;
- Size denotes the number of amino-acid residues contained in the peptide; and
- E [highRf(j)/size] represents the correction factor which takes into account the number of high mobility amino acids in the sequence of the said peptide.

In a preferred embodiment of step (g) of the protein identification method of the invention, each set of theoretical localisation values which is part of the (i) pre-built ordered collection of sets of theoretical localisation values, or which is part of the (ii) successive sets of theoretical localisation values, is generated by processing the amino acid sequence of any known protein, so as to generate, for each known protein, a set of amino acid sequences wherein each amino acid sequence corresponds to a peptide fragment of the said protein which is expected to be generated by subjecting the said known protein to the same enzymatic or chemical cleavage as that which has been performed for the unknown protein at step (a) of the method. Then, for each peptide amino acid sequence thus generated from the complete amino acid sequence of the known protein, the theoretical localisation values of the said peptides is calculated by using the same calculation method as that which is used for calculating the theoretical localisation value of each of the marker proteins which are incorporated at step (b) of the method. Thus, the protein identification method of the invention is also characterised in that, at step (g), the (i) pre-built ordered collection of sets of theoretical localisation values or the (ii) successive sets of theoretical localisation values, wherein each set of theoretical localisation values characterises the peptide fragments which are expected to be generated by subjecting a given protein of a known sequence to the same enzymatic or chemical cleavage as that experimentally performed on the unknown protein at step (a) of the method, or alternatively each set of theoretical values which is so generated, is generated by a method which comprises the following steps:

(g1) generating, from the complete amino acid sequence of a known protein, a set of sub-sequences, wherein each sub-sequence corresponds to the amino acid sequence of a peptide fragment which is expected to be generated when the said known protein is subjected to the same enzymatic or chemical cleavage as that experimentally performed for the unknown protein at step (a) of the method ;

(g2) calculating, for each sub-sequence generated at step (g1), the theoretical localisation value of the said sub-sequence by performing an identical computation as that which is performed for calculating the theoretical localisation value of the marker compounds, whereby a set of theoretical localisation values corresponding to the said set of sub-sequences is generated, it being understood that the said set of sub-sequences corresponds to the said known protein,

(g3) comparing the set of corrected localisation values obtained at step (f) for the unknown protein with, respectively, each set of theoretical values corresponding to every known protein contained in the ordered collection of sets of theoretical values.

According to a specific embodiment of the identification method of the invention, wherein the identification of a chemical modification e.g. post-translational modifications phosphorylated amino acid residues, such as is sought, whether or not the protein which is processed is of a known or an unknown sequence, then, at step g2), the theoretical localisation value of every sub-sequence having one or several chemically modified amino acid residues is also calculated, whereby, at step (h), the said unknown (chemically modified) protein is assigned to consist of the protein(s) corresponding to the said selected set(s) contained in the ordered collection of sets.

In a still further embodiment of the method, when no set of theoretical values is selected at step (h), then steps (g) and (h) of the method are performed a second time with sets of theoretical localisation values, wherein each set contains theoretical localisation values corresponding to the peptide fragments which are expected to be generated in the case wherein the enzymatic or chemical cleavage to which is subjected the corresponding known protein is not complete.

In another embodiment of the method, when more than one set of theoretical localisation values is selected at step (h), then a similarity score value is assigned to each of the selected sets of theoretical localisation values.

According to a particular aspect of the said embodiment, at step (h), the unknown protein is assigned to consist of the protein corresponding to the said selected set of theoretical localisation values to which has been assigned the highest similarity score value.

The said similarity score is calculated, when comparing two sets of theoretical localisation values, respectively (i) a first set of theoretical localisation values corresponding to the set of peptide fragments generated from the unknown protein and (ii) a second set of theoretical localisation values corresponding to the predicted peptide fragments generated from a known protein, by:

(i) calculating the number of theoretical localisation values of the unknown protein which are identical to the theoretical localisation values of the known protein;
(ii) calculating the number of theoretical localisation values of the unknown protein which are closely similar to the theoretical localisation values of the known protein;
(iii) calculating the percentage of similarity between the two sets of theoretical localisation values which are compared.

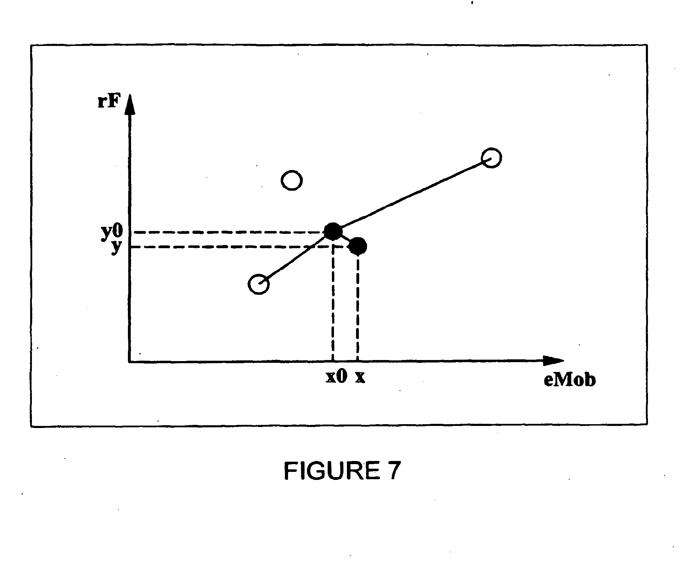

For the comparison of (i) the theoretical localisation value of a given protein-specific peptide fragment from the unknown protein with (ii) the theoretical localisation value of a peptide generated from a known protein in a database, are compared, respectively : (i) the theoretical X-axis coordinate ("x0") of the protein-specific peptide fragment with the theoretical X-axis coordinate ("x") of the peptide generated from the unknown protein and (ii) the theoretical Y-axis coordinate ("y0") of the protein-specific peptide fragment with the theoretical Y-axis coordinate ("y") of the peptide generated from the unknown protein, in order to calculate a distance between the two peptides, and then in order to calculate the similarity score which is the reverse of the distance so calculated. The calculation formula, which is also illustrated in Figure 7, is the following :

$$\underline{Distance} = \text{square root of } [(y-y0)^2 + (x-x0)^2]$$

$$\underline{Score} = 1/Distance$$

In a most preferred embodiment of the protein identification method of the invention, steps (d) to (h) are performed by executing the program instructions of a computer whereby the computer program has been previously loaded within the memory of a computer.

The flow-charts detailing the various steps of the protein identification method of the invention, including those steps which may be performed with a computer program, are illustrated in figures 1 to 5.

As illustrated according to the description of the preferred embodiments of the protein identification method of the invention, further in the present specification, steps (d) through (f) of the said method are preferably performed through the execution of computer instructions included in a specific "program module" which firstly generates a set of image data from the two-dimensional array of peptide spots obtained at the end of step (c). A preferred embodiment of the said first program module is detailed as the "Resolve" program module, further in the present specification. The computer module "Resolve" is notably illustrated in Figure 3.

Using the said first Resolve program module, primary image data from the two-dimensional array of peptide spots are acquired in the memory of a computer. In a most preferred embodiment, the image file which is so generated is formatted under the conventional TIFF standard, for example the TIFF standard 6.0. Then, the Resolve program module maps the image and determines the localisation of at least one, most preferably five, marker compound(s), which are herein also termed "landing lights", and then corresponding experimental localisation data are loaded in the computer memory, this step corresponds to step (d) of the method. In a further step, the Resolve program module determines the localisation discrepancies between the calculated theoretical localisation values for each marker compound of a known amino acid sequence and the actually observed experimental localisation values of each marker compound which have formerly been mapped, and then correction factors are generated for adjusting the calculated theoretical mobility of each marker compound, to compensate for the observed discrepancies, which corresponds to step (e) of the method. The correction factors are then applied for calculating the theoretical localisation value of each protein-specific peptide fragment spot contained in the mapped image, and then the corrected localisation values thus generated are stored in the computer memory for their further use by a subsequent module of the program, which has herein been termed "Resolution" program module, which corresponds to step (f) of the method.

The second program module, which is herein termed "Resolution", is specifically designed for performing steps (g) and (h) of the protein identification method of the invention. The computer module "Resolution" is notably illustrated in Figure 5. In a first step, Resolution contains computer instructions for executing a comparison between (i) the calculated theoretical localisation values of the various protein-specific peptide fragment spots generated from the unknown protein, whose theoretical localisation values were formerly generated by the Resolve program module, and (ii) the calculated theoretical localisation values of the expected peptide (or protein) fragments generated by subjecting known protein sequences contained in pre-built protein database(s) to the same enzymatic or chemical cleavage as that undergone by the unknown protein at step (a) of the method. In a first comparison step, the Resolution module positively selects, as being a pertinent target protein, any protein contained in the database(s) for which there exists an identity, or alternatively a close similarity, between (i) the calculated theoretical localisation value of at least one calculated peptide fragment derived from the said known protein and (ii) the calculated theoretical localisation value of at least one protein-specific peptide fragment spot from the unknown protein, whereby a first set of pertinent protein targets is selected. Once the Resolution module has selected the said first set (or group) of potential pertinent target proteins, among those contained in the said database(s), the selected target protein sequences are further processed for a more accurate and extensive comparison analysis. The more accurate and extensive comparison analysis is carried out by performing successive iterations of the comparison between the respective theoretical localisation values (from the unknown protein and from the compared known protein, respectively), with a requirement for increasing similarity scores, i.e. a requirement that an increasing number of peptide fragments from, respectively, the selected target proteins on one hand, and the unknown protein in the other hand, be identical, or alternatively highly similar. The iteration of the comparison analysis for an increasing number of cycles is herein designed to progressively increase the "stringency" of the protein identification method of the invention. In an eventual final comparison step, theoretical localisation values calculated for each and every peptide fragment for a given selected pertinent target protein thus compared to the calculated theoretical values of each and every protein-specific peptide fragment formerly generated for the unknown protein.

If, after having performed the iterated steps described above, the selection of pertinent target proteins is not univocal, then the Resolution module performs the calculation of a new set of theoretical localisation values for each protein contained in the database(s), or alternatively for each protein contained in the final set of pertinent target proteins which was formerly selected through the iterated comparison steps above, wherein it is taken into account the value corrections to be applied for possible post-translational modifications of the said known proteins, respectively : protein phosphorylation on tyrosine, threonine and serine; glycosylation on serine, threonine, asparagine and hydroxylysine; C- and N-terminal modification by methylation or myristilation; and modification of internal lysine by acetylation.

Preferably, for calculating the theoretical localisation values of those modified peptides skilled in the art will refer to Table 2 of the present specification.

Then, if after having performed the steps described above, including the comparison steps with the theoretical localisation values for post-translationally modified known proteins, the selection of pertinent target proteins is not univocal, then the Resolution module performs the calculation of a new set of theoretical localisation values for each protein contained in the databases(s), or alternatively for each protein contained in the final set of pertinent target proteins which was formerly selected, wherein it is taken into account the value corrections to be applied for possible incomplete enzymatic or chemical cleavage of the said known proteins.

At the final comparison step, the known protein for which the theoretical localisation values are more than 99% similar (value of > 0.99 for the similarity Score) to the the theoretical localisation values calculated for the unknown protein is assumed to be identical to said unknown protein, whereby the unknown protein initially contained in the sample at the beginning of step (a) of the protein identification method of the invention is finally identified and characterized.

In the unlikely case wherein more than one known protein shows a greater than 99% similarity with the unknown protein, the Resolution module prompts the user to enter data for peptide mobility derived from a distinct enzymatic or chemical cleavage as that actually performed for the unknown protein at step (a) of the method, in order to finally assign the unknown protein to consist of a known protein.

Another object of the invention consists of computer memory support which is readable by a computer wherein the said computer memory support contains digital information consisting of a computer program and wherein the said computer program is loadable in the memory of the said computer for performing steps d) to h) of the method according to the invention.

The present invention also concerns the computer program containing the instructions necessary for performing steps d) to h) of the method of the invention.

A further object of the invention consists of a computer the memory of which is loaded with a computer program as defined above.

### _MOST PREFERRED EMBODIMENTS OF THE IDENTIFICATION METHOD_

### A. Preferred embodiments of the biological or chemical techniques used.

### 1. Protein labeling

Proteins for analysis can be labeled prior to separation and digestion. In a typical embodiment proteins from mammalian fibroblasts are radioactively marked by substituting certain amino acids with radioactively labeled amino acids. To those familiar with the art methionine labeled with [$^{35}$S] is frequently used, although this limits the peptide analysis to peptides containing methionine and can give erroneous results. An alternative is to substitute amino acids with [14C] labeled amino acids. This method allows for the choice of amino acids which complement the cleavage sites used to cleave the protein into peptides. An example would be to substitute arginine and lysine with [14C] arginine and [14C] lysine for a protein to be cleaved with trypsin. This would result in each peptide generated from the cleavage containing a radio label. Other alternatives are to make use of biotinylated amino acids in which lateral side chains of the amino acid are covalently linked to a biotin molecule.

### 2. Protein sample separation

The method used to obtain proteins to be identified in this invention is not limiting. A wide variety of techniques for separating proteins are well known to those skilled in the art (Deutscher and Abelson, Methods in Enzymology) and may be employed to provide material for the present invention. By way of example and not limitation, proteins may be separated on the basis of isoelectric point (e.g. by isoelectric focusing or chromatography) of electrophoresis in the presence of a denaturing agent such as urea or sodium dodecyl sulphate (SDS) with and without prior exposure to a reducing agent such as dithiothreotol or 2-mercaptoethanol), by chromatography, including HPLC or FPLC, on any suitable matrix (e.g. gel filtration chromatography, ion exchange chromatography, reverse phase chromatography or affinity chromatography for instance with an immobilized antibody or lectin) or by centrifugation ( e.g. velocity centrifugation, isopycnic centrifugation).

In most cases it is preferable that 2 different separation steps are performed exploiting different characteristics of the protein. For example two-dimensional electrophoresis involving a first step in which proteins are separated on the basis of the charge and a second step in which separation is achieved on the basis of a protein's physical mass.

### 3. Protein labeling and detection

After the separation step proteins can be detected by a number of classically used techniques (colorimetric and/or radioactive labeling). The method of preference for detecting proteins involves incorporation of radioactive amino acids which correspond to the cleavage sites of either a proteolytic enzyme or chemical process. For example for trypsin, cells are incubated for an appropriate time in the presence of [$^{14}$C] arginine and [$^{14}$C] lysine. After separation, radioactive proteins are detected by standard techniques, auto-radiography on film, an imaging technology (for example Instant Imager from Packard Instruments) or phosphorescence imaging (Storm Phosphorimager, Molecular Dynamics Inc).

### 4. Proteolytic digestion

A wide variety of techniques for cleaving polypeptides into peptides, well known to those skilled in the art, such as the technique disclosed by Boyle et al. may be employed in the present invention. By way of example, but not limitation, proteolytic cleavage involving enzymes which cleave specific sites on polypeptides (e.g. but not limited to: Trypsin, chymotrypsin, Clostipain, Trypsin [lys blocked], Proline Endopeptidase, Myxobacter protease, Armillaria Protease, Staphylococcal Protease , Endoproteinase Asp-N) chemical fragmentation (e.g. Cyanogen Bromide, 2-nitro-5-thiocyanobenzoic acid [NTCB]) and acid hydrolysis ( pH [2.5]). Under the simplest of methods, a protein can be fragmented by a single method or enzymatic treatment, however a combination of two methods (using two different proteolytic enzymes) applied separately on the same unknown protein significantly improve the accuracy of the technique. The use of two methods simultaneously (i.e. digesting the unknown sample protein with two enzymes in the same tube) can in some circumstances be an effective method for large proteins and the program is capable of calculating the profiles for the search.

### 5. Landmark peptides inclusion

A key process to this invention is the inclusion of landmark peptides in the separation phases. These peptides serve as standards and enable the distance migrated by the peptides from the unknown protein to be quantified in terms of emobil and Rf. In one embodiment, marker peptides are in the form of specific peptide sequences synthesized in vitro using standard methods familiar to those with experience in the art. The precise sequence of these peptides is not of importance. However, and exact biological knowledge of the sequences is essential. Any number of landmark peptides can be used and in the present invention we have chosen 4. Increasing numbers of landmark peptides decreases the potential for errors resulting from inconsistencies in the media used in the separation phases. With high quality commercial TLC plates these inconsistencies are rare. The use of synthetic peptides is the preferred form for landmarks since they can be synthesized accurately guaranteeing the sequence. Furthermore they can be synthesized with post-translational modification introduced chemically during the synthesis process thus confirming the changes in mobility in the separation phases. Finally, they can be synthesized in large quantities. In another embodiment, peptides arising from the cleavage of a known protein present in the cleavage reaction could be used as landmarks. One such example of these could be the cleavage products of the proteolytic enzyme used to cleave the unknown protein. In such an example digestion of an unknown protein with trypsin, the peptides arising from trypsin digestion could be used as landmarks. Such a case would require that the peptides arising from trypsin were distinguishable from the peptide from the unknown protein. Typically, trypsin would be covalently labeled with a fluorescent moiety (such as fluorescein and the unknown peptide marked radioactively). An alternative would be to add a protein of known sequence to the digestion mixture. Again the peptides of known form would be used after detection to standardize the separation steps.

In another embodiment, landmarks could be synthetic standards (oligonucleotides, chemical agents) of which the exact characteristics in terms of mobilities in the separation phase are known. Such molecules would have to be standardized to the separation phases with respect to amino acids.

### 6. Labeling landmark peptides

Landmark peptides need to be detected after the separation phase, any technique common to those in the art can be used including, but not limited to, radioactive labeling, fluorescent labeling, direct coloration techniques e.g. Ninhydrin staining. Labeling the peptides with a radioisotope is at this time the preferred method. Peptides can be radiolabeled by the incorporation of a radioactive amino acid into the sequence during the synthesis, through the subsequent modification of an amino acid side chain after synthesis or through the radioactive labeling of another element of the peptide (e.g. through the labeling of the spacer sequence). To label peptides during synthesis, a radioactive amino acid is incorporated during the synthesis process in a manner similar to that for other amino acids (i.e. TBOC or FMOC). The radio label should not be included in a labile group which may be lost during synthesis. [$^{14}$C] carbon or [$^{3}$H] labeled amino acids are available from common sources (e.g. Amersham Pharmacia PLC) and are suitable because of their long half lives, but this does not exclude the use of any other radio source. There is no restriction on the amino acid to be labeled.

In another embodiment, landmark peptides can be radioactively labeled after synthesis through the covalent conjugation of radioactive side chains. One example (but not limiting) involves the covalent acetylation of a carboxy terminal cysteine with [$^{14}$C] labeled iodoacetymide. Many peptides are synthesized with amino or carboxy terminal cysteine residues for subsequent protein conjugation. Techniques for such conjugation are common to those familiar with the art (Pierce Catalog) an example would be the dissolution of 5 mg of purified peptide in an inert buffer (50mM phosphate pH 7.2). To this solution a 1 M excess of Iodo [1-$^{14}$C] acetamide in DMSO (dimethy sulphoxide) is added, after mixing and incubation for 30 minutes the reaction is stopped by the addition of 50 molar excess of dithiothreitol in water for 5 minutes. Peptide and radioisotope are separated by HPLC on a C14 column developed from 0-10% acetonitrile. Alternative strategies involving the conjugation of amino acid side chains (such as on lysine, arginine) or other groups can also be used with similar efficiency.

A similar approach can be used to covalently bond a fluorescent agent based on fluorescein, rhodamine or other such derivatives (such as those described in the Handbook of Fluorescent Probes, Molecular Probes, Inc. directly to the peptide after synthesis. Fluorescently labeled peptides are purified under less harsh conditions such as by gel filtration chromatography. In another embodiment, the landmark peptides can be identified by classical staining techniques for example direct labeling of free amino groups with ninhydrin (maximal sensitivity: 1-2 ug).

In another embodiment, the landmark peptides could be peptides arising from a known protein cleaved by the same method as the unknown protein. An example would be to use the peptide products from the autolysis of a protease such as trypsin. Trypsin can be fluorescently labeled on lysine and arginine side chains with a amino reactive fluorescent moiety such as DiChloroTriazinyl amino fluorescein (DTAF) essentially as described by the manufacturer (Molecular Probes Handbook of Fluorescent Probes, technical sheet).

Usually proteins need to be subject to reduction and alkylation before they are used to generate peptides. This ensures that all disulfide bonds in a protein are broken. If the protein has not been treated in this manner, check "with all cysteines in reduced form". The choice of iodoacetamide, iodoacetic acid, 4-vinyl pyridene, and acrylamide can be used to reduce and alkylate cysteines. Note that in proteins prepared by polyacrylamide gel electrophoresis, it is common for cysteines to react with free acrylamide monomers during the electrophoretic process.

## 7. Peptide labeling

If the protein to be identified comes from a non-radioactive source (blood sample, tissue sample biopsy etc.) and is not available in sufficient quantities to be detected by ninhydrin (1-2 ug/peptide), the protein can be labeled prior to cleavage. Various methods exist and are known to those familiar with the art and include conjugation of a fluorescent moiety to specific amino acids, biotinylation of specific amino acids and coupling a radioactive moiety to specific amino acids. For fluorescent labeling a wide variety of potential probes exist and amine and thiol reactive probes are most widely found in the literature. Typical amine reactive probes include fluorophores conjugated to isothio cyanates, sulfonyl chlorides or succinimidyl esters. A wide spectral range of fluorophores are available (c.f. Molecular Probes, Handbook of Fluorescent Probes and Research Chemicals). Alternatively, peptides can be fluorescently conjugated using thiol reactive agents (iodo acetamides and maleamide are examples) which react principally with cysteine residues. When using a thiol reactive probe to label peptides, the database search must also be limited to only peptides containing cysteine residues. Typically after cleavage with an appropriate proteolytic enzyme, the peptide mixture is reacted with a 10 molar excess of the appropriate fluorochrome for 30 minutes before being separated as described below. The reaction can be quenched by the addition of any amine containing solution for example Trisma Base. For thiol reactive agents, the same procedure is followed excepting that the reaction is quenched by the addition of 10 molar excess of DTT, beta mercapto ethanol or any other thiol containing reagent. In a similar manner peptides can be labeled by the incorporation of biotin groups onto specific amino acids and biotin conjugated succinimidyl esters for labeling amino groups are available. Another alternative, peptides can be labeled by the incorporation of [$^{14}$C] containing groups. One such example is to label peptides with [$^{14}$C] iodoacetic acid (available from Amersham Pharmacia Biotech) in a reaction similar to that described for the fluorescent thiols since iodo acetamide reacts with cysteines. Again in the analysis program, the search needs to be performed in the specific knowledge that only peptides containing cysteines are visible.

## 8. Peptide separation

Peptides are separated by a number of techniques familiar to those in the art including but not limited to electrophoresis and chromatography on thin layer cellulose acetate or silica plates. A full description of this type of technique is given in the HPTLC handbook (CBS Scientific Inc, San Diego). Such plates consist of a thin layer of cellulose (or silica) adherent to a solid support to give two dimensional spatial support. The solution containing the peptide mixture from the unknown protein derived from the cleavage step should be dehydrated (by lyophilisation for example) such that the peptide mixture does not contain salts or other contaminants arising from the cleavage step. The peptide mixture is rehydrated in a buffer suitable for the pH at which the electrophoresis is carried out. A typical example would be 2% (w/v) formic acid 8% (w/v) acetic acid pH 1.9 in water essentially as described in Boyle et al., 1999). After resuspension, the landmark peptides are added and the mixture spotted onto the TLC plate minimizing the size of the origin. Peptides are subject to HPTLC at 990 V for 30 minutes essentially as described by the manufacturers. Other methods for electrophoresis (for example the Pharmacia Multiphor, Amersham Biosciences) or the use of other types of support including, but not limited to, plastic backed silica TLC plates can be substituted for the glass backed TLC plate. 20 X 20 cm and 10 x 10 cm plates have been used successfully. Electrophoresis must be carried out for fixed time lengths to obtain reproducible results, the length of the run is not limiting except that peptides may be lost into the chromatography baths. After electrophoresis, plates are dried to remove solvents before being subject to chromatography. While many combinations of buffer could be used, three buffers are commonly used as described in Hunter et al 1988.

In another embodiment, the separation phases could involve separation in polyacrylamide gels using electrofocusing and electrophoresis steps. Such methods are known to those familiar in the art. Calibration of the mobilities under the two separation phases using amino acids and peptides of known sequence would be required to enable identification of unknown peptides. The difficulties in accurately attaching the electofocusing phase to the electrophoretic phase may introduce errors reducing the efficiency of this embodiment.

## 9. Peptide detection

After separation peptides can be detected by a number of techniques currently in use including (but not limited to) auto-radiographic exposure of light sensitive film, phosphorscene imaging such as with a Storm phosphor imager Molecular Dynamics electroimaging using an Instant Imager (Packard Instruments Inc.), according to the manufacturer's instructions. In a typical embodiment, the TLC plate is air dried after the electrophoretic and chromatographic steps to evaporate solvents and exposed directly in an Instant Imager (Packard Instruments Inc.) according to the manufacturers instructions ( Instant Imager User's Guide No 7001158, Publication, pp. 169-4106 ). Images are saved in standard TIFF format directly from the imager since this is a well established industry standard for these types of image, though this does not exclude storage in other image formats (GIF, JPG, SGI-RGBA). In another embodiment of this invention, TLC plates are imaged on a phosphorescence image scanner (Storm 450 Molecular Dynamics Inc.).

In a further embodiment, TLC plates are exposed on high-speed film such as Kodak XAR Lightning Plus (Kodak address) until the radioactive spots become visible. After development and drying the exposed film is scanned using a 16 bit (or better) flatbed (or other) scanner such as an Agfa Cool scan. Such 16 bit images are subsequently saved in an appropriate format compatible for reading by Resolve (Tiff 6 for example). Alternatively, the photographic film can be photographed with a camera equipped with a digital capture surface such as the Kodak M3 or M5 or M7 chips. Examples of these exist in the Kodak DCS400 or similar, although any digital camera capable of high resolution imaging in 12 or more bits is sufficient. A high resolution digital CCD camera such as a Princeton Imaging CoolPix 2000 can also be used to image the exposed film. Images are stored on a digital storage device on a computer in any image format suitable for storing both image data and the textual information concerning the buffer conditions and chromatography and other user data particularly cleavage method, protein source, protein purification method and other related information the user may consider important. In a typical example the images are stored in TIFF 6 format.

In another example the image could be stored in RGBA format (Silicon Graphics Standard Image format). An image depth of 12, 16 or more bits/pixel is preferable. Images can be printed for user analysis and archives.

## 10. Peptide mobility localization

The invention describes a method for creating a peptide mobility array, attributing an electrophoretic mobility (emobile) and chromatographic mobility (Rf) to the plurality of peptides visualized on the image. The process behind this is revealed in Figure 4 and involves: reading the image, removing imperfections from the image, identification of the peptide spots on the image, input of the landmark peptides and their position, calculation of pixel/emobile and pixel/Rf ratios, attribution of an Rf and emobile to each peptide, construction of a peptide mobility array for the plurality of peptides from the protein and storage of this array for use in the identification of the protein through comparison with known proteins.

In an embodiment, a computer assisted method is used to read images in TIFF format from an Instant Imager.

Standard TIFF library functions are used as described in both the TIFF 6.0 specification and the LibTIFF (Leffer et al, SGI). The invention can open images stored in a variety of formats from different types of imaging machines. By preference images in TIFF format are preferred since they are the most commonly used in imaging devices at present. TIFF images which do not conform to the TIFF 6 specification (e.g. because of dis-coordination in the TIFF tags) are also opened by the computer assisted method correcting the tag position as the image is read. The image is displayed to allow manual user intervention. This involves defining those regions in the image where the peptides do not migrate. In all cases this is the area below the origin and to the left of the origin at pH 1.9. At higher pH values peptides may migrate in both directions. The same operation is used to define the limits of the TLC plate thus restricting the area of the image analyzed to find peptides to that in which peptides are likely to have migrated. Subsequently, the image is subject to automated noise reduction using standard techniques common to those in the art. Typically, the first step involves the removal of image imperfections through a thresholding technique to define the center of each peptide. Secondly the image is subject to watershed analysis to define the perimeter of each peptide identify peptides which overlap. After definition of the peptides, the position of the landmark peptides are defined on the image as is information of interest about the protein, its apparent molecular weight and isoelectric point (pl). Finally the image and associated textual data can be stored in the form of a computer readable file and printed for user analysis.

### B. Preferred embodiments for performing steps (d) to (h) of the method through the use of a computer program.

### I. General features of steps (d) to (h) of the computerised method.

According to the invention, computer programs comprised of a first and a second module have been designed to acquire and manipulate the said data output generated by the method according to the first aspect of the invention. In a preferred embodiment, the said modules perform as follows :

- the said first module, so-called RESOLVE, is advantageously designed to acquire primary image data derived from the proteolytic digestion of the unknown protein, after separation of the peptides by two dimensional electrophoretic and chromatographic separation, whereby mobility coordinates such as relative molecular mass (mW) and charge (pl) are annotated, and then read in the image file. Images can be acquired for proteins preferably either labeled with radioactivity or with specific fluorescent probes

- once acquired into the memory, the said first module maps the image and determines the localization of the marker peptides, preferably 4 peptide markers. In the acquisition step of the said image, the said first module advantageously uses a PhosphorImager (Molecular Dynamics Inc, Mountain View, CA) or Instant Imager and the image is transferred to a SGI Iris Indigo 2, deconvoluted using preferably a program designed for acquiring images preferably Tiffimager, conforming to the Tiff version specification.

In one embodiment, once images are acquired, the said first module allows the user to manually identify the position of the marker peptides and the origin of the peptide map. This is done via a mouse driven grid, which enables the user to accurately pinpoint the center of the marker peptides and attribute this a number using the right mouse button. In addition, the said first module advantageously incorporates important information directly in the image file (including precise conditions of the peptide map, experimental conditions such as buffer conditions, pH of the first dimension, approximate molecular mass and pl of the protein as identified from the two-dimensional separation step and the class of the marker peptides, which are pH dependent).

Once the marker peptides are memory mapped, the said first module determines errors or differences between the normalized sample mobility and the actual observed values and adjusts the calculated mobility to compensate for these changes, the corrected values for each peptide being stored for subsequent use. In this particularly preferred embodiment, at least two of the marker peptides carry post-translational modification(s). Thus by permitting calculation of the errors resulting from possible post-translational modification and/or failures to correctly proteolytically digest, the method of the invention brings a new level of accuracy to the processes of protein identification and analysis. In this preferred embodiment, the said first module determines compensation factors for both the electrophoretic and chromatographic mobilities in areas surrounding the marker peptides.

Essentially, a peptide map is advantageously divided into 28 zones (7 per marker peptide) and the said first module calculates the corrected mobility in both the first and second dimension for each marker peptide to determine a local correction factor, which is used to correct the mobilities of the peptides from the unknown protein. Although sufficient information to standardize migration of the peptides can be obtained by correcting as few as 3 marker peptides, it is preferred to use 4 marker peptides to obtain maximal coverage of the chromatographic support, notably a thin layer chromatography (TLC) plate, in the second dimension of the two-dimensional separation step. Preferably, there is a set of markers for each pH range used in the primary electrophoretic step.

The second module, so-called Resolution, of the computer program is advantageously designed to compare the thus stored image or mobility data and data file generated by the first module, against a calculated public database of all known proteins

- The said second module further reads into memory the determined amino acid sequence from the published public database and determines the predicted relative and chromatographic mobilities for the protein after digestion with the proteolytic enzymes used for the peptide mapping of the unknown protein. Essentially, in a first alternative of this embodiment, called high speed mode, the said second module compares the most extreme peptides from the *in vivo* data (max rfo-min Ec, min rfo-max Ec) against the same coordinates/values for a database sequence.

Once the ideal values for the known proteins are calculated the said second module compares the latter against those calculated for the unknown protein after adjustment for experimental running conditions and eventually the known protein is classified as a potential target and stored for further analysis.

Said second module systematically addresses each protein in the said public data base.

Once a group of potential target proteins is thus isolated, the said second module then performs then an extensive analysis, each and every peptide for a given protein is identified and compared against those observed and corrected,

The said second module further analyses each protein, identifying known proteins with higher and higher similarities to the unknown protein.

Then the said second module, most preferably, « algorithmically » subjects each peptide to different post-translational modification to determine if the discrepancies between the known protein and the unknown protein result from modification of the unknown protein

The said second module compensates for the possibility that proteins have been incompletely digested by a chosen proteolytic enzyme resulting in sites that are not cut ; in a preferred embodiment of this particular step performed by the second module, these "no cut" sites are mapped to be preferably phosphorylated.

The said second module further identifies proteins which have peptide maps in which more than 99% of peptides display mobility similar to those of the unknown protein and are thus assumed to be identical and given as a correct hit.

In case no proteins are retained, in another equally preferred alternative to the so-called high speed mode, the said second module automatically performs a so-called high stringency search comprising the steps of calculating the complete mobility profile for each protein in the database one after the other and then comparing each peptide profile to that of the unknown protein and retaining all proteins with peptide profiles showing greater than a user set similarity to the experimental data, generally 50%,. Advantageously, the said second module takes into consideration only proteins which have a molecular mass of about 20 kDa, preferably 10 kDa, greater or less than the identified mass of the unknown protein and with a pl (protein charge) $\pm$ about 1,5 pH unit.

Following the so-called high speed procedure or the eventual high stringency one, the said second module advantageously draws at least two peptide fingerprints overlapped and aligned to the said marker peptides, thereby allowing, for example by simply "double clicking" on a spot of the searched for peptide, the predicted sequence of the unknown protein to be displayed.

### II. Detailed features of the computerized method

### II.A. Module RESOLVE

#### *1. General features*

The first module/component is a computation module which acquires primary image data derived from the proteolytic digestion of an unknown protein after separation of the peptides by two-dimensional electrophoretic and chromatographic separation. Resolve reads an image of the protein acquired by any standard image acquisition software in the format by defined the TIFF standard 6.0. Images can be acquired for proteins either labeled with radioactivity of with specific fluorescent probes (see figures 1 to 3). If peptides are derived from proteins labeled with specifically labeled amino acids ([$^{14}$C] or [$^{35}$]S for example). Resolve communicates this limitation to the resolution module in a subsequent analysis. The same is true if peptides are marked post translationally by [$^{32}$P]. Once acquired into the memory, Resolve maps the image and determines the localization of 5 marker peptides (landing lights) (see figures 3 and 4). The purpose of the landing lights is to provide relative mobility in two dimensions of peptides of absolute and invariant mobility which serve to compensate for variations in plate manufacture and electrophoretic and chromatographic running. Once the marker peptides are memory mapped Resolve determines the errors between the normalized sample mobility and resolution and the actual observed values and adjusts the calculated mobility to compensate for these changes. The corrected values for each peptide in the mapped image are stored by the program for subsequent use by the resolution module.

The principle functions of this Resolve module are, in brief, the acquisition of a digital image, the definition of the limits of the electrophoretic zone, the creation of an image in Resolve format, the identification of the plurality of the peptide spots on the image, the identification of the landmark peptides and the conversion of the distances in pixels/cm into relative electrophoretic and chromatographic mobilities.

*2. Detailed description of the module RESOLVE*

a) Function ACQUIRE:

This is an image acquisition module running under MOTIF which includes functions to acquire the image of the peptide map and incorporate positional data for the origin of the peptide map, the upper level of the chromatography buffer and the position of the 4 marker peptides. The primary function of this module is to read in the image file of the peptide map. Resolve currently supports standard TIFF images conforming to the TIFF Version 6 specification, images using the Molecular Dynamics modified TIFF specification and images conforming to the Silicon Graphics RGBA image specification. Other image formats including GIFa or JPEG can be read by Resolve although some loss of image data results when images are stored in these compressed formats.

Once images have been acquired, according to one embodiment, the program allows the user to manually identify the position of the 4 marker peptides and the origin of the peptide map. This is done via a mouse driven grid which enables the user to accurately pinpoint the center of the marker peptide and attribute this position a number using the right mouse button. In addition, this module incorporates important information directly into the image file. This includes the precise conditions of the peptide map, buffer conditions, pH of the first dimension, the approximate molecular mass and pl of the protein as identified from the 2D gel and the class of marker peptides (which are pH dependent) used. Finally, the modified image can be saved as a specific Resolution image.

The expression "Read Image" corresponds to the functions of resolve module, namely in particular, to acquire the image data, annotate it for the position of the marker peptides, molecular weights and protein pl.

The saved image comprises a unique header (to identify the file as a resolution image) an offset to the image data, an offset to the mobility coordinates. The latter are single lines per peptide, emobile rF, and peptide number. In addition, the file contains the annotation information and the positional information of the marker peptides.

b) Definition of the electrophoretic/chromatographic area

In order to be able to perform the pixel to Rf and pixel to *emobile* conversions it is necessary to define the electro-chromatographic area. This area is defined as the region on the TLC plate in which the peptides have migrated. Peptides are applied to the TLC plate at a single site, the origin (c.f. Hunter TLC 7000 user manual). With respect to the chromatography phase, because peptides migrate in the direction of the solvent front, the chromatographic area is defined as the area from the origin to the upper solvent limit. In most cases this is the edge of the TLC plate opposing the edge of the TLC plate at which the solvent entered the plate. The electrophoretic area depends on the pH at which the electrophoresis was run since pH changes affect peptides' net charge. At pH 1.9 all peptides migrate from the origin towards the anode (+), defining the electrophoretic area as the area from origin to the anode edge of the TLC plate. Thus the effective electro-chromatographic area would exclude the areas from the cathode edge of the plate to the origin and from the edge at which the solvent entered the height of the origin. When electrophoresis is performed at a higher pH, peptides may migrate towards both the cathode and anode. For example at pH 5, the origin should be placed in the center of the plate in order to keep all the peptides on the plate and the electro-chromatographic area would only exclude the area from the edge at which the solvent entered the height of the origin. At pH 8.9, all peptides migrate toward the cathode. As such the electro-chromatographic area would exclude the area from the anode edge to the origin and from the edge at which the solvent entered the height of the origin. In one embodiment the electro-chromatographic area is delineated by the user directly on the image and can be both adjusted by the user to compensate for error and saved with the image for subsequent use.

c) Module for the identification of peptide positions

The next function is to identify the position of the plurality of peptide spots on the image. This is achieved by standard morphological image analysis algorithms such as those described in *Morphological Image Analysis, principle and applications,* (P. Soille, Springer 1998). The first stage involves image pretreatment to identify and subsequently remove pixel noise. One embodiment (but not limited to this) involves sequential applications of dilation and erosion filters. Erosion filters (well known to those familiar with the art) involve determination of the minimal value of adjacent pixels which is subsequently used to round down the pixel values in the image scanned by the filter. In contrast dilation involves determining the maximal value for a group of pixels and rounding the pixels in the adjacent group up to this value. Both functions serve to filter the image to improve the signal to noise ratio. In another embodiment, the same result could be obtained by thresholding the image to a standard value determined from the image background. A further embodiment to achieve a similar result is to apply median or means filters to smooth the image for noise. Smoothing via mean filtering involves calculating the average value for a fixed number of pixels surrounding the pixel to be analyzed. Typically this involves calculating the average pixel value for a square of pixels (3x3, 5x5, or 9x9) excluding the central pixel and attributing this value to the pixel in the center of the square (Morphological Image Analysis, principle and applications, P. Soille, Springer, 1998). The square is sequentially moved along the image row pixel by pixel and repeated for each row in the image. Alternatively, the signal to noise ratio can be reduced by application of a median filter which involves essentially the same method as for the mean filter with the exception that instead of calculating the average for the pixels in the square, the median is calculated from the 9 pixels in the square and applied to the central pixel of the square. The net result is that the image is scanned in its entirety and erroneous pixel noise reduced.

Once the image has been smoothed the next step involves the identification of the pixel positions of the centers of the plurality of the peptides on the image. In one embodiment (but not limited to this method) this involves the automated computer assisted identification of internal markers defined as the points representing the centers or point of greatest signal of each peptide spot. In one example this involves the method of regional extrema (c.f. Chapter 6 Geodesic transformations, section 6.4, Reconstruction based operators, in *Morphological Image Analysis,* P.Soille, Springer) known to those familiar with the art. In this method the regional minima is calculated for the plurality of pixels making up the image. Regional minima are calculated because under TIFF 6, maximal values are attributed to white such that black = 0 and white 65535 for an image encoded in 16 bits. The same methodology based on regional maxima can be used on images in which black is attributed the maximal value or to images which are encoded with more or fewer bits per pixel (8, 10, 12, 32, 64 or any other value). In this example images are encoded as gray level images but the same method can be applied to images in color. The detection of regional minima by simple derivation, is extended by the derivation of Detriche, Using Canny's criteria to derive a recursively implemented optimal edge detector (R. Detriche, 1987.. The International Journal of Computer Vision, 1(2):167-187). A typical mask used in the simple derivation is shown in Table 3.

The position of the peptides could be determined by many other methods known to those familiar with the art and include (but are not limited to): Blob analysis, double thresholding (hysteresis thresholds), Top Hat reconstruction and other methods as described in *Morphological Image Analysis, principle and applications,* P. Soille, Springer 1998. These methods are used to identify the pixel in each spot which represents the highest value for that cluster of pixels representing the spot. The exact coordinates of this peak are used as the center of interest of the peptide and are stored as X and Y co-ordinates. Once the centers of interest have been identified, image segmentation and contour analysis methods which delineate the boundary of each peptide spot are used to ensure that closely migrating peptides are not lost from the identification. Many techniques for image segmentation have been described (c.f. P. Soille, Springer 1998, Chapter 9 and subsections therein). The method chosen combines both methods for image analysis using regional growth and edge detection as a morphological approach to image segmentation through watershed transformation. A typical example for the segmentation of images and the identification of boundaries is given in Figure 9.8 (P. Soille, Springer 1998, page 241). Such methods allow for the identification of the peaks of interest in peptide spots which overlap. Other methods for calculating peaks in peptides which overlap could also be used. After image analysis the peptide positions (the X and Y co-ordinates of the peptide peaks) are stored as a pixel mobility array.

In another embodiment, the user could directly identify the position of the spots on the image by manual methods. One such manual method would be to directly pinpoint the position of the peptides using an existing image analysis program (such as Photshop). Alternatively the user could directly measure with a ruler the position of the peptides. While these alternatives are not favored due to the possibility of erroneously pinpointing the exact center of the spot, they cannot be discounted as solutions to relatively simple peptide map arrays.

d) Landmark identification

Landmark peptides are peptides of known biochemical and biophysical nature which act as internal standards for establishing the pixel to Rf and emobile ratios. To calculate the pixel/Rf and pixel/emobile correction factors, centers of interest representing the absolute center of each of the landmark peptide spots have to be calculated with the best precision. In one embodiment, these peptides are synthesized by standard methods and their properties characterized by Mass Spectroscopy by methods known to those skilled in the art. Alternatively the biochemical properties and sequence are determined through a combination of HPLC and direct Edmann degradation sequencing.

The landmark peptides are added to the mixture of peptides resulting from the cleavage process prior to spotting on the TLC plate and electrophoresis and chromatography. In general, the signals from these peptides are significantly stronger than the other peptide signals. The landmark peptides are automatically identified or manually identified by the operator. In an embodiment, a computer assisted method provides the user with a mask comprising circles joined by a wire. The circles can be displaced around the image by means of a pointing device coupled to the computer to give one circle per landmark peptide and one for the origin. Once the circles are placed the operator enters the peptide number and the position of the center of the circle is calculated and defines the pixel position of that landmark peptide. The size of the circle can be varied in a dynamic manner to maximally enclose the surface of the landmark peptide prior to calculating the center of interest for that peptide. In another embodiment, the centers of interest for the landmark peptides are determined automatically. Since the exact level of radioactivity for each landmark peptide is known (they can be analyzed in a scintillation counter) equal amounts of each peptide (in counts per minutes) can be added to the unknown peptide mixture. Subsequently, when the position of the plurality of the peptides is determined (see above) peptides which have the highest radioactivity and equivalent levels of radioactivity represent the marker peptides. The origin is also found in this manner since a proportion of each peptide remains at the origin making it more radioactive than the individual peptides on the plate.

In another embodiment the user could identify the pixel positions representing the centers of the peptide landmarks manually, either on a printed copy of the image, a photographic representation of the image, or on the computer screen.

e) Detection of the origin

The position of the origin is also required for subsequent calculation of the emobility and RF /pixel ratios. In most cases the origin stands out on the image due to the presence of peptides which have not migrated (making it stain darker with Ninhydrin or radioactive peptides and brighter with fluorescent peptides) or due to the physical damage done to the plate during the loading process. In one embodiment the position of the origin is detected in a manner similar to that above for the marker peptides through users directly placing an image pointer (such as a circle) directly at the position of the origin. In another embodiment, the position of the origin is detected automatically by a computer assisted method similar to that described for the detection of peptide spots. In yet another embodiment the position of the origin can be determined manually by direct measurement on an image of the TLC plate in any format, printed film or otherwise. Once the position of the origin is known it is determined or detected automatically.

Once the centers of interest for each peptide have been identified in X,Y pixels (X pixels being pixels along the axis of electrophoresis and Y pixels along the axis of chromatography) these have to be converted to relative electrophoretic mobility (emobile) and relative chromatographic mobility (Rf). The relative distance travelled from the origin is linear with time with respect to the electrophoretic mobility from the origin. Thus the emobile per pixel factor is calculated by dividing the known electrophoretic mobility value for each landmark peptide by the number of pixels from the origin migrated by that peptide. Because the process is linear, the electrophoretic to pixel value for any of the landmark peptides should be sufficient and may be represented as shown below.

Electrophoretic to pixel values:

$$X1 \text{ emobile}/X1 \text{ pixels} =$$
$$X2 \text{ emobile}/X2 \text{ pixels} = \qquad \text{emobility/pixel}$$
$$X3 \text{ emobile}/X3 \text{ pixels} =$$
$$X4 \text{ emobile}/X4 \text{ pixels} =$$

However, slight deviations from linearity may occur due to plate deficiencies. To compensate for this, mean and median values, respectively, are calculated and compared to the individual emobile/pixel rations for each landmark peptide, as it is shown below.

Calulation of the mean value :

Mean value =

$$\frac{\text{X1 emobile/X1 pixels + X2 emobile/X2 pixels + X3 emobile/X3 pixels + X4 emobile/X4 pixels}}{4}$$

Calculation of the median value

Or the median of [X1 emobile/X1 pixels <> X2 emobile/X2 pixels <> X3 emobile/X3 pixels <> X4 emobile/X4 pixels]

A simple statistical test (Students T) is used to identify if non linear behavior of the peptides is observed. If a deviation is observed, zonal correction factors are applied to the peptide map. In one such method the peptide mobility area is divided in zones from the origin to the first landmark peptide, from the first to second landmark, second to third, third to fourth. For each zone the emobile to pixel ratio is calculated as shown below.

Calculation of the emobile to pixel ratio :

$$\text{X1 emobile/X1 pixels = emobile1/pixel zone 0-1}$$

$$\text{X2 emobile/X2 pixels = emobile2/pixel zone 1-2}$$

$$\text{X3 emobile/X3 pixels = emobile3/pixel zone 2-3}$$

$$\text{X4 emobile/X4 pixels = emobile4/pixel zone 3-4}$$

$$\text{X4 emobile/X4 pixels = emobile5/pixel zone 4-5}$$

A similar process is used to calculate the Rf to pixel ratio for the chromatographic phase. The distances from the origin to the pixel center of the peptide for the landmark peptides are calculated for each peptide, Rf1, Rf2, Rf3, Rf4 and used to calculate the Rf./pixel ratio. Peptides run in a linear manner which is confirmed by comparing the mean mobility/pixel for each individual landmark peptide to the mean of the mobilities of all four landmark peptides. In almost all cases, if differences are observed in the Rf/pixel ratios between markers, then the plates are not used and the separation step is repeated until a single Rf/pixel ratio is found for all marker compounds. In rare situations, if differences are observed in the Rf/pixel ratios between markers, then the plate surface area is divided into distinct Rf.-mobility zones, taking into account the values found for each marker compound. RfJpixel ratios are then calculated for peptides migrating in these different zones.

f) Function: Correct peptide mobilities

This module corrects the physical mobility of peptides resolved on the peptide map with respect to the position of the marker peptides. The purpose of this module is to compensate for errors or differences in peptide mobility on the TLC plate due to the running conditions etc. For each marker peptide, the program determines compensation factors for both the electrophoretic and chromatographic mobilities in the areas surrounding the marker peptide. Essentially the peptide map is divided into 28 zones (7 per marker peptide) and the program calculates the corrected mobility in both first and second dimension for each marker peptide to determine a local correction factor, which is used to correct the mobilities of the peptides from the unknown protein (see figure 3).

Although sufficient information to standardise the migration of the peptides can be obtained by correcting from as few as 3 marker peptides, we use 4 in order to obtain maximal coverage of the chromatographic support, in particular, the TLC plate. There are a set of markers for each pH range used in the primary electrophoretic step these are for pH 1.9.

Creating the emobile/Rf. peptide array

The pixel/RF and pixel/emobility rations are used to convert the X and Y pixels in the pixel mobility array calculated above to emobile and Rf. mobility array (subsequently termed the peptide mobility array) which is used in peptide comparison. In one embodiment, X and Y pixel values for the plurality of peptides identified in the peptide analysis process are converted to emobility and Rf. by multiplication of the value obtained in pixels for emobility by the emobility/pixel ratio for that region of the peptide map. In a similar manner the Rf. value for a given peptide is obtained by multiplying the pixel value for a given peptide by the Rf./pixel value for that region of the peptide map. The totality of peptide mobilities (emobile and Rf.) for all the peptides (excepting the landmarks) are stored in an array for subsequent use in the identification process.

In another embodiment, the process of peptide recognition, positioning of the landmark peptides and origin can be performed manually by direct measurement on an image of the peptide map. The image can be any form including (but not limited to) an image printed on paper, film or any other type of support, an image obtained directly by exposure of the peptide map to a film sensitive to radioactivity (in the case of radioactive peptides) or light (in the case of fluorescent peptides) or any other type of electromagnetic emissions (phosphorescence, luminescence, natural fluorescence and fluorescence), a scanned representation of the peptide map obtained using a direct image scanner (eg. an Alphamatic, Agfa) or an image scanned for radioactivity or fluorescence (type Storm). The image may be obtained by direct photography using photographic film and taken from the negative or an image printed from the negative or an image obtained from a digital camera.

For the plurality of peptides two distances are measured; the distance from a line emanating from the center of the origin to the center of the peptide along the axis of electrophoretic mobility (emobile) and the distance from a line emanating from the center of the origin to the center of the peptide along the axis of chromatography (Rf.). In the absence of a measure of the maximal intensity for each peptide the center of the peptide spot is used. These distances can be represented as any form of measurement, (mm, cm, m, inches) and from the values obtained for the landmark peptides, values for electrophoretic and chromatographic distances are converted into emobile and Rf. values respectively as described above.

h) Data storage

Once the image has been analyzed and the peptide mobility array calculated, the data is stored. In one embodiment, this involves writing the image data, image annotation and peptide mobility array to a computer file. Typically the image is stored in TIFF format since this allows the annotation data to be stored with the image data. Alternatively, the peptide mobility array is directly entered into the search process. In one embodiment this would involve directly initiating the search program from within Resolve. Another implementation would involve transferring the data through a direct connection to a distant server to be searched by the search engine.

i) Calculation of the theoretical location of the known marker peptides (generation of the « landing lights") on the gel

In a first step, in brief a sub-module named « Net charge » calculates the theoretical net charge of each of the marker peptides for a given pH value, by taking into account the « number» of each amino acid which affects the isoelectric point and the specific pK value for each of these amino acids, including the influence of the amino and carboxy termini.

More precisely, the netcharge of a peptide (or protein) is calculated by the summation of the charges brought to the peptide by its constituent amino acids at a given pH. For amino acids contained at the interior of the peptide, only amino acids which have charged side chains charged at a given pH contribute to the charge of the peptide chain. With respect to the terminal amino acids, the charge of the amino and carboxy groups and the nature of the amino acid are used to calculate the netcharge of the peptide. The netcharge is dependent on the pH at which the electrophoretic step is run which is entered by the user. Netcharge can be calculated according to the following algorithm which returns the netcharge of the protein at a given pH, a count of the number of each amino acid affecting the isoelectric point, and the pK's for those amino acids. Note that the count of amino acids includes the number of amino and carboxy termini.

j) Calculating electrophoretic mobility (emobile)

In the first phase of the separation process peptides are separated according to their charge by electrophoresis in thin layer silica plates (TLC). Electrophoresis can be carried out in the pH range 1.9 to 9.5 by varying the buffer used for the electrophoresis. At pH 1.9 all peptides migrate from the cathode to anode since they are all negatively charged. At pH 9.5 most peptides are positively charged and migrate to the cathode. The electrophoretic step is a non equilibrium process at pH 1.9 and to ensure reproducibility peptide mixtures must migrate for exactly similar times to be comparative.

The electrophoretic mobility can be calculated for any peptide on the basis of physiochemical properties of its constituent amino acids and its molecular weight. The initial step involves determining the net charge of the peptide which is derived from the charged status of the carboxy and amino terminal groups of the peptide and the amino acid side chains essentially on lysine, arginine, histidine, tyrosine, glutamic acid, aspartic acid, phospho serine, tyrosine and threonine. Positive net charges are contributed by lysine, arginine, histidine and the peptides' amino termini while negative charges are brought by tyrosine, phospho-tyrosine glutamic acid, aspartic acid, phospho serine, tyrosine, threonine, phospho threonine and the carboxy terminal group. Net charge is essentially calculated by determining the concentration of hydrogen ions, then the sum of the netcharges for the positively charged amino acids in the peptide (netplus) and negatively charged groups. The process can be summarized as follows:

*Calculate hydrogen ion concentration [H+] which is given by:*

$$10^{x(-pH)}$$

*Calculation of netplus:*

$$([H^+] / Kd + [H^+]) + ([H^+] / pK1 + [H^+]) \quad \text{Equation 1}$$

Where Kd is $10^{x(-pKr)}$ and pKr is the pKa for the positively charged amino acids and pK1 is the pKa of the amino-terminal group of the peptide. Netplus amino acids are [ K, R, H +NH$_2$ ].

*Calculation of netminus:*

$$1-( [H^+] / Kd + [H^+] ) + ( 1- ([H^+] / pK2 + [H^+] ) ) \quad \text{Equation 2}$$

Where Kd is $10^{x(-pKr)}$ and pKr is the pKa for the negatively charged amino acids and pK2 is the pKa of the carboxy-terminal group of the peptide. Netminus amino acids are [SYEDsty + COOH] where s, t and y are the phosphorylated forms of serine, threonine and tyrosine respectively.

*Calculation of the netcharge :*

Netcharge = ( netplus - netminus ) or for any :

$$\{([H^+]/Kd + [H^+]) + ([H^+]/pK1 + [H^+])\} - \{1-( [H^+]/Kd + [H^+] ) + ( 1- ([H^+]/pK2 + [H^+] ) ) \}$$

The charges for the amino and carboxy terminal amino acids are given in Table 2 and are taken from (Expasy web site at www.expasy.ch).

For example the artificial peptide: NH$_2$-Y-V-A-H-L-R-COOH would be calculated using the formula given in figure 3:

Once the netcharge has been calculated, the emobil can be estimated from the formula below.

*Calculation of the electrophoretic mobility:*

$$Emobil = netcharge / Mr^{(2.0/3.0)}.$$

Where Mr is the molecular weight of the peptide in daltons. As mentioned above, the emobil is essentially a non equilibrium value for all peptides at the pH extremes 1.9 and 9.5. At neutral pH values, some of the peptides may attain their pKa and as such although reach equilibrium this is unlikely to be the case for all peptides. However, the electrophoretic mobility of a peptide will also be influenced by its mass since the there is an element of resistance on free peptide mobility conveyed by the matrix. The extent to which the molecular mass of the peptide influences its mobility on the TLC plate is dependent on the nature of the matrix and in our hands the 2/3 value corresponds to a reasonable compromise. In effect, the absolute position of peptides with respect to their emobility will depend on the length of the electrophoretic running time and as such, is highly variable. However, what is important for the present process is that the positional differences between individual peptides in the mixture on the plate will be proportionally the same with respect to the origin and independent on how long the electrophoresis has run. As such, by including known peptides in a complex mixture we can directly measure the distance traveled by a peptide and using the formula above translate, that distance into an estimate of electrophoretic mobility, emobile.

k) Calculating the chromatographic mobility

The second component required for the present process is an estimation of the relative mobilities of peptides migrating in a known solvent mixture. While both peptides and amino acids migrate in solvent mixtures, the mobility of the peptide cannot be estimated simply as the sum of the mobilities of the individual amino acids which make up the peptide. The estimation of the chromatographic mobilities is made on the basis of a de novo algorithm which takes into account the differences in mobility observed between aliphatic, aromatic, hydrophobic and hydrophilic amino acids. The initial step is to calibrate the relative mobilities of the 25 amino acids under the chromatographic conditions to be used. There are 25 amino acids since in addition to the 20 common amino acids, which assumes that methionine and cysteine are in their reduced form), we also include phospho-serine (s), phospho-threonine (t), phospho-tyrosine (y) and the oxidized forms of cysteine (c) and methionine (m). To distinguish the latter amino acids from their unmodified counterparts we use capital letters for the normal form and lower-case for the modified form as shown above. For calibration, the 25 amino acids are resuspended in purified deionised water at 1.0 mg/ml. The amino acids are differently soluble but all can be obtained in solution at this concentration. Amino acids (1-3 ug) are then spotted onto TLC plates on a drawn line 1.2 cm from the lower edge of the plate at 1.0 cm intervals from each other. Typically 10 x 20 cm plates are used for amino acid calibration. The plate is placed (amino acid edge downward) into a chromatographic tank containing acetic-acid, n-butanol, pyridine and water (7.55, 37.5%, 25% py 7.5%) and the plate is developed until the visible front (which represents water/pyridine) reaches a point 0.8 cm from the upper plate edge. The plate is air dried and then amino acid positions identified by ninhydrin spray according to the manufacturer's instructions. The rF values for the individual amino acids are calculated by dividing the relative distance migrated by an individual amino acid by the overall distance the solvent migrates along the axis run by the amino acid. The distance run by an amino acid (or peptide) is defined as the distance from the point of origin (the spot on the line 1.2 cm from the plate base) to the front of the ninhydrin spot. The distance run by the front is calculated as the distance from the point of origin of an amino acid (1.2 cm from the plate base) to the front at approximately 0.8 cm from the upper edge along the line of axis of the amino acid (or peptide). An illustration of this calculation for a given peptide is given in Example 1. The rF is calculated as:

$$\{ mob\text{-}aa / mob\text{-}front \} / 100$$

In the present instance, this distance is determined from a digitized image of the TLC plate, however, the values can be determined by physical measurement. Once the rF values for the individual amino acids are determined they are stored such as to be used for the calculation of peptide rFs. It is generally the case that the values we have used are consistent for all peptides run on MERCK TLC plates using this buffer combination such that a third person would not have to calculate these values unless they wished to. However, for the calculation of peptide mobilities in buffers other than the one used here, it is necessary to calculate the individual amino acid rF values for the different buffer solution as described here. The algorithm used to compensate for the differences in mobility of each amino acid in a peptide in comparison to their mobility when they are together in a peptide, applies to all cases independently of the buffer used provided that the correct rF values for the individual amino acids are known.

*k1) Calculating the mobility of a peptide on the basis of its amino acid composition.*

In the first step of the calculation the absolute mobility of a peptide is calculated as the average of the chromatographic mobilities of the amino acids which comprise the peptide. This is simply calculated as the sum of the individual rF values divided by the number of amino acids as given in equation 1.

## Equation 1
### rf-aa(1) + rf-aa(2) + rf-aa(3) + rf-aa(n) /n

This absolute mobility "gmob" only correctly estimates the chromatographic mobility of the peptide if the percentage of amino acids with rf values less than 0.36 is less than 54% in the peptide and if the absolute mobility "gmob" is less than 0.36, such as for the amino acids R, N, D, Q, E, G, H, K, S, s, t, y, c, as it is shown in Table 4. Under all other circumstances, the absolute mobility has to be corrected through the use of one or more correction factors. Correction factors do not apply to peptides with less than 4 amino acids. The formula is given in equation 2.

## Equation 2: [ ( Σ  aa x CF) /n

Where CF is a correction factor.

The correction factor CF varies in a manner in an inverted proportional manner, more precisely in a linear manner, dependent on the % of low mobility amino acids in the range of low mobility amino acids from 54-100% such that the correction factor is 1 for 54% low mobility amino acids or less and 0.05 for 100% low mobility amino acids:

The third component which we have identified as influencing the mobility of a peptide is the additional contribution of the hydrophobic and aromatic amino acids. These amino acids migrate with mobilities generally greater than 0.5 as such their contribution to overall mobility of a peptide was underestimated in equation 1. The final equation which completely describes the mobility of a peptide in terms of the individual rf values of its constituent amino acids is therefore given by equation 3:

### [ ( Σ  aa x CF) + (Σ L,V,I,Y,F,W,M, m, C) ] /n

In equation 3 the rf of a high mobility amino acid are summed and added to the result of equation 2.

-    Low mobility amino acids Rf = {RNDcQEGHKSsty},
-    High mobility amino acids Rf = {LVICYFMW}. From this we derive the following equations:

If 54<%lowRf<100 and gmob >0.36 :

$$Rf(i,j) \approx \sum \frac{mob(i)}{size} * \left[ \frac{-0.9(\%lowRf)}{44} + \frac{23.15}{11} \right] + \sum \frac{highRf(j)}{size}$$

if 54<%lowRf<100%:

$$Rf(i) = \sum \frac{mob(i)}{size} * \left[ \frac{-0.9*(\%lowRf)}{44} + \frac{23.15}{11} \right]$$

if gmob>0.36 :

$$Rf(i,j)=\sum\frac{mob(i)}{size}+\sum\frac{highRf(j)}{size}$$

else:

$$Rf(i)=\sum\frac{mob(i)}{size}$$

## II. B. Module RESOLUTION

### A. General features of the Module Resolution.

The second module/component of the computer program is the module responsible for calculation and comparison of these data against a calculated database of all known proteins. A schematic representation of the computer module "Resolution" is illustrated in Figure 3B. In addition protein fragments derived from computational translation of the expressed sequence tag database at the Genbank public database resource are also used by the program to determine or identify a protein. Resolution reads into memory the determined amino acid sequence from the published public database and determines the predicted mobility for the proteins after digestion with the proteolytic enzymes used for the peptide mapping of the unknown protein. Once the idealized values for the known proteins are calculated they are compared against those calculated for the unknown proteins after adjustment for the running conditions of the gel. If the mobility of the peptides from the unknown protein are within 10% of those of the known protein is classified as a potential target and stored for further analysis. If this is not the case the known protein is no longer considered for analysis. The program systematically addresses each protein in the database. In the case where the species of the unknown protein is known, the program initially scans the data base for this species before extending the search to other species (in the absence of a hit). Once the program has isolated a group of potential target proteins, it returns to these close hits and performs extensive analysis. In this case, each and every peptide for a given protein is identified and compared against those observed and corrected. The program analyses each protein identifying known proteins with higher and higher similarity to that of the unknown protein. Resolution subsequently algorithmically subjects each peptide to different post translational modifications to determine if the discrepancies between the known protein and the unknown result from modification of the unknown protein. The types of post translational modification include protein phosphorylation on tyrosine, threonine and serine, glycosylation on serine, threonine, asparagine and hydroxylysine or C- and N-terminal modification by methylation or myristilation or modification of internal lysine by acetylation. The program does not compensate for glycosylation. Resolution also compensates for the possibility that proteins may have been incompletely digested by the proteolytic enzyme resulting in sites which are not cut. These NO Cut sites can also be mapped to be phosphorylated. Proteins which have peptide maps in which more than 99% of peptides display mobility similar to those of the unknown protein are assumed to be identical and given as a correct hit. In the unlikely case that two proteins in the known database show greater than 99% similarity, resolution prompts the user to enter data for peptide mobility derived after digestion of the unknown protein with another proteolytic enzyme (generally chymotrypsin).

The component of the program which identifies the unknown protein is kept within a module termed Resolution. Resolution has four main components, a comparison module, a fitting module, a high stringency search module and a display module, as can be seen from Figure 5.

### B. Detailed description of the module RESOLUTION

#### a) Function Peptide Resolution

The first function of resolution is to read the image and data file generated by resolve. Both the corrected image and idealized peptide mobility tables are loaded. In the second function, resolution compares the peptide mobilities obtained from the experimental sample to the empirical peptide mobilities of all proteins currently available in the public databases. This function of the program reads successive sequences from the database and calculates a mobility table which is then compared with the experimental samples. Initially, the program performs a low stringency scan which compares only the maximal and minimal rF and emobile for each protein in the database to that calculated for the unknown protein. This initial scan does not compensate for changes in peptide mobility resulting from post translational modification. Once this scan process is completed, retained proteins are analyzed more stringently by peptide fitting.

Accordingly, in the comparison process, the Rf values for each peptide generated from a known protein are compared against values for the unknown protein.

In the first step of the comparison process, the values for peptides containing 4 or more amino acids which migrate at the 4 extremes of the TLC plate are compared to the 4 extreme values for the unknown protein. These represent peptides which have (min emob-minRf), (max emob-minRf), (min emob-maxRf), (max emob-maxRf). Proteins which are found to have 2 or more of these values similar within 5% to those values from the unknown protein are retained for further searching. Other proteins which do not achieve these threshold values are rejected. The function of this step is to increase search speed, any combinations of peptides can be used, the calculation of the maximal and minimal values is simply easier. The comparison step can use all peptides derived from the cleavage process including those consisting of single amino acids. Including single amino acid peptides or peptides containing less than 4 amino acids considerably increases the search time and does not significantly increase search accuracy. For this reason it is not advisable. In the second comparison step, the complete peptide profile for proteins which have been retained in the first filter are compared to that obtained for the experimental protein. The peptide mobility arrays for the two proteins are compared and proteins which have greater than 90% peptide mobility similarity across the entire peptide arrays are retained. Iterative cycles are performed until only the protein with the highest number of similar peptides is retained. In cases where a similar number of peptides are found for two proteins, other criteria such as post translational modification, cleavage failure and original protein size are taken into consideration. (See below, section post-translational modifications).

By default, and as mentioned before, the program does not process peptides shorter than 4 amino acids. The user can override this default, although it does not appear to improve either speed or accuracy. One optional component of the low stringency search program allows users to search with peptides that contain specific amino acids. This is useful when peptides are generated from radioactively labeled proteins which have been marked using a specific radioactive amino acid (alanine or leucine for example).

b) Function: peptide fitting.

In this second procedure, the program attempts to fit peptides with aberrant mobilities to the idealized mobility profiles by simulating the effects of post translational modification or enzymatic inaccuracy. The program ignores peptides with correct mobility and sequentially determines the relative emobile and rF for each of the remaining peptides under the following conditions: a) after addition of one or more post translational modifications, b) after simulating a no cut, the failure of the proteolytic enzyme to cut a peptide at the correct site, c) after a combination of a. and b. d) after a wrong cut, the enzyme cuts non specifically or e) a combination of a. and d. The latter two being very rarely necessary. These different combinations are then directly compared to the unclassified peptides to search for alignment. The simulations are performed on the idealized data from the database file. A similar approach substracting these effects, gives the same result.

c) Complete resolution mapping

In a second embodiment, the invention compares peptide mobility arrays for known proteins using the entire peptide array. Proteins having greater than a fixed percentage of similarity (chosen by the user) are retained and classed in order of their percentage similarity with the unknown protein. This embodiment overcomes problems encountered when the unknown protein is incomplete, has undergone limited mutation, is incompletely cleaved or has extensive post-translational modifications.

d) Determining post-translational modifications

In another third embodiment, the invention can identify the state of post-translational modification of a protein. Post-translational modifications involve the addition of phosphate (phosphorylation), acetyl groups (acetylation), myristile groups (myristilation) and methyl groups (methylation). The method does not identify modification due to glycosilation or the addition of sugar residues. These groups are removed by enzymatic treatment of the unknown protein prior to cleavage. Post-translational modification of amino acids changes their chromatographic and electrophoretic properties. These changes affect the electrophoretic and chromatographic mobility of the peptides containing the modified amino acid. These changes have been calculated experimentally and disclosed in Table 2 which is used in the computer assisted calculation of peptide mobility arrays. To identify post-translational modifications, a peptide mobility array search is performed as described above. The unknown protein is identified by the alignment of the majority of the peptides with a known protein. Peptides which migrate correctly (peptide hits) are then subtracted from the protein sequence to generate a pool of peptide misses. The sequence of each peptide miss is scanned to identify the presence or absence of potential sites for post-translational modification (Serine, Threonine and Tyrosine for phosphorylation, lysine for acetylation, leucine for methylation). For each such peptide, the effect on its mobility of introducing each of these modifications and subsequently combinations of these modifications is determined and compared to the mobilities observed for the unknown protein.

### e) Cleavage failure.

In a similar manner, the program simulates the change in peptide mobility resulting from a failure in the cleavage process. Table 3 shows the predicted sites for cleavage, however these may not all be used due to the 3-dimensional morphology of the protein, its state of post-translational modification or other factors. Again the predicted peptide mobility of peptides undergoing incomplete cleavage is determined and compared to that of the observed peptide. The invention also allows for the combined application of these effects (post translational modification and cleavage failure).

Sites of post-translational modification can also be identified after the protein is digested using two different cleavage methods. Under these circumstances the regions of the protein from which peptide misses are derived can be rapidly identified and subject to the recursive process of determining the effects on peptide mobility of post-translational modification of these peptides. Although there is relatively little chance that the two different methods result in a cleavage failure at the same site, peptides can also be probed to determine if a cleavage failure has resulted.

In the case where proteins have been labeled efficiently on residues cleaved by the cleavage reactions for example arginine and lysine residues (for example using radioactive [14C] arginine and lysine) for proteins digested with trypsin, cleavage failures manifest as doubling (or more depending on the number of cleavage failures) in the signal strength for the uncleaved peptide in comparison to the other singly cleaved peptides.

### f) Protein databases

The databases from which the peptide mobility predictions are calculated can be public such as Genbank at the NCB (National Center for Biotechnology Information,), EMBL (European Molecular Biology Laboratory), PIR (Protein Information Resource) University of George Washington and others, or private such as Swiss Prot, Insight Pharmaceuticals Inc. Sequences from any database should be converted to FatsA format for use by the present embodiment of the invention. The present invention uses protein sequences in FastA format for the computer aided comparison although sequences in any format can be used. Nucleic acid sequences can also be used after translation of each codon into its corresponding amino acid sequence according to standard translation tables.

### g) Resolution database

In yet another embodiment, peptide mobility arrays are calculated and stored in the form of a searchable database. This database consists of the peptide mobilities and other characteristics calculated for the known proteins in public and other databases of protein sequences or from nucleic acid sequences after translation. The use of a peptide mobility array database speeds up the search process.

### h) Function: High Stringency Search

If at the end of the scan period, no proteins have been retained, Resolution automatically performs a high stringency search. In this procedure, the program calculates the complete peptide mobility profile for each protein in the database one after the other and compares each peptide profile to that of the unknown protein. Proteins with at least 50% similarity are retained for peptide fitting. The user can modulate this threshold level. To reduce search load, resolution takes into consideration only proteins which have a molecular mass 20 kDa greater or less than the identified molecular mass of the unknown protein and with a pl (protein charge) plus/minus 1 pH unit. This option can be overridden by the user such that the high stringency search is performed against all sequences in the protein database.

Resolution systematically calculates the effect of all potential post translational modification and failures in proteolytic activity ( steps a. to e. mentioned above).

### i) Function: Draw map

After Resolution has identified the protein with the greatest degree of similarity to the unknown protein which was mapped, Resolution viewer draws the two peptide fingerprints overlapped and aligned to the marker peptides. Peptides which align only after addition of a post translational modification or change in the proteolytic activity are for example marked as red (post translational modification), green (enzymatic change) or yellow (a combination of effects).

By double clicking on the peptide spot the predicted sequence for that peptide is displayed.

j) Peptide display

The next process is to display the results of the search. In an embodiment, this takes the form of a computer assisted method in which the peptide profile obtained experimentally is displayed as an image onto which the theoretical peptide mobility profile is merged. In addition, various data about the unknown protein such as its molecular weight (as entered by the user), the potential Pi, method of cleavage, pH of the analytical step and chromatography buffer. For the protein which is identified various data are also displayed for example accession number, theoretical molecular mass, Pi. If post-translational modifications or cleavage failure have been observed or cleavage failure the peptides concerned are highlighted. These images can be stored on a storage device or printed.

k) Comparison using values other than RF. and emob

It is also possible to calculate a variety of other characteristics of the peptides from known proteins and use these to compare against the peptides for the unknown protein. The program also calculates a number of other physicochemical characteristics of the peptide including its mass, theoretical retention time on an HPLC column using C14 resin at pH 7.4 and pH2.1, the acidity of the peptide, how basic the peptide is, its aromatic index, hydrophobicity and hydrophilicity. Calculations involve determining the mean value for a given peptide based on a summation of the values for each amino acid in the peptide divided by the amino acid number.
As an example for Retention at pH2.1:

$$\frac{\text{Retn2.1(aa1)} + \text{Retn2.1(aa2)} + \text{Retn2.1(aa3)} + .... \ \text{Retn2.1(aaN)}}{N}$$

Where N is the number of the last amino acid in the peptide. In another embodiment, the retention values on an HPLC for the peptides from the unknown protein could be measured at two different pH values (2.1 and 7.4) and calibrated using peptides of known characteristics. Subsequently these values could be used in a comparison against the retention values determined for peptides in the databases.

l) Database Generation Module

The final module allows users to generate a local copy of the Resolution database. Instead of reading in a protein sequence database and searching for peptide similarities on the fly, Resolution can put the coordinates into a file. The format of the Resolution coordinate files is the same as that of the text component generated with the Resolution image file.

m) Identification of peptides from a known protein

This brings us to the final part of the application which is the use of this data to identify unknown proteins on the basis of the mobilities of their composite amino acids. This is simply a scale up of the proof of concept using peptides of known composition. The first example provided is a human protein which we have isolated as a homogeneous spot by standard 2-dimensional electrophoresis. The spot was identified by the immunoblotting of an identical gel as the protein vimentin. The protein used in the example was radioactively labeled by growing the cells in the presence of [35S] labeled methionine an essential amino acid which is incorporated into most proteins. Any amino acid could be used, but since [35S] is a relatively strong beta its use shortens the length of the experiment in comparison to using [14C] labeled amino acids. Using methionine decreases the numbers of peptides we anticipate analysing (since not all peptides contain this amino acid). We firstly identified the peptides in vimentin using the known sequence for this protein which is in all the major protein databases including Genbank, PIR and Swiss-prot. Of the 10 potential methionine containing peptides (without modifications) derived from vimentin which contain methionine, we have unambiguously identified all of them. In addition we identified a number of changes in mobility derived from either post translational modifications or enzyme cleavage failures (no cuts) since the peptide map has 26 methionine containing spots. The remaining peptide is probably subject to a post-translational modification which we do not currently cater for. Searching the primate database from Genbank release 130 (June 2002) the program identified vimentin as the most statistically probable protein. The program searched the data base only looking for mobilities of methionine containing peptides. The program uses both forward and reverse no-cuts.

The present invention is further illustrated by, without in any way being limited to, the following examples.

## EXAMPLES

### Example 1 : Illustration of the calculation of the netcharge of a protein.

A specific illustration of the calculation of the netcharge of a protein of a known amino acid sequence is given herein, for the test protein possessing the following amino acid sequence : NH2-YVAHLR-COOH.

The overall value of netcharge is derived from:

$$\text{netcharge} = (\text{netplus} - \text{netminus})$$

Where:

$$\underline{netplus} = \Sigma \left\{ N' \times \left( [H^+] / 10^{x(-pKs)R} + [H^+] \right) + N'' \times \left( [H+] / 10^{x(-pKs)H} + [H+] \right) + N''' \times \left( [H^+] / 10^{x(-pKs)K} + [H^+] \right) + 1 \times \left( [H^+] / 10^{x(-pK1)} + [H^+] \right) \right\}$$

$$\underline{netminus} = \Sigma \left\{ N' \times (1 - ( [H^+] / 10^{x(-pKs)Y} + [H^+] )) + N'' \times (1 - ( [H+] / 10^{x(-pKs)E} + [H+] )) + N''' \times (1 - ( [H^+] / 10^{x(-pKs)D} + [H^+] )) + N^{-s} \times (1 - ( [H^+] / 10^{x(-pKs)s} + [H^+] )) + N^{-t} \times (1 - ( [H^+] / 10^{x(-pKs)t} + [H^+] )) + N^{-y} \times (1 - ( [H^+] / 10^{x(-pKs)y} + [H^+] )) + 1 \times (1 - ( [H^+] / 10^{x(-pK2)} + [H^+] )) \right\}$$

Where N' is the number of arginine residues in the peptide, N" is the number of histidines, N''' is the number of lysines, N-' is the number of tyrosines, N''' the number of glutamines, N"" the number of glutamic acid residues, N-s the number of phospho-serines, N-t the number of threonines and N-y the number of phospho-tyrosines. (pKs)R is the pKa for arginine, (pKs)H the pKa for histidine, (pKs)K the pKa for lysine, (pKs)Y the pKa for tyrosine, (pKs)E the pKa for glutamine, (pKs)D the pKa for glutamic acid, (pKs)s the pKa for phosph-serine, (pKs)t the pKa for phospho-threonine, (pKs)y the pKa for phospho-tyrosine, pK1 is the pKa of the amino terminal amino acid and pK2 the pKa of the carboxy terminal amino acid.

The example given in this document would give:

$$\underline{netplus} = \Sigma \left\{ 1 \times \left( [H^+] / 10^{x(-pKs)R} + [H^+] \right) + 1 \times \left( [H+] / 10^{x(-pKs)H} + [H+] \right) + 1 \times \left( [H^+] / 10^{x(-pK1)} + [H^+] \right) \right\}$$

$$\underline{netminus} = \Sigma \left\{ 1 \times (1 - ( [H^+] / 10^{x(-pKs)Y} + [H^+] )) + 1 \times (1 - ( [H^+] / 10^{x(-pK2)} + [H^+] )) \right\}$$

since the values for N''', N-", N-''', N-s, and N-t are 0. Such that at pH 1.9: netplus = S {0.0125/

### Example 2 : Cell culture and metabolic labeling

Rat embryo fibroblasts and human fibroblasts were cultured and microinjected as described previously. Cells growing on glass coverslips were microinjected and subjected to metabolic labeling with [32P] essentially as described previously (Lamb et al., 1989). For peptide mapping, cells were cultured for 36 hours in 10 uCi[14C]. Alanine plus 10uCi [14C] leucine. Cells were subsequently microinjected with kinases and allowed to continue for 30-60 minutes before isolation and analysis as described before.

### Example 3 : Two dimensional electrophoresis

Cells growing on 2mm$^2$ glass chips analyzed by 2D gel electrophoresis as described before.
Essentially, cells growing on chips were washed with PBS to remove excess label before transfer into 10 $\mu$l of lysis buffer (1% NP40, 50 mM Tris pH 6.8). Cells were lysed by the addition of 2 $\mu$l 5X SDS sample buffer (50 mM Tris pH 6.8(NaOH), 5 mM DTT, 0,5 % (w/v) SDS), vortexed extensively and boiled at 98°C for 5 minutes in the presence of 50 $\mu$l of cell extract produced by harvesting a 60 mM dish in 250 $\mu$l PBS and resuspending the resulting cell pellet after centrifugation in 500 $\mu$l of 1X SDS sample buffer. After boiling samples were lyophilized, resuspended in DNA/Rnase (1.0 mg/ml in 0.1 X PB5) and after incubation at RT for 5 minutes diluted into 100 $\mu$l of IEF sample buffer.
Samples were analyzed by electrophoresis on a Pharmacia 1PGphor. After a first dimension on ampholyte slips according to the manufacturers instructions, samples were resolved in the second dimension on 15% SDS PAGE gels. Gels were dried, exposed for 50 minutes on an INSTANT-IMAGER (Packard Instruments Inc., Palo Alto, CA) and spots corresponding to those which underwent increased phosphorylation isolated from the gel.

### Example 4 : Peptide mapping

Spots to be identified were excised from the dried gels after autoradiography and extracted from the gel by digestion with trypsin or chymotrypsin as described previously (Lamb et al. 1989). Peptides were subject to repeated cycles of lyophilization and rehydration to remove ammonium bicarbonate and resuspended in water before addition of 10 ng of 6 marker peptides, then spotted onto MERCK HPTLC cellulose plates. Samples were electrophoresed at pH 1.9 for 30 minutes and separated by chromatography in P-Chromo buffer (Kitzmann et al. 1999) in the perpendicular axis until the front reached the upper plate edge. After drying to remove solvent, radio labeled peptides were visualized by autoradiography of the thin layer chromatography plate. Peptide maps were acquired either using a phosphorimager (Storm Molecular Dynamics Inc., Mountain View , CA) or Instant Imager and images transferred to a SGI Iris Indigo 2, deconvoluted using Tifflmager.

### Example 5 : Resolution analysis of peptide fingerprints

Images of radioactive peptide fingerprints were acquired into the analysis software using Resolve. Briefly, images are read into Resolve and the user subsequently aligns the 6 peptide markers to the grid provided. Resolve subsequently caculates the differences in absolute and predicted mobility for these peptide markers and determines one or more correction ratio(s). Subsequently the position of each peptide from the sample is identified using a spot recognition algorithm, and the appropriate correction ratio applied to the peptide with respect to the nearest peptide marker. The corrected mobility for each peptide is stored in a specific "res" file.

### Example 6 : Resolution Module

To identify unknown proteins, the observed corrected peptide fingerprint is compared to the peptide profiles of each protein in the database. The program Resolution can run in two modes stringent and high speed which differ significantly in their rate of execution and most commonly we anticipate the high speed versions will be sufficient. Briefly, in the high speed version, primary amino acid sequences from both Genbank Genpept database. Swiss prot and PIR are subject to *in silico* proteolysis and the relative electrophoretic and chromatographic mobility determined empirically. These empirical mobilities are compared to the unknown protein mobility profile. Peptide fingerprints having greater than 50% homology are stored for more detailed analysis. For speed, the initial analysis compares the most extreme peptides from the *in vivo* data (max rfo-min Ec, mm rto-max Ec, max rfo-max Ec) against the same values for a database sequence. Profiles showing similarities for the extreme values are subsequently reanalyzed comparing each peptide mobility. Profiles with increasing similarities are kept until either a single sequence is retained or profiles having greater than 85% similarity are identified. At this point peptides with aberrant mobility are reanalyzed compensating for the effects on mobility of potential post translational modification or a failure in proteolytic digestions. The program empirically calculates the effects of phosphorylation, acetylation, myristilation, and methylation or the consequences of the proteolytic enzyme failing to digest at a specific site on peptide mobility are determined as well as combinations of these effects. Finally, if a peptide falls to align, the program empirically tests every combination of event including complete loss of enzyme specificity. The high stringency method essentially applies the systemic analysis to each protein sequence. However, the high stringency approach requires users to input the molecular mass obtained by 2D electrophoresis and the approximate pKa of the protein (determined from the first dimension electrophoresis). These values are used by the program to rapidly eliminate proteins with an empirical mass which differs by more than 10 kDa from the unknown protein and a pKa which differs by more than 1.5 pH units above or below.

### Example 7 : Determination of the set of theoretical localisation values for a peptide of a known amino acid sequence

In the previous examples, we examined if the method could be used to identify the peptides derived from a short 20 amino acid peptide of which the sequence is known. A digitized map of a two-dimensional protein separation of a human protein extract is shown in Figure 6. The boxed part in Figure 6 indicates the surface area containing human vimentin. This peptide is digested *in vitro* and resolved by 2D TLC. In the following example we calculate the electrophoretic and chromatographic mobilities of 5 peptides derived from the digestion of a peptide of known composition. The peptide was taken from the regulatory site of human cdc2 (cdk1 Nurse 1997) and has the sequence TKIEKIEKIGEGTYGVVYKGRHC [SEQ ID N°1]. Peptides were synthesized by Neosystems (Strasbourg France) in three forms, the native peptide, the same peptide with a chemically phosphorylated Threonine at position 14 TKIEKIEKIGEGtYGWYKGRHC or Tyrosine at position 15 TKIEKIEKIGEGTyGVVYKGRHC. The phosphorylated amino acids are denoted by lower case letters.

The peptide was digested with trypsin and subject to 2 dimensional peptide mapping as described elsewhere (Kitzmann et al 1999) with the exception that two landing light peptides of known composition were added to the mixture just prior to spotting on the TLC plate. In this case the peptides were (YPYDVPDYA) [SEQ ID N°2] (HA) a peptide sequence corresponding to a region of the Haempholis influence virus A hemaglutinin prepropeptide (Genbank accession number J02135, now AAA43189.1). The second peptide (EQKLISEEDL) [SEQ ID N°3] derived from the human c-myc protein (Genbank accession number M38057). These peptides are not digested by trypsin, however other peptides could be used even those containing sites for digestion by trypsin since, by adding them at the last moment before electrophoresis, the trypsin does not have time to effect digestion. After separation, peptides were visualized by ninhydrin staining as described by the manufacturer (Sigma, St. Louis Missouri) according to procedures standard to those familiar with the art. Plates were photographed after the major spots became visible and the digitized image analyzed to identify the position of the maximal signal for the different spots.

Many commercial image analysis software packages can be used for this function however we used our own program developed to be optimized for the identification of the boundaries of the spots and the position of the maximal intensity of the spot. The contour and pixel peaks were determined each spot to ensure only a single spot was associated for each peptide. The peak for each peptide is determined as the point of maximal pixel density. This identification could also be performed manually.

The results are given in Tables 4 and 5 below as well as in Figure 8.

Correction factors averaged from mobility of the marker peptides are 0.000094 u/pixel for emobile and 0.00152 u/pixel for rF.

The digitized map for the digested peptides is given in Figure 8A. After correction this map was used to generate the coordinate map which was then used in comparison with the peptide mobilities estimated from the peptide sequence to give the result shown in Figure 8B. From this map and coordinates we can determine that there are two peptides which run aberrantly. None of the peptides observed run at the coordinates predicted for peptide 2 (shown as the dark circle in figure 8B). However one peptide runs with coordinates 0.0429 and 0.0491 which correspond to those of a peptide resulting from a cleavage failure at lysine 2 giving rise to the peptide TKIEK. Thus, under these conditions peptide 2 is not seen or seen only as a no cut with peptide 1. When the phospho-isoform of this cdc2 peptide modified at either threonine 14 or tyrosine 15 was used, a variant of peptide 3 was observed in addition to the wild type form which showed both lower electrophoretic and chromatographic mobilities (data not shown). From these data it is clear that for a simple peptide sequence, cleaved by an enzymatic process, we can predict the finite mobilities of peptides using the method given above.

### Example 8 : Identification of an "unknown" protein contained in a sample

### A. Methods

Human vimentin was made radioactive by growing primary human fibroblasts in the presence of medium containing [35S] methionine. After separation by 2D PAGE, the methionine labeled vimentin spot was excised from the gel, digested with trypsin and subjected to 2D tryptic peptide mapping. The autoradiograph of the vimentin containing peptides was digitized and after calibration using 2 peptides of known sequence, pixel coordinates were calibrated as relative electrophoretic mobilities and chromatographic mobilities. These values were compared to those of the methionine containing peptides of vimentin. Out of 13 potential peptides, 6 were immediately identified from their mobility. Subsequently, we compared the values obtained against a database containing peptide coordinates for primate sequences. This data again lead to the identification of a single polypeptide sequence with these exact coordinates, that of human vimentin.

Human MRC5 cells were grown on 35 mm petri dishes under standard conditions (Girard et al 1991). Cell were grown in the presence of 200 uCi of [$^{35}$S]-methionine for 24 hours. Cells were washed twice with PBS before lysis in 200 ul of lysis buffer (50mM Tris pH6.8, 10mM DTT, 5% glycerol, 1.0%SDS). After boiling for 5 minutes to inhibit protease activity and denature proteins, cells were disrupted by passage through a 26 gauge needle. Proteins were precipitated by the addition of 9 volumes of ice cold acetone and collected by centrifugation at 15000g for 10 minutes. Protein pellets were resuspended in 100 ul of IEF sample buffer (9.0 M urea, 50mM DTT, 10% NP40) and proteins separated by 2D electrophoresis as described elsewhere (Girard et al 1991). After fixation in 50% methanol, 20% acetic acid 30% water, the gel was dried on Whatman paper by vacuum and exposed on film by autoradiography.

The radioactive protein was digested using trypsin as described elsewhere (Kitzmann et al 1999, Hunter et al., 1991). Essentially, the region of the gel corresponding to the radioactive spot pointed by the arrow was excised and digested in 1 % TCPK treated trypsin in 200 ul of 200 mM ammonium bicarbonate in water pH 4.5 for 36 hours. The solution containing the peptides was transferred to a new Eppendorf tube and subject to 3 cycles of lyophilization to remove the ammonium bicarbonate. Peptides were resuspended in (Formic acid, 88%), acetic acid, water, 2.5:7.8:89.7) and spotted on a TLC plate 1.0 cm from the edge of the plate. Peptides were separated by electrophoresis for 15 minutes at 2900 volts. After drying to remove excess acid, the plate was placed in a humidified tank containing 0.5 cm of Pchromo buffer (butano, pyridine, acetic, acid, water, 35:25:7.5:30) and the front allowed to migrate to within 0.5 cm of the upper edge of the plate.

Following chromatography, the plates were marked with radioactive ink for subsequent alignment between images of the non radioactive marker peptides and the peptides identified by radioactivity. Plates were marked at the edges outside the area in which peptides were resolved. Any asymmetric form could be used as a mark up but we in general mark the plates with X's at three or four places using china ink containing 1uCi/ml tritiated glucose. Any radioactive element or ink could be added to the ink since its purpose is to make the X's visible on the autoradiograph and the X visible on the standard photographic image. Alternatives include using radioactively labeled marker peptides or the use of an auto-fluorescent ink. After markup, plates were air dried and stained with ninhydrin reagent to visualize the position of the landing light peptides. The plate was photographed using a Kodak DCS420 camera to identify the position of the landing light peptide, the X's, the plate edges and the origin of migration. After heating and fixation, plates were exposed using a Kodak Xmax and Xmax film for 2 days. The film was photographed using the same method as above. Alternatively, the radioactive image could be obtained using a phospho-imager or instant imager or similar such non film based technology. The resulting autoradiograph of the radiolabeled peptides was used for the quantification. Two images of the plate stained with ninhydrin and the autoradiograph were superimposed (the autoradiograph was photographed using the camera at the same position as the image of the plate). One of the simplest methods for this procedure involves using a standard image processing package such as Adobe photoshop or GIMP (Gnu ImageProcessing package). The first step involves cropping the image to the exact size of the TLC plate. Subsequently the identically sized images are each entered as a layer and after alignment, the two images are integrated using the merge layers function. A perfect overlap can be achieved in almost every case. The resulting image was analyzed to identify the maximal signal to background value for each peptide. Such analysis included a water shed analysis to define the contours of each spot and identify where two or more peptides were in close proximity and if a spot consisted of one or more peptides. The maximal pixel value for each spot was used to position where the majority of peptides run for a given spot. The maximal peak density rarely corresponded to the center of the spot. The resulting digitised image is shown in figure 10A.

B. Calculating pixel emobility and pixel Rf mobilities

The next step involves calculating the actual distance that each peptide has run with respect to the origin. This is calculated by substracting for each peptide the distance of the origin from the edge of the plate from the total distance run in emobile by the peptide. A similar calculation is made for Rf.

C. Calculating pixel/emobnil and pixel/Rf values.

In the next step each pixel is attributed an emobile/pixel and Rf/pixel ratio. The exact electrophoretic mobility values for the two (or more) marker peptides are known. The theoretical emobil for the marker peptide is divided by the distance from the origin to the peak of the marker in pixels.

## Emobility/pixel = emobility(theoretic)/pixels from origin to peak.

This is carried out for each marker peptide and should give the same value. A similar calculation is made for the Rf values. Subsequently for each unknown peptide the origin to peptide peak in pixels is converted to emobility and Rf values by multiplying the observed pixel distances by the respective conversion factors.

The same calculations can be made from the edge of the plates. However, a paper wick covers an undefined area on each side of the plate (such that the current can flow) and it would be necessary to mark the edge of this wick each time if the exact electrophoretic origin were to be used. A similar problem exists in the chromatographic step since the plate is immersed into a buffer of some depth. It is therefore more convenient to use the origin.

From this value in pixel-emobil and pixel-rf the distance from the origin to the two marker peptides was calculated by subtracting the origin from the observed x-origin-emobile and y-origin-rF for each marker peptide. The resulting distances in pixels were used to calculate the pixel to emobile ratio by simply dividing the calculated emobile for each marker peptide by the number of pixels. From this, four values were determined (emobile pep1, emob pep2, rF pep1 and rF pep2) Both pairs of values are very close and in the present example are x-avg. 0.0000585 and y-avg. 0.0014 which also confirms that the separation steps have correctly functioned. The digitized image with the position of the marker peptides is shown in figure 10B. The image was digitized using the Spotfinder option of the program and the results are given below. An example of the raw data generated from the program is given in Table 6. The calculated corrected coordinates (emobile and Rf) are given below.

List of detected spots

Spot #M1: (0.017,0.528)   Marker peptide 1
Spot #A: (0.017,0.476)
Spot #B: (0.021,0.504)
Spot #C: (0.022,0.571)
Spot #D: (0.017,0.675)
Spot #E: (0.017,0.707)
Spot #F: (0.02,0.374)
Spot #G: (0.036,0.365)
Spot #H: (0.028,0.295)
Spot #I: (0.012,0.546)
Spot #M2: (0.007,0.774)   Marker peptide 2.
Spot #J: (0.017,0.769)
Spot #K: (0.012,0.588)
Spot #L: (0.017,0.594)
Spot #M: (0.016,0.323)

The attribution of the spots is done on and arbitrary basis in the case of this example starting from the spot closest to the center. However, any other method could be used since the precise numerical attribution is not important. The correspondence between the spots and the numbers above is given in figure 10C.

The next step in the process is to calculate the theoretical emobil and Rf values for the protein used in the digestion. In this example we entered the poly-peptide sequence for human vimentin (Genbank accession M14144) into the program peptFinder module and the theoretical coordinates for emobile and rF calculated under the same experimental conditions as used for the digestion. The resulting theoretical peptide coordinates were selected only for peptides containing methionine (other peptides would not be detected under these experimental conditions). Vimentin when digested in this manner using trypsin would generate 62 peptides of which 9 are single amino acids, 6 are di-amino acids none of which contain methionine. Of the 47 peptides remaining 10 contain methionine, i.e. 21 %. The coordinates obtained from the experimental analysis revealed 16 methionine containing peptides (listed above) since 2 correspond to the migration of the peptide markers. The theoretical 2D peptide map for vimentin is shown in figure 10D. The position of the two marker peptides is also displayed on the image since these values (up to 4) can be entered into the program manually at this stage. The marker peptides are not displayed as digested since as mentioned above, they do not contain arginine or lysine and secondly the trypsin is autodigested.

44

The theoretical calculations for emobil and Rf for vimentin peptides containing methionine are given below with the exact output from the program given in Table 7.

D. Alignment

Once the theoretical map and physical map of the coordinates are known, the subsequent procedure is to identify the spots which show the closest coherence between the theoretical values for the peptide and those observed. For some spots there is already a perfect alignment since the peptides are not subject to any form of cleavage failure or post translational modification. This is the case for 2 peptides for vimentin. The method for alignment involves calculating for each peptide the complete list of co-ordinates it may have after phosphorylation, oxidation etc. The best matching theoretical and observed values are then retained. The alignment can be summarized as follows.

In order to identify the other peptides the theoretical mobilities for each peptide is calculated taking into account the post translational modifications to which it may have been subject (in this case phosphorylation), the possibilities that a cleavage failure has occurred or finally that the methionine (or cysteine if the peptide contained both) has been incompletely oxydized by the treatment before analysis. A subsequent filter was applied which instigated combinations of these events i.e. no cut and phosphorylation and finally complete combinations. The results are shown in Tables 8--9.

For each set of combinations the resulting mobilities were compared to those of the observed peptides and the peptides showing the closest alignments identified. At this point a statistical filter can be applied in order to identify the peptides which have the most significant similarity between the observed and calculated rF and emobile. As shown in figure 10D, there is a very close similarity between the observed and calculated values for most of the peptides. In the example below, peptides were scored on a range 1-450, those with the higher scores being closest to the observed value. Peptides with scores above 70 are considered to be identified and those above 200 the theoretical maxima for the peak calculated for the peptide in within 2-3 pixels of the observed peak.

On the basis of these data we have identified all the spots observed after digestion of [$^{35}$S]methionine labeled vimentin, the data are given in Table 10. From these data is it clear this method enables the prediction of the electrophoretic and chromographic properties of peptides. With this knowledge it is possible to identify peptides from a given protein on the basis of these characteristics.

E. Extension to identifying unknown proteins

In a further extension of this method, a peptide fingerprint obtained from the digestion of an unknown protein could be used to identify the unknown protein provided that the sequence of the protein has already been entered into a database. This essentially involves extending the method described above such that instead of comparing the peptide co-ordinates with a single sequence, they are confronted with any number of sequences in a database. The process is essentially the same as that used for vimentin with the following exceptions: Firstly, instead of determining the theoretical rF and emobile values for a single protein sequence, the program generates these values for all the proteins in a database. These data can either be stored as a file of coordinates in the form of a database or calculated on the fly for each sequence in a file containing multiple sequences. For the second difference, the program compares the coordinates for each sequence with those observed by TLC and scores the number of peptides which align perfectly with those of the observed TLC. Proteins are ranked by the increasing numbers of perfect alignments with the closest matching protein having the most positive hits. In the case of methionine labeled proteins this would be the peptide number as a percentage of the peptides containing methionine.

Finally, we examined if this computational method could be used to screen a database comprising the known poly-peptide sequences from primates collated by the National Center for Biotechnology Information (NCBI) and comprised in the Genbank Primate sequence databank. Accordingly, the Genbank primate amino acid sequences (presently 99704 as of the Genbank 137 release, Aug. 15 2005) were extracted from the Genbank flat files using gt2fasta (NCBI-toolbox 1995) and stored in a single file in fasta format (Pearson 2000). The file was processed using Resolutions peptFinder program and the emobil and rF for methionine containing peptides from each sequence compared to those observed experimentally.

Using the peptide coordinates obtained for human vimentin, we searched a database of the human protein sequences (93580 sequences). We resolved 1732 sequences which contained peptides which had mobilites equal to the maximal and minimal values identified by TLC which was refined to 443 sequences when only methionine peptides were considered. Application of the reiterative search for missed cleavages, phosphorylation and oxidation reduced the number of sequences to 15 of which 3 showed complete alignment with all 14 peptides. These sequences were M14144X56134 and X16478.

On the basis of these data, it is clear that from the accurately determined coordinates of an unknown protein calibrated by 2 or more peptides of a known sequence, peptides of a known protein can be identified and using a database of known peptide sequences peptides of an unknown protein may be identified.

## Table 1

| Name | Three letter code | One letter code | Mw | pK$_R$ |
|------|------|------|------|------|
| Alanine | Ala | A | 71 | - |
| Cysteine | Cys | C | 103 | 8,3 |
| Aspartic acid | Asp | D | 114 | 3,9 |
| Glutamic acid | Glu | E | 128 | 4,3 |
| Phenylalanine | Phe | F | 147 | - |
| Glycine | Gly | G | 57 | - |
| Histidine | His | H | 137 | 6 |
| Isoleucine | Ile | I | 113 | - |
| Lysine | Lys | K | 129 | 10,8 |
| Leucine | Leu | L | 113 | - |
| Methionine | Met | M | 131 | - |
| Asparagine | Asn | N | 114 | - |
| Proline | Pro | P | 97 | - |
| Glutamine | Gln | Q | 128 | - |
| Arginine | Arg | R | 157 | 12,5 |
| Serine | Ser | S | 87 | 13 |
| Threonine | Thr | T | 101 | 13 |
| Valine | Val | V | 99 | - |
| Tryptophan | Trp | W | 186 | - |
| Tyrosine | Tyr | Y | 163 | 10,1 |

## TABLE 2

Values used to calculate the net charge, molecular mass, retention times on HPLC C18 columns and the electrophoretic and chromatographic mobilities in three commonly used buffers. Rf are under non modified conditions whereas pf are after addition of phosphate to serine, threonine or tryosine.

Hydrophobicities assigned according to J. Janin Nature 277:492 (1979)

| AmAcid | MolWt | Charge | Aroma | Acid | Base | Sulfur | Phil | phob | retn2.1 | retn7.4 | Rf-reg | Rf-P.chr | Rf-Iso | pf-reg | pf-P.chr | pf-Iso |
|------|------|------|------|------|------|------|------|------|------|------|------|------|------|------|------|------|
| Ala | 89.09 | 0 | 0 | 0 | 0 | 0 | 0 | 1 | -0.1 | 0.5 | 0.35 | 0.41 | 0.61 | | | |
| Arg | 174.20 | 1 | 0 | 0 | 1 | 0 | 0 | 1 | -4.5 | 0.8 | 0.20 | 0.33 | 0.69 | | | |
| Asn | 132.12 | 0 | 0 | 0 | 0 | 0 | 1 | 0 | -1.6 | -0.8 | 0.15 | 0.25 | 0.44 | | | |
| Asp | 133.10 | -1 | 0 | 1 | 0 | 0 | 1 | 0 | -2.8 | -8.2 | 0.14 | 0.25 | 0.46 | | | |
| Cys | 121.15 | 0 | 0 | 0 | 0 | 1 | 0 | 1 | -2.2 | -6.8 | 0.08 | 0.45 | 0.23 | | | |
| CysOx | 137.14 | | | | | | | | | | | 0.25 | | | | |
| Gln | 146.15 | 0 | 0 | 0 | 0 | 0 | 1 | 0 | -2.5 | -4.8 | 0.20 | 0.32 | 0.51 | | | |
| Glu | 147.13 | -1 | 0 | 1 | 0 | 0 | 1 | 0 | -7.5 | -16.9 | 0.22 | 0.33 | 0.52 | | | |
| Gly | 75.07 | 0 | 0 | 0 | 0 | 0 | 0 | 1 | -0.5 | 0.0 | 0.23 | 0.33 | 0.50 | | | |
| His | 155.16 | 0 | 0 | 0 | 0 | 0 | 1 | 0 | 0.8 | -3.5 | 0.19 | 0.33 | 0.67 | | | |
| Ile | 131.17 | 0 | 0 | 0 | 0 | 0 | 0 | 1 | 11.8 | 13.9 | 0.76 | 0.71 | 0.92 | | | |
| Leu | 131.17 | 0 | 0 | 0 | 0 | 0 | 0 | 1 | 10.0 | 8.8 | 0.81 | 0.73 | 0.95 | | | |
| Lys | 146.19 | 1 | 0 | 0 | 1 | 0 | 1 | 0 | -3.2 | 0.1 | 0.14 | 0.27 | 0.58 | | | |
| Met | 149.21 | 0 | 0 | 0 | 0 | 1 | 0 | 1 | 7.1 | 4.8 | 0.26 | 0.62 | 0.59 | | | |
| MetOx | 165.20 | | | | | | | | | | | 0.45 | | | | |
| Phe | 165.19 | 0 | 1 | 0 | 0 | 0 | 0 | 1 | 13.9 | 13.2 | 0.77 | 0.71 | 0.93 | | | |
| Pro | 115.13 | 0 | 0 | 0 | 0 | 0 | 1 | 0 | 8.0 | 6.1 | 0.40 | 0.46 | 0.73 | | | |
| Ser | 105.09 | 0 | 0 | 0 | 0 | 0 | 1 | 0 | -3.7 | 1.2 | 0.25 | 0.35 | 0.48 | | | |
| pSer | 184.00 | -1 | 0 | 1 | 0 | 0 | 1 | 0 | | | | | | 0.25 | 0.20 | 0.35 |
| Thr | 119.12 | 0 | 0 | 0 | 0 | 0 | 1 | 0 | 1.5 | 2.7 | 0.34 | 0.41 | 0.63 | | | |
| pThr | 198.07 | -1 | 0 | 1 | 0 | 0 | 1 | 0 | | | | | | 0.34 | 0.23 | 0.43 |
| Trp | 204.23 | 0 | 1 | 0 | 0 | 0 | 0 | 1 | 18.1 | 14.9 | 0.69 | 0.67 | 0.72 | | | |
| Tyr | 181.19 | 0 | 1 | 0 | 0 | 0 | 0 | 1 | 8.2 | 6.1 | 0.64 | 0.62 | 0.72 | | | |
| pTyr | 260.16 | -1 | 0 | 0 | 0 | 0 | 0 | 1 | | | | | | 0.64 | 0.28 | 0.53 |
| Val | 117.15 | 0 | 0 | 0 | 0 | 0 | 0 | 1 | 3.3 | 2.7 | 0.56 | 0.59 | 0.82 | | | |

46

### Table 3 : List of proteolytic enzymes

| Enzyme Name | Cuts at resudue | Cuts which residue | Full Name |
|---|---|---|---|
| TrypK | 1 | K'-P | Trypsin(Arg blocked) |
| TrypR | 1. | R'-.P | Trypsin(Lys blocked) |
| Chymo | 1 | (F,W,Y,M,L)'~P | Chymotrypsin |
| Staph | 1 | E' | Staphylococcal Protease |
| V8 AmoniumAcetate | 1 | E'~P | Staphylococcus Aureus V8 |
| NoCut | 0 | | Calculations on whole peptide |
| Thermolysin | 0 | (L,I,M,F,W,V) | Thermolysin |
| CnBr | 1 | M | Cyanogen Bromide |
| Clos | | R | Clostripain |
| AspN1 | 0 | D | Endoproteinase Asp-N |
| ProEn | 1 | P | Proline Endopeptidase |
| Pancreatic Elastase | 1 | (A,G,S.V)' | |
| Armillaria Mellea | 0 | K | |
| NTCB | 0 | C | NTCB + Ni |
| NH$_2$OH | 0 | N'G | Hydroxylamine |
| Myxo | 1 | K | Myxobacter Protease |
| LysC | 1 | K | Lys-C, Endoproteinase |
| IBzO | 1 | W | IodosoBenzoate |
| Acid | 1 | D'P | pH 2.5 |
| 0 means enzyme cuts at the amino side of the residue, 1 means carboxy. | | | |

### TABLE 4

| | Table 4 : Spot coordinates were as follows | | | | | |
|---|---|---|---|---|---|---|
| | Y coordinate Pixels | X coordinate Pixels | Y coordinate Pixels from 0 | X Coordinate Pixels from 0 | Y corrected RF | X corrected Emobile |
| Origin | 110 | 128 | 0 | 0 | | |
| Marker 1 | 606 | 202.4 | 496 | 74.4 | 0.754 | 0.007 |
| Marker 2 | 452 | 314 | 342 | 186 | 0.52 | 0.0175 |
| Pep 1 | 657 | 285 | 547 | 157 | 0.831 | 0.0147 |
| Pep 2 | 425 | 579 | 315 | 451 | 0.472 | 0.0423 |
| Pep 3 | 356 | 650 | 246 | 520 | 0.373 | 0.0488 |
| Pep 4 | 337 | 668 | 227 | 540 | 0.345 | 0.0507 |
| Pep 5 | 325 | 694 | 215 | 562 | 0.331 | 0.0528 |

## TABLE 5

| Digestion by TrypKR et pH 1.9 | | Number of peptides #5 | |
|---|---|---|---|
| ----------Peptide#1---------- | | ----------Peptide #4---------- | |
| Sequence: (TK) | | Sequence: (GR) | |
| Composition: K-1 T-1 | | Composition: G-1 R-1 | |
| Longueur: | 2 | Longueur | 2 |
| Molweight: | 247.294 | Molweight: | 231.255 |
| Mass : | 247.153 | Mass: | 231.133 |
| NetCharge: IsoElecPoint: | 1.979 9.676 | NetCharge: IsoElecPoint: | 1.979 10.529 |
| MobElec: | 0.050 | MobElec: | 0.053 |
| MobChro: | 0.340 | MobChro: | 0.330 |
| Rtn2.1: | -1.700 | Rtn2.1: | -5.000 |
| Rtn7.4: | 2.800 | Rtn7.4: | 0.800 |
| ----------Peptide#2---------- | | ----------Peptide #5---------- | |
| Sequence: (IEK) | | Sequence: (HC) | |
| Composition: E-1i-1 k-1 E-1i-1 K-1 Longueur. | 3 | Composition: C-1 H-1 C-1 H-1 Longueur | 2 |
| Molweight: | 388.464 | Molweight : | 258.301 |
| Mass : | 388.232 | Mass: | 258.079 |
| NetCharge: | 1.974 | NetCharge: | 1.979 |
| IsoElecPoint: | 6.446 | IsoElecPoint | 7.289 |
| MobElec: | 0.037 | MobElec: | 0.049 |
| Mobchro: | 0.666 | MobChro: | 0.390 |
| Rtn2.1: | 1.100. | Rtn2.1: | -1.400 |
| Rtn7.4: | -2.900 | Rtn7.4: | -10.300 |
| ----------Peptide#3---------- | | ----------No-Cut Peptide#1+2----- | |
| Sequence: (IGEGTYGWYK) | | Sequence: (TKIEK) | |
| Composition: | | Composition: | |
| Longueur | 11 | Longueur | 5 |
| Molweight: | 1165.343 | Molweight: | 617.744 |
| Mass: | 1184.808 | Mass: | 617.375 |
| NetCharge: | 1.974 | NetCharge: | 2.974 |
| IsoElecPoint: | 6.440 | IsoElecPoint | 9.513 |
| MobElec: | 0.018 | MobElec: | 0.041 |
| MobChro: | 0.751 | MobChro: | 0.491 |
| Rtn2.1: | 24.100 | Rtn2.1: | -0.600 |
| Rtn7.4: | 17.4 | Rtn7.4: | -0.100 |

## TABLE 6

| | | | | Marker2: | | eMob | 0.0175 | Rf | 0.53 |
|---|---|---|---|---|---|---|---|---|---|
| | Observed | | | | | Calculated | | | |
| 0.017, | 0.48 | A | 4-5p | | eMob | 0.0164 | Rf | 0.48 | |
| | | | 51 | | eMob | 0.018 | Rf | 0.49 | |
| 0.011, | 0.505 | B : | 4-5 | | eMob | 0.022 | Rf | 0.50 | |
| | | | 20-21 | | eMob | 0.0225 | Rf | 0.51 | |
| 0.022, | 0.57 | C : | 26-27 | | eMob | 0.021 | Rf | 0.58 | |
| 0.017, | 0.66 | D : | 21 | | eMob | 0.017 | Rf | 0.63 | |
| | | | 30nox | | eMob | 0.019 | Rf | 0.65 | |
| 0.0165, | 0.70 | E: | 55 | | eMob | 0.016 | Rf | 0.70 | |
| | | | 21nox | | eMob | 0.017 | Rf | 0.69 | |

(continued)

| Observed | | | | | Calculated | | | |
|---|---|---|---|---|---|---|---|---|
| 0.020, | 0.375 | F : | 4 | eMob | 0.022 | Rf | 0.395 |
| 0.035, | 0.365 | G : | 49 | eMob | 0.035 | Rf | 0.37 |
| 0.027, | 0.30 | H : | 52 | eMob | 0.027 | Rf | 0.33 |
| 0.012, | 0.55 | I : | 50 | eMob | 0.012 | Rf | 0.55 |
| | | | 50-51 | eMob | 0.013 | Rf | 0.54 |
| 0.012, | 0.58 | J : | 50nox | eMob | 0.012 | Rf | 0.59 |
| 0.017, | 0.59 | K : | 27 | eMob | 0.017 | Rf | 0.57 |
| 0.015, | 0.33 | L : | 4p | eMob | 0.015 | Rf | 0.35 |
| 0.020, | 0.38 | M : | 3-4 | eMob | 0.028 | Rf | 0.387 |

**Table 7 : The theoretical emobile and chromatographic mobilities of methionine containing peptides generated after digestion of human Vimentin (466 amino acids).**

| -----Peptide #1----- | | ----Peptide #4------ | |
|---|---|---|---|
| Sequence: (MSTR) [SEQ ID N°4] | | Sequence: (MFGGPGTASRPSSSR) | |
| MobElec: | 0.032 | [SEQ ID N°5] | |
| Position | aa 1-4 | MobElec: | 0.023 |
| | | Position | aa 14-28 |
| --------Peptide #21----- | | ---------Peptide #27——— | |
| Sequence: (LGDLYEEEMR)[SEQ ID N°6] | | Sequence: (DNLAEDIMR) | |
| MobElec: | 0.017 | [SEQ ID N°7] | |
| Position | aa 146-155 | MobElec: | 0.018 |
| | | Position | aa 176-184 |
| -------Peptide #30 -------- | | ---Peptide #49------ | |
| Sequence: (LQEEMLQR) [SEQ ID N°8] | | Sequence: (QMR)[SEQ ID N°9] | |
| MobElec: | 0.019 | MobElec: | 0.034 |
| Position | aa 189-196 | Position | aa 343-345 |
| --------- Peptide #50---- | | ----Peptide #51 --- | |
| Sequence: (EMEENFAVEAANYQDTIGR) | | Sequence: (LQDEIQNMK [SEQ | |
| [SEQ ID N°10] | | ID N°11] | |
| MobElec: | 0.011 | MobElec: | 0.018 |
| Position | aa 346-364 | Position | aa 365-373 |
| ---------Peptide #52 -------- | | -----Peptide #55---- | |
| Sequence: (EEMAR) [SEQ ID N°12] | | Sequence: (MALDIEIATYR))SEQ | |
| MobElec: | 0.026 | ID N°13] | |
| Position | aa 374-378 | MobElec: | 0.016 |
| | | Position | aa 391-401 |

## TABLE 8

### Table 8 : Theoretical peptides generated from mis-cleavage and phosphorylation of VIMENTIN

| | | | | |
|---|---|---|---|---|
| 448.54 | NCPO | 444-455 | pep#20-21 | vsRLGDLYEEEmR |
| 183.84 | NCO | 14-36 | pep#4-5 | mPGGPGTASRPsSSRSYVTTSTR |
| 129.42 | NCPO | 14-36 | pep#4-5 | mPGGPGTASRPsSSRSYVTTSTR |
| 129.42 | NCPO | 14-36 | pep#4-5 | mPGGPGTASRPSsSRSYVTTSTR |

**Table 9**

Conditions: pH= 2 buffer= chr

>vim

Sequence:

(MSTRSVSSSSYRRMPGGPGTASRPSSSRSYVTTSTRTYSLGSALRPSTSRSLYASSPGGVYATRSSAVRLRSSVPGVRLLQDSVDFSLADAINTEPKNTRTNBKVELQBLNDRFANYIDKVRPLE
QQNKILLABLEQLKGQQKSRLGDLYEEEMRELRRQVDQLTNDKARVEVERDNLABDIMRLREKLQEBMLQRBBAENTLQSFRQDVDNASLARLDLLERKVESLQEEIAFLKKLHREEIQBLQAQIQEQHVQIDVD
VSKPDLTAALRDVRQQYESVAAKNLQBABBWYKSKFADLSEAANRNNDALRQAKQESTEYRRQVQSLTCEVDALKGTNBSLERQMREMEENFAVEAANYQDTIGRLQDEIQNMKEBMARHLRBYQDLLNVKMAL
DIBIATYRKLLEGBESRISLPLPNFSSLANLRETNLDSLPLVDTHSKRTFLLIKTVBTRDGQVINETSQHHDDLB) [SEQ ID N°14].

Composition:

A-33 C-1 D-29 B-55 F-11 G-14 H-6 I-15 K-22 L-54 M-10 N-23 P-9 Q-33 R-43 S-43 T-25 V-26 W-1 Y-13

Longueur:      466
Proprietes:    1e07
Molweight :    53685.8
Maas :         53653.1
----------------------------------
NetCharge:     71.3217

51

IsoElecPoint: 4.7447

MobElec: 0.0501166

MobChro: 0.592997

Rtn2.1: 217.2

Rtn7.4: -103.5

-------------------------------------------------------------------

**********************************************************************
*                                                                    *
*                    List of selected peptides                       *
*                                                                    *
**********************************************************************

---------Peptide #1---------

Sequence: (MSTR) [SEQ ID N°4]

Composition:

M-1 R-1 S-1 T-1

Longueur: 4

Molweight : 493.585

Mass : 493.232

NetCharge: 1.973

IsoElecPoint: 10.519

MobElec: 0.032

---

MobChro: 0.582

Rtn2.1: 0.400

Rtn7.4: 9.500

---------Peptide #4---------

Sequence: (MPGGPGTASRPSSSR) [SEQ ID N°5].

Composition:

A-1 P-1 G-3 M-1 P-2 R-2 S-4 T-1

Longueur: 15

Molweight : 1494.651

Mass : 1493.705

NetCharge: 2.973

IsoElecPoint: 12.480

MobElec: 0.023

MobChro: 0.447

Rtn2.1: 13.100

Rtn7.4: 39.800

---------Peptide #21---------

EP 1 577 823 B1

Sequence: (LGDLYEEEMR) (SEQ ID N°6)

Composition:

D-1 E-3 G-1 L-2 M-1 R-1 Y-1

Longueur:        10

Molweight :      1254.384

Mass :           1253.560

NetCharge:       1.935

IsoElecPoint:    3.753

MobElec:         0.017

MobChro:         0.674

Rtn2.1:          5.000

Rtn7.4:          -29.600

---------Peptide #27---------

Sequence: (DNLAEDIMR) (SEQ ID N°7)

Composition:

A-1 D-2 E-1 I-1 L-1 M-1 N-1 R-1

Longueur:        9

Molweight :      1076.196

Mass :           1075.497

NetCharge:       1.940

IsoElecPoint:    3.859

MobElec:         0.018

MobChro:         0.651

Rtn2.1:          9.600

Rtn7.4:          -5.300

---------Peptide #30---------

Sequence: (LQEEMLQR) (SEQ ID N°8)

Composition:

E-2 L-2 M-1 Q-2 R-1

Longueur:        8

Molweight :      1046.213

Mass :           1045.523

NetCharge:       1.956

IsoElecPoint:    4.226

MobElec:         0.019

MobChro:         0.648

Rtn2.1:          2.600

Rtn7.4:          -20.200

---------Peptide #49---------

Sequence: (QMR) (SEQ ID N°9)

Composition:

M-1 Q-1 R-1

Longueur:        3

Molweight :      433.532

Mass :           433.211

NetCharge:       1.973

IsoElecPoint:    10.519

MobElec:         0.034

MobChro:         0.526

Rtn2.1:          0.100

Rtn7.4:          0.800

---------Peptide #50---------

Sequence: (EMEENFAVEAANYQDTIGR) [SEQ ID N°10]

Composition:

A-3 D-1 E-4 F-1 G-1 I-1 M-1 N-2 Q-1 R-1 T-1 V-1 Y-1

Longueur:        19

Molweight :      2187..329

Mass :           2185.958

NetCharge:       1.927

IsoElecPoint:    3.645

MobElec:         0.011

MobChro:         0.589

Rtn2.1:          2.000

Rtn7.4:          -36.500

NetCharge:       1.952

IsoElecPoint:    4.162

MobElec:         0.018

MobChro:         0.547

Rtn2.1:          8.800

Rtn7.4:          -7.900

---------Peptide #52---------

Sequence: (EEMAR) [SEQ ID N°12]

Composition:

A-1 E-2 M-1 R-1

Longueur:        5

Molweight :      634.711

Mass :           634.274

NetCharge:       1.956

IsoElecPoint:    4.226

MobElec:         0.026

MobChro:         0.478

Rtn2.1:          -12.500

Rtn7.4:          -27.700

---------Peptide #51---------

Sequence: (LQDEIQNMK) [SEQ ID N°11]

Composition:

D-1 E-1 I-1 K-1 L-1 M-1 N-1 Q-2

Longueur:        9

Molweight :      1118.277

Mass :           1117.544

54

--------Peptide #55--------
Sequence: (MALDIBIATYR) [SEQ ID N° 13]
Composition:
A-2 D-1 B-1 I-2 L-1 M-1 R-1 T-1 Y-1

Longueur:        11
Molweight :      1295.523
Mass :           1294.659
NetCharge:       1.952
IsoElecPoint:    4.162
MobElec:         0.016
MobChro:         0.811
Rtn2.1:          35.400
Rtn7.4:          26.900

List of detected spots

Spot #1: (0.017,0.528)
Spot #2: (0.017,0.476)
Spot #3: (0.021,0.504)
Spot #4: (0.022,0.571)
Spot #5: (0.017,0.675)
Spot #6: (0.017,0.707)
Spot #7: (0.02,0.374)
Spot #8: (0.036,0.365)
Spot #9: (0.028,0.295)
Spot #10: (0.012,0.546)
Spot #11: (0.007,0.774)
Spot #12: (0.017,0.769)
Spot #13: (0.012,0.588)
Spot #14: (0.017,0.594)
Spot #15: (0.016,0.323)
Spot #16: (0.026,0.466)
Spot #17: (0.028,0.377)
Spot #18: (0.025,0.318)

**Table 10**

List of putative correspondences

Spot #1: (0.017,0.S2θ):

| | | | | | |
|---|---|---|---|---|---|
| 116.19 | NCP | 189-207 | pep#30-31 | (0.016,0.531) | LQEEMLQREEAEMTLQsFR [SEQ ID N°15] |
| 93.43 | NC | 189-207 | pep#30-31 | (0.017,0.538) | LOEEMLOREEAENTLQSFR [SEQ ID N°15] |

(continued)

| | | | | List of putative correspondences | |
|---|---|---|---|---|---|
| 45.44 4 | NCO | 189-207 | pep#30-31 | (0.016,0.510) | LQEEmLQREEAENTLQSFR [SEQ ID N°15] |
| 44.50 | N | 365-373 | pep#51 | (0.018,0.547) | LODEIQNMK [SEO ID N°11] |
| 40.58 | NCP | 343-364 | pep#49-50 | (0.015,0.544) | QMREMEENFAVEAANYQDtIGR [SEQ ID N°16] |

Spot #2: (0.017,0.476):

| | | | | | |
|---|---|---|---|---|---|
| 79.83 | NCP | 189-207 | pep#30-31 | (0.016,0.481) | LQEEMLQREEAENtLQsFR [SEQ ID N°15] |
| 74.38 | NCP | 189-207 | pep#30-31 | (0.016,0.487) | LQEEMLQREEAENtLQSFR [SEQ ID N°15] |
| 73.43 | O | 365-373 | pep#51 | (0.017, 0 .489) | LQDEIQNmK [SEQ ID N°11) |
| 47.04 | P | 146-155 | pep#21 | (0.016,0.495) | LGDLyEEEMR [SEQ ID N°6] |
| 45.09 | NCP | 343-364 | pep#49-50 | (0.015,0.470) | QMREMEENFAVEAANyQDtIGR [SEQ ID N°16] |

Spot #3: (0.021,0.504):

| | | | | | |
|---|---|---|---|---|---|
| 250.82 | NCO | 14-36 | pep#4-5 | (0.021,0.503) | MFGGPGTASRPSSSRSYVTTSTR [SEQ ID N°17] |
| 209.94 | NCPO | 144-155 | pep#20-21 | (0.021,0.504) | sRLGDLYEEEmR [SEQ ID N°18] |
| 133.48 | NCP | 14-36 | pep#4-5 | (0.022,0.503) | MFGGPGtASRPSSSRSYVTTSTR [SEQ ID N°17] |
| 133.48 | NCP | 14-36 | pep#4-5 | (0.022,0.503) | MFGGPGtASRPSSSRSYVtTSTR [SEQ ID N°17] |
| 133.48 | NCP | 14-36 | pep#4-5 | (0.022,0.503) | MFGGPGtASRPSSSRSYVTtSTR [SEQ ID N°17] |

Spot #4: (0.022,0.571):

| | | | | | |
|---|---|---|---|---|---|
| 68.35 | NC | 144-155 | pep#20-21 | (0.022,0.557) | SRLGDLYEEEMR [SEQ ID N°18] |
| 42.23 | NCP | 144-155 | pep#20-21 | (0-022,0.548) | sRLGDLYEEEMR [SEQ ID N°18] |
| 27.71 | NCO | 171-184 | pep#26-27 | (0.020,0.597) | VEVERDNLAEDImR [SEQ ID N°19] |
| 22.90 | NC | 14-36 | pep#4-5 | (0.022,0.527) | MFGGPGTASRPSSSRSYVTTSTR [SEQ ID N°17] |
| 22.04 | N | 365-373 | pep#51 | (0.016,0.547) | LQDEIQNMK [SEQ ID N°11] |

Spot #5: (0.017,0.675) :

| | | | | | |
|---|---|---|---|---|---|
| 257.71 | N | 146-155 | pep#21 | (0.017,0.674) | LGDLYEEEMR [SEQ ID N°6] |
| 53.74 | NCP | 382-401 | pep#54-55 | (0.016,0.688) | EyQDLLNVKMALDIEIATyR [SEQ ID N°20] |
| 41.68 | PO | 391-401 | pep#55 | (0.015,0.672) | mALDIEIATyR [SEQ ID N°13] |
| 38.80 | NCPO | 382-401 | pep#54-55 | (0-015,0.660) | EyQDLLNVKmALDIEIATyR [SEQ ID N°20] |
| 35.71 | N | 176-184 | pep#27 | (0.018,0.651) | DNLAEDIMR [SEQ ID N°7] |

Spot #6: (0.017.0.707):

| | | | | | |
|---|---|---|---|---|---|
| 56.70 | NCPO | 382-401 | pep#54-55 | (0.015,0.708) | EyQDLLNVKmALDIEIATYR [SEQ ID N°20] |
| 56.70 | NCPO | 382-401 | pep#54-55 | (0.015,0.708) | EYQDLLNVKmALDIEIATyR [SEQ ID N°20] |
| 55.53 | P | 391-401 | pep#55 | (0.015,0.707) | MALDIEIAtyR [SEQ ID N°13] |
| 48.53 | P | 391-401 | pep#55 | (0.016.0.724) | MALDIEIATyR [SEQ id N°13] |
| 44.73 | NCPO | 382-401 | pep#54-55 | (0-015.0.699) | EyQDLLNVIUnALDIEIAtYR [SEQ ID N°20] |

Spot #7: (0.02,0.374):

| | | | | | |
|---|---|---|---|---|---|
| 116.22 | NCPO | 144-155 | pep#20-21 | (0.021,0.367) | SRLGDLyEEEmR [SEQ ID N°18] |
| 72.23 | P | 14-28 | pep#4 | (0.021,0.380) | MFGGPGtAsRPSSSR [SEQ ID N°5] |
| 72.23 | P | 14-28 | pep#4 | (0.021,0.380) | MFGGPGtASRPsSSR [SEQ ID N°5] |
| 72.23 | P | 14-28 | pep#4 | (0.021,0.380) | MFGGPGtASRPSsSR [SEQ ID N°5] |
| 72.23 | P | 14-28 | pep#4 | (0.021,0.380) | MFGGPGtASRPSSsR [SEQ ID N°5] |

Spot #8: (0.036,0.36S):

| | | | | | |
|---|---|---|---|---|---|
| 19.01 | 0 | 343-345 | pep#49 | (0.031,0.352) | QmR [SEQ ID N°9] |

(continued)

| | | | | | | |
|---|---|---|---|---|---|---|
| | | | | List of putative correspondences | | |
| 13.40 | P | 1-4 | pep#1 | (0.029,0.373) | MStR [SEQ ID N°4] | |
| 11.85 | NCP | 13-28 | pep#3-4 | (0.028,0.363) | RMFGGPGtASRPSSSR [SEQ ID N°21] | |
| 11.10 | NCO | 13-28 | pep#3-4 | (0.027,0.387) | RmFGGPGTASRPSSSR [SEQ ID N°21] | |
| 10.75 | NCP | 13-28 | pep#3-4 | (0.027,0.356) | RMFGGPGtAsRPSSSR [SEQ ID N°21] | |

Spot #9: (0.028,0.295):

| | | | | | |
|---|---|---|---|---|---|
| 26.92 | NCPO | 13-28 | pep#3-4 | (0.026,0.324) | RmFGGPGtAsRPSSSR [SEQ ID N°21] |
| 26.92 | NCPO | 13-28 | pep#3-4 | (0.026,0.324) | RmFGGPGtASRPsSSR [SEQ ID N°21] |
| 26.92 | NCPO | 13-28 | pep#3-4 | (0.026,0.324) | RmFGGPGtASRPSsSR [SEQ ID N°21] |
| 26.92 | NCPO | 13-28 | pep#3-4 | (0.026,0.324) | RmFGGPGtASRPSSsR [SEQ ID N°21] |
| 25.75 | NCPO | 13-28 | pep#3-4 | (0.026,0.331) | RmFGGPGtASRPSSSR [SEQ ID N°21] |

Spot #10: (0.012,0.546):

| | | | | | |
|---|---|---|---|---|---|
| 400.71 | NCO | 346-373 | pep#50-51 | (0.012,0.544) | EmEENFAVEAANYQDTIGRLQDEIQNmK [SEQ ID N°22] |
| 90.78 | NCP | 346-373 | pep#50-51 | (0.013, 0.552) | EMEENFAVEAANYQDtIGRLQDEIQNMK [SEQ ID N°22] |
| 84.36 | P | 346-364 | pep#50 | (0.011,0.554) | EMEENFAVEAANYQDtIGR [SEQ ID N°10] |
| 71.34 | NCPO | 346-373 | pep#50-51 | (0.012,0.532) | EmEENFAVEAANYQDtIGRLQDEIQNMK [SEQ ID N°22] |
| 71.34 | NCPO | 346-373 | pep#50-51 | (0.012,0.S32) | EMEENFAVEAANYQDtIGRLQDEIQNmK [SEQ ID N°22] |

Spot #11: (0.007,0.774):

| | | | | | |
|---|---|---|---|---|---|
| 12.17 | PO | 391-401 | pep#55 | (0.015,0.743) | mALDIEIAtYR [SEQ ID-N°13] |
| 12.01 | O | 391-401 | pep#55 | (0.015,0.760) | Maldieiatyr [SEQ ID N°13] |
| 11.52 | NCPO | 382-401 | pep#54-55 | (0.015,0.747) | EYQDLLNVKmALDIEIAtYR [SEQ ID N°20] |
| 11.41 | NCO | 382-401 | pep#54-55 | (0.016,0.756) | EYQDLLNVKmALDIEIATYR [SEQ ID N°201] |
| 11.10 | NCP | 382-401 | pep#54-55 | (0.016,0.775) | EYQDLLNVKMALDIEIAtYR [SEQ ID N°20] |

Spot #12: (0.017,0.769):

| | | | | | |
|---|---|---|---|---|---|
| 86.04 | NCP | 382-401 | pep#54-55 | (0.016,0.775) | EYQDLLNVKMALDIEIAtYR [SEQ ID N°20] |
| 61.30 | NC | 382-401 | pep#54-55 | (0.016,0.784) | EYQDLLNVKMALDIEIATYR [SEQ ID N°20] |
| 51.31 | NCO | 382-401 | pep#54-55 | (0.016,0.756) | EYQDLLNVKmALDIEIATYR [SEQ ID N°20] |
| 49.16 | O | 391-401 | pep#55 | (0.015,0.760) | mALDIEIATYR [SEQ ID N°13] |
| 35.35 | P | 391-401 | pep#55 | (0.016,0.795) | MALDIEIAtYR [SEQ ID N°13] |

Spot #13: (0.012, 0.588):

| | | | | | |
|---|---|---|---|---|---|
| 174.85 | N | 346-364 | pep#50 | (0.011,0.589) | EMEENFAVEAANYQDTIGR [SEQ ID N°10] |
| 80.60 | NC | 346-373 | pep#50-51 | (0.013,0.583) | EMEENFAVEAANYQDTIGRLQDEIQNMK [SEQ ID N°22] |
| 39.71 | NCO | 346-373 | pep#50-51 | (0.013,0.563) | EmEENFAVEAANYQDTIGRLQDEIQNMK [SEQ ID N°22] |
| 39.71 | NCO | 346-373 | pep#50-51 | (0.013,0.563) | EMEENFAVEAANYQDTIGRLQDEIQNmK [SEQ ID N°22] |
| 31.41 | O | 346-364 | pep#50 | (0.011,0.559) | EmEENFAVEAANYQDTIGR [SEQ ID N°10] |

Spot #14: (0.017,0.594):

| | | | | | |
|---|---|---|---|---|---|
| 191.69 | O | 176-184 | pep#27 | (0.027,0.589) | DNLAEDImR [SEQ ID N°7] |
| 70.50 | O | 189-196 | pep#30 | (0.017,0.581) | LQEEmLQR (SEQ ID N°8] |

(continued)

| | | | | | | |
|---|---|---|---|---|---|---|
| | | | List of putative correspondences | | | |
| 65.27 | NC | 343-364 | pep#49-50 | (0.015,0.595) | QMREMEENFAVEAANYQDTIGR [SEQ ID N°16] | |
| 38.96 | NCO | 171-184 | pep#26-27 | (0.020,0.597) | VEVERDNLAEDImR [SEQ ID N°19] | |
| 33.43 | O | 146-155 | pep#21 | (0.015,0.619) | LGDLYEEEmR [SEQ ID N°6] | |
| | | | | | | |
| Spot #15: (0.016, 0.323): | | | | | | |
| 21.24 | PO | 14-28 | pep#4 | (0.020,0.345) | mFGGPGtAsRPSSSR [SEQ ID N°5] | |
| 21.24 | PO | 14-28 | pep#4 | (0.020.0.345) | mFGGPGtASRPsSSR [SEQ ID N°5] | |
| 21.24 | PO | 14-28 | pep#4 | (0.020,0.345) | mFGGPGtASRPSsSR [SEQ ID N°5] | |
| 21.24 | PO | 14-28 | pep#4 | (0.020,0.345) | mFGGPGtASRPSSsR [SEQ ID N°5] | |
| 18.67 | NCPO | 144-155 | pep#20-21 | (0.020,0.360) | sRLGDLyEEEmR [SEQ ID N°18] | |
| | | | | | | |
| Spot #16: (0.026,0.466): | | | | | | |
| 74.94 | N | 374-378 | pep#52 | (0.026,0.478) | EEMAR [SEQ ID N°12] | |
| 61.30 | NC | 1-12 | pep#1-2 | (0.024,0.467) | MSTRSVSSSSYR [SEQ ID N°23] | |
| 37.20 | NCP | 1-12 | pep#1-2 | (0.023,0.457) | MsTRSVSSSSYR [SEQ ID N°23] | |
| 37.20 | NCP | 1-12 | pep#1-2 | (0.023,0.457) | MSTRsVSSSSYR [SEQ ID N°23] | |
| 37.20 | NCP | 1-12 | pep#1-2 | (0.023,0.457) | MSTRSVsSSSYR [SEQ ID N°23] | |
| | | | | | | |
| Spot #17: (0.028,0.377): | | | | | | |
| 143.97 | P | 1-4 | pep#1 | (0.029,0.373) | MStR [SEQ ID N°4] | |
| 81.90 | NCO | 13-28 | pep#3-4 | (0.027,0.387) | RmFGGPGTASRPSSSR [SEQ ID N°21] | |
| 67.20 | NCP | 13-28 | pep#3-4 | (0.028,0.363) | RMFGGPGtASRPSSSR [SEQ ID N°21] | |
| 63.17 | NCPO | 13-28 | pep#3-4 | (0.026,0.379) | RmFGGPGTASRPSSSR [SEQ ID N°21] | |
| 63.17 | NCPO | 13-28 | pep#3-4 | (0.026,0.379) | RmFGGPGTASRPsSSR [SEQ ID N°21] | |
| | | | | | | |
| Spot #18: (0.025,0.318): | | | | | | |
| 113.93 | NCPO | 13-28 | pep#3-4 | (0.026,0.324) | RmFGGPGtAsRPSSSR [SEQ ID N°21] | |
| 113.93 | NCPO | 13-28 | pep#3-4 | (0.026,0.324) | RmFGGPGtASRPsSSR [SEQ ID N°21] | |
| 113.93 | NCPO | 13-28 | pep#3-4 | (0.026,0.324) | RmFGGPGtASRPSsSR [SEQ ID N°21] | |
| 113.93 | NCPO | 13-28 | pep#3-4 | (0.026,0.324) | RmFGGPGtASRPSSsR [SEQ ID N°21] | |
| 62.15 | NCP | 1-12 | pep#1-2 | (0.023,0.322) | MSTRSVSSSSyR [SEQ ID N°23] | |

Annex 3

| | | | | | |
|---|---|---|---|---|---|
| | | | List of putative correspondences | | |
| Spot #1: (0.017,0.528): | | | | | |
| 126.60 | NCP | 189-207 | pep#30-31 | (0.016,0.531) | LQEEMLQREEAENTLQsFR [SEQ ID N°15] |
| 96.02 | NC | 189-207 | pep#30-31 | (0.017,0.538) | LQEEMLQREEAENTLQSFR [SEQ ID N°15] |
| | | | | | |
| Spot #2: (0.017,0.476): | | | | | |
| 76.90 | NCP | 189-207 | pep#30-31 | (0.016,0.487) | LQEEMLQREEAENtLQSFR [SEQ ID N°15] |
| 75.00 | O | 365-373 | pep#51 | (0.017,0.489) | LQDEIQNmK [SEQ ID N°11] |
| | | | | | |
| Spot #3: (0.021,0.504): | | | | | |
| 448.54 | NCPO | 144-155 | pep#20-21 | (0.021,0.504) | sRLGDLYEEEmR [SEQ ID N°19] |

(continued)

| | List of putative correspondences | | | | |
|---|---|---|---|---|---|
| 183.84 | NCO | 14-36 | pep#4-5 | (0.022,0.503) | mFGGPGTASRPSSSRSYVTTSTR [SEQ ID N°17] |
| 129.42 | NCPO | 14-36 | pep#4-5 | (0.021,0.496) | mFGGPGTAsRPSSSRSYVTTSTR [SEQ ID N°17] |
| 129.42 | NCPO | 14-36 | pep#4-5 | (0.021,0.496) | mFGGPGTASRPsSSRSYVTTSTR [SEQ ID N°17] |
| 129.42 | NCPO | 14-36 | pep#4-5 | (0.021,0.496) | mFGGPGTASRPSsSRSYVTTSTR [SEQ ID N°17] |
| Spot #4: (0.022,0.571): | | | | | |
| Spot #5: (0.017,0.675): | | | | | |
| 348.75 | N | 146-155 | pep#21 | (0.017,0.674) | MLGDLYEEEMR [SEQ ID N°6] |
| Spot #6: (0.017,0.707): | | | | | |
| Spot #7: (0.02,0.374): | | | | | |
| Spot #8: (0.036,0.365): | | | | | |
| 20.72 | O | 343-345 | pep#49 | (0.031,0.352) | QmR [SEQ ID N°9] |
| Spot #9: (0.028,0.295): | [SEQ ID N°25] | | | | |
| 26.23 | NCPO | 13-28 | pep#3-4 | (0.027,0.331) | RmFGGPGtASRPSSSR[SEQ ID N°21] |
| Spot #10: (0.012,0.546): | | | | | |
| 309.50 | NCO | 346-373 | pep#50-51 | (0.012,0.544) | EmEENFAVEAANYQDTIGRLQDEIQNmK [SEQ ID N°22] |
| 88.78 | P | 346-364 | pep#50 | (0.011,0.5.54) | EMEENFAVEAANYQDtIGR [SEQ ID N°10] |
| 85.13 | NCP | 346-373 | pep#50-51 | (0.013,0.552) | EMEENFAVEAANYQDtIGRLQDEIQNMK [SEQ ID N°22] |
| 68.69 | NCPO | 346-373 | pep#50-51 | (0.013,0.532) | EmEENFAVEAANYQDtIGRLQDEIQNMK [SEQ ID N°22] |
| 68.69 | NCPO | 346-373 | pep#50-51 | (0.013,0.532) | EMEENFAVEAANYQDtIGRLQDEIQNmK [SEQ ID N°22] |
| Spot #11: (0.007,0.774): | | | | | |
| Spot #12: (0.017,0.769): | | | | | |
| 91.09 | NCP | 382-401 | pep#54-55 | (0.016,0.775) | EYQDLLNVKMALDIEIAtYR [SEQ ID N°20] |
| 55.29 | O | 391-401 | pep#55 | (0.015,0.760) | mALDIEIATYR [SEQ ID N°13] |
| 55.27 | NCO | 382-401 | pep#54-55 | (0.016,0.756) | EYQDLLNVKmALDIEIATYR [SEQ ID N°20] |
| Spot #13: (0.012,0.588): | | | | | |
| 206.02 | N | 346-364 | pep#50 | (0.012,0.589) | EMEENFAVEAANYQDTIGR [SEQ ID N°10] |

(continued)

| List of putative correspondences | | | | | | |
|---|---|---|---|---|---|---|
| 75.72 | NC | 346-373 | pep#50-51 | (0.013,0.583) | EMEENFAVEAANYQDTIGRLQDEIQNMK [SEQ ID N°22] | |
| 39.01 | NCO | 346-373 | pep#50-51 | (0.013,0.563) | EmEENFAVEAANYQDTIGRLQDEIQNMK [SEQ ID N°22] | |
| 39.01 | NCO | 346-373 | pep#50-51 | (0.013,0.563) | EMEENFAVEAANYQDTIGRLQDEIQNmK [SEQ ID N°22] | |

Spot #14: (0.017,0.594):

| 160.27 | O | 176-184 | pep#27 | (0.017, 0.589) | DNLAEDImR [SEQ ID N°7] |
|---|---|---|---|---|---|
| 68.78 | NC | 343-364 | pep#49-50 | (0.016, 0.595) | QMREMEENFAVEAANYQDTIGR [SEQ ID N°16] |
| 64.75 | O | 189-196 | pep#30 | (0.018,0.581) | LQEEmLQR [SEQ ID N°8] |

Spot #15: (0.016,0.323):

Spot #16: (0.026,0.466):

| 72.32 | N | 374-378 | pep#52 | (0.027,0.478) | EEMAR [SEQ ID N°12] |
|---|---|---|---|---|---|
| 63.01 | NC | 1-12 | pep#1-2 | (0.024,0.467) | MSTRSVSSSSYR [SEQ ID N°23] |

Spot #17: (0.028,0.377):

| 131.32 | P | 1-4 | pep#1 | (0.029,0.373) | MStR [SEQ ID N°4] |
|---|---|---|---|---|---|
| 89.66 | NCO | 13-28 | pep#3-4 | (0.027,0.387) | RmFGGPGTASRPSSSR [SEQ ID N°21] |
| 71.49 | NCPO | 13-28 | pep#3-4 | (0.027,0.379) | RmFGGPGTAsRPSSSR [SEQ ID N°21] |

Spot #18: (0.025,0.318):

| 63.78 | NCP | 1-12 | pep#1-2 | (0.023,0.322) | MSTRSVSSSSyR [SEQ ID N°23] |
|---|---|---|---|---|---|
| 58.31 | NCO | 335-345 | pep#48-49 | (0.023,0.326) | GTNESLERQmR [SEQ ID N°31] |
| 48.97 | NCPO | 13-28 | pep#3-4 | (0.027,0.331) | RmFGGPGtASRPSSSR [seq ID N°21] |
| 41.43 | NCPO | 335-345 | pep#48-99 | (0.023,0.317) | GTNEsLERQmR [SEQ ID N°24] |

## REFERENCES:

### Manufacturers

- MERCK TLC, HPTLC and PLC plates (glass) Chemically modified layers.

10 x 10, 10 x 20 and 20 x 20 Article numbers
Merck KGaa, Darmstadt, Deutschland.

### Molecular Probes:

- Handbook of Fluorescent Probes and Research Products, Molecular Probes Inc. PO Box 22010 29851 Willow Creek Road, Eugene, OR 97402-0469 USA.
- Amersham Pharmacia Biotech:

  PhosphorImager, Storm, Amersham Biosciences US Limited, Amersham Place, Little Chalfont, Buckinghamshire, HP7 9NA U.K.

**Packard Instruments:**

- Instantlmager (U.S. Patent 5,138,168) Packard Instruments Inc., Perkin Elmer Life Sciences Inc. 549 albany Street, Boston, MA 02118-2512, SA. Scientific References.

**Molecular Probes:**

- Handbook of Fluorescent Probes and Research Products, Molecular Probes Inc. PO Box 22010 29851 Willow Creek Road, Eugene , OR 97402-0469 U.S.A.

- Boyle WJ, Van der Geer P, Hunter T. 1991. Phosphopeptide mapping and phosphoamino acid analysis by two-dimensional separation on thin layer cellulose plates. Methods Enzymol. 201:110-49.

- Girard F, Strausfeld U, Fernandez a, Lamb NJ. 1991 Cyclin A is required for the on set of DNA replication in mammalian fibroblasts. Cell. 67:1169-79.

- Kitzmann M, Vandromme M, Schaeffer V, Carnac G, Labbe JC, Lamb N, Fernandez A. 1999, cdl1- and cdk2-mediated phosphorylation of MyoD Ser200 in growing C2 myoblasts: role in modulating MyoD half-life and myogenic ac tivity. Mol Cell Biol. 19: 3167-76.

- Lamb NJ, Fernandez A, Feramisco JR, Welch WJ, 1989 . Modulation of vimentin containing intermediate filament distribution and phosphorylation in living fibroblasts by the cAMP-dependent protein kinase. J. Ce'll Biol. 108:2409-22.

- Pearson WR. 2000 Flexible sequence similarity searching with the FASTA3 program package. Methods Mol Biol. 132:185-219.

- Soille, P. 1999. Morphological Image analysis: Principles and applications. Springer Verlag. ISBN 3540656715.

**Protein purification.**

- Deutscher, M.P. and Abelson J.N. 1990. Guide to Protein Purification . Methods in Enzymology, Volume 182: ISBN: 0122135857.

**Claims**

1. A method for identifying one unknown protein contained in a sample, based on the migrating properties on a substrate of peptides generated from said unknown protein, wherein said method comprises the steps of :

(a) subjecting the said sample containing the said unknown protein to an enzymatic or a chemical cleavage, whereby a pool of protein-specific peptide fragments is obtained;
(b) incorporating in the said pool of protein-specific peptide fragments at least two marker peptide compounds of known amino acid sequence, whereby an assay sample is obtained;
(c) subjecting the said assay sample to a two-dimensional separation step on the surface area of a separation substrate, wherein the said two-dimensional separation step consists of :

(i) a first separation step whereby the protein-specific peptide fragments and the at least two marker compounds migrate along the X-axis on the surface area of a first separation substrate; and

(ii) a second separation step whereby the protein-specific peptide fragments and the said marker compounds migrate along the Y-axis on the surface area of a second separation substrate,

wherein (1) the first and second separation substrates are identical or (2) the first and second separation substrates are distinct, whereby a two-dimensional array of protein spots is formed from the pool of peptide fragments and the marker compounds which were initially contained in the assay sample;

(d) determining the experimental localisation value for each peptide spot contained in the two-dimensional array, wherein the said experimental localisation value consists of the X and Y co-ordinates for each peptide spot contained in the two-dimensional array, respective to a unique predetermined point of origin in the array;

(e) comparing the experimental localisation value determined at step (d) for each marker compound with a predetermined value for the theoretical localisation value of the said marker compound on the two-dimensional array, and then calculating two correction factors, respectively :

(i) a first correction factor "Ce" which is representative of the distance, along the X-axis of the two-dimensional array, between the experimental and theoretical X co-ordinates determined for the said marker compound; and

(ii) a second correction factor "CrF" which is representative of the distance, along the Y-axis of the two-dimensional array, between the experimental and theoretical Y co-ordinates determined for said marker compound;

(f) calculating, for each peptide spot corresponding to the peptide fragments generated by the cleavage of the said unknown protein, the corrected X and Y co-ordinates of said peptide spot on the two-dimensional array by applying to the experimental X and Y co-ordinates of the said peptide spot determined at step (d) the correction factors "Ce" and "CrF" determined at step (e), respectively, whereby a set of corrected localisation values for the set of peptide spots corresponding to the peptide fragments is obtained;

(g) comparing the set of corrected localisation values obtained from the set of peptide spots at the end of step (f), wherein said set of corrected localisation values characterise the unknown protein, with either:

(i) a pre-built ordered collection of sets of theoretical localisation values, wherein each set of theoretical localisation values contained in the said ordered collection characterises the set of peptide fragments which is expected to be generated by subjecting a given protein of a known amino acid sequence to the same enzymatic or chemical cleavage as that experimentally performed on the unknown protein at step (a) of the method; or

(ii) successive sets of theoretical localisation values, wherein each set of theoretical localisation values characterises the set of peptide fragments which is expected to be generated by subjecting a given protein of a known amino acid sequence to the same enzymatic or chemical cleavage as that experimentally performed on the unknown protein at step (a) of the method, and wherein each set of theoretical localisation values is generated immediately before it is compared with the set of corrected localisation values for the set of protein spots corresponding to the set of peptide fragments which was generated from the unknown protein at step a);

(h) selecting, among the sets of theoretical localisation values contained in the ordered collection of sets defined at step (g-i), or alternatively the sets of theoretical localisation values defined at step (g-ii), (i) the set(s) which exhibit the closest similarity with, or (ii) the set(s) which is (are) identical to, the set of corrected localisation values obtained at the end of step (f) for the unknown protein to be identified, whereby the said unknown protein is assigned to consist of the protein(s) corresponding to said selected set(s).

2. The method of claim 1, wherein the protein cleavage consists of an enzyme cleavage using an enzyme selected from the group consisting of trypsin, trypsin [lys blocked], chymotrypsin, clostipain, proline endopeptidase, myxobacter protease, armilaria protease, staphylococcal protease, and endoproteinase Asp-N.

3. The method of claim 1, wherein the protein cleavage consists of a chemical cleavage using a compound selected from the group consisting of cyanogen bromide, 2-nitro-5-thiocyanobenzoic acid and an acidic compound suitable for acid hydrolysis at pH 2.5.

4. The method of claim 1, wherein the at least one peptide marker compound is labelled with a detectable molecule.

5. The method of claim 1, wherein the unknown protein, or the peptide fragments derived thereof by an enzyme or a chemical cleavage, is (are) labelled with a detectable molecule.

6. The method of claim 1, wherein both the at least one marker compound and the unknown protein or the peptide fragments derived thereof by an enzyme or a chemical cleavage are labelled with a detectable molecule.

7. The method of claim 6, wherein (i) the at least one marker compound and (ii) the unknown protein, or the peptide fragments derived thereof by an enzyme or a chemical cleavage, are respectively labelled with two distinct detectable molecules.

8. The method of claim 1, wherein at step (c), the first separation step consists of a high voltage electrophoresis and the second separation step consists of an ascending Chromatography.

9. The method of claim 1, wherein 2 to 10 marker peptide compounds of known amino acid sequences are incorporated at step (b).

10. The method of claim 1, wherein (the predetermined value for) the theoretical localisation for each marker compound, when said marker compound consists of a marker protein, consists of determining respectively the theoretical X and Y co-ordinates for a peptide of known sequence such as each marker peptide is calculated as follows :

Theoretical X co-ordinate value:

(i) determine the net charge of the marker peptide of a known amino acid sequence as follows :

(i-1) calculate the positive charge of the marker peptide according to the following formula (1) :

$$netplus = ([H^+] / Kd + [H^+]) + ([H^+] / pK1 + [H^+]) ,$$

wherein Kd is $10^{x(-pKr)}$ and pKr is the pKa for the positively charged amino acids and pK1 is the pKa of the amino-terminal group of the marker peptide.
(i-2) calculate the negative charge of the marker peptide according to the following formula (2) :

$$netminus = 1-( [H^+] / Kd + [H^+] ) + ( 1- ([H^+] / pK2 + [H^+] ) )$$

wherein Kd is $10^{x\,(-pKr)}$ and pKr is the pKa for the negatively charged amino acids and pK2 is the pKa of the carboxy-terminal group of the marker peptide.
(i-3) calculate the net charge of the marker peptide according to the following formula (3) :

$$netcharge = netplus - netminus$$

(ii) calculate the electrophoretic mobility of the marker peptide according to the following formula (4) :

$$Emobil = netcharge / Mr^{(2.0/3.0)},$$

wherein Mr is the molecular weight of the marker peptide expressed in daltons.

Theoretical Y co-ordinate value :

(i) calculate the absolute Y-mobility value of said marker peptide according to the following formula (5) :

$$AbsMob = [rf\text{-}aa_1 + rfaa_2 + \ldots + rfaa_n]/n$$

Wherein $rf\text{-}aa_1$. $rfaa_2$, ..., $rfaa_n$ are the respective Y-axis mobilty values for each of the amino acid residues contained in said marker protein of a known amino acid sequence and wherein « n » is the total number of amino acids contained in said marker peptide ;

(ii) calculate a corrected value for the absolute Y-mobility value of said marker peptide, whereby the corrected value takes into account (a) the percentage low mobility amino acids and (b) the number of high mobility amino acids in the sequence of said marker peptide, according to the following formula (6) :

$$Rf = AbsMob . CF$$

Wherein CF is the correction factor applied.

11. The method of claim 10, wherein the theoretical value for the absolute Y-mobility (Rf) for the said marker peptide is calculated according to the following conditions:

If 53<%lowRf<99 and gmob >0.36 :

$$Rf(i,j) = \sum \frac{mob(i)}{size} * \left[ \frac{-0.9(\%lowRf)}{44} + \frac{23.15}{11} \right] + \sum \frac{highRf(j)}{size}$$

if 53<%lowRf<99:

$$Rf(i) = \sum \frac{mob(i)}{size} * \left[ \frac{-0.9*(\%lowRf)}{44} + \frac{23.15}{11} \right]$$

if gmob>0.36 :

$$Rf(i,j) = \sum \frac{mob(i)}{size} + \sum \frac{highRf(j)}{size}$$

else :

$$Rf(i) = \sum \frac{mob(i)}{size}$$

wherein :

- Rf (i,j) and Rf(i) both mean the Rf value wherein the distinct conditions above are fulfilled ;
- $\Sigma$ [mob(i)/size] represents AbsMob;
- %lowRf is the percentage value of low mobility amino acids contained in the sequence of the said marker peptide which is used for calulating the correction factor which takes into account the precentage of low mobility amino acids and wherein the said correction factor is calculated according to the formula above, depending on the condition which is fulfilled ;
- gmob means the general mobility of the peptide under consideration;

- Size denotes the number of aminoacid residues contained in the peptide; and
- Σ [highRf(j)/size] represents the correction factor which takes into account the number of high mobility amino acids in the sequence of the said marker peptide.

**12.** The method of claim 1, wherein, at step (g), the (i) pre-built ordered collection of sets of theoretical localisation values or the (ii) successive sets of theoretical localisation values, wherein each set of theoretical localisation values characterises the peptide fragments which are expected to be generated by subjecting a given protein of a known sequence to the same enzymatic or chemical cleavage as that experimentally performed on the unknown protein at step (a) of the method, or alternatively each set of theoretical values which is generated, is generated by a method which comprises the following steps :

g1) generating, from the complete amino acid sequence of a known protein, a set of sub-sequences, wherein each sub-sequence corresponds to the amino acid sequence of a peptide fragment which is expected to be generated when the said known protein is subjected to the same enzymatic or chemical cleavage as that experimentally performed on the unknown protein at step (a) of the method ;
g2) calculating, for each sub-sequence generated at step g1), the theoretical localisation value of the said sub-sequence by performing an identical computation as that which is performed for calculating the theoretical localisation value of the marker compounds, whereby a set of theoretical localisation values corresponding to the said set of sub-sequences is generated, wherein the said set of sub-sequences corresponds to said known protein is generated;
g3) comparing the set of corrected localisation values of said unknown protein obtained at step (f) with, repectively, each set of theoretical values corresponding to every known protein which are contained in the ordered collection of sets of theoretical values.

**13.** The method of claim 12; wherein at step g2), the theoretical localisation value of said sub-sequence having one or several chemically modified amino acid residues is also calculated, whereby, at step (h), said unknown (chemically modified) protein is assigned to consist of the protein(s) corresponding to said selected set(s) contained in the ordered collection of sets.

**14.** The method of claim 1, wherein, when no set of theoretical values is selected at step h), then steps g) and h) of the method are performed a second time with sets of theoretical localisation values, wherein each set contains theoretical localisation values corresponding to the peptide fragments which are expected to be generated if the enzymatic or chemical cleavage to which is subjected the corresponding known protein is not complete.

**15.** The method of claim 1, wherein, when more than one set of theoretical localisation values is selected at step h), then a similarity score value is assigned to each of the selected set of theoretical localisation values.

**16.** The method of claim 15, wherein at step h) the unknown protein is assigned to consist of the protein corresponding to said selected set of theoretical localisation values to which has been assigned the highest similarity score value.

**17.** The method of claim 1, wherein steps d) to h) are performed by executing instructions of a computer software, which computer software has been previously loaded within the memory of a computer apparatus.

**18.** A computer memory support which is readable by a computer apparatus, wherein said computer memory support comprises instructions to cause the computer apparatus to carry out steps d) to h) of the method according to claim 1 when loaded into said computer apparatus.

**19.** A computer program comprising software code adapted to perform steps e) to h) of the method of claim 1.

**20.** A computer apparatus, comprising means for carrying out steps e) to h) of the method of claim 1.


**Patentansprüche**

**1.** Verfahren zum Identifizieren eines unbekannten Proteins, das in einer Probe enthalten ist, auf der Grundlage der Wanderungseigenschaften von Peptiden, die aus dem unbekannten Protein erzeugt werden, auf einem Substrat, wobei das Verfahren die folgenden Schritte umfasst:

(a) Durchführen einer enzymatischen oder chemischen Spaltung mit der Probe, die das unbekannte Protein enthält, wodurch ein Pool von proteinspezifischen Peptidfragmenten erhalten wird;

(b) Einbringen von wenigstens zwei Markerpeptidverbindungen mit bekannter Aminosäuresequenz in den Pool von proteinspezifischen Peptidfragmenten, wodurch eine Assayprobe erhalten wird;

(c) Durchführen eines zweidimensionalen Trennschritts mit der Assayprobe auf der Oberfläche eines Trennsubstrats, wobei der zweidimensionale Trennschritt aus Folgendem besteht:

(i) einem ersten Trennschritt, wodurch die proteinspezifischen Peptidfragmente und die wenigstens zwei Markerverbindungen entlang der X-Achse auf der Oberfläche eines ersten Trennsubstrats wandern; und

(ii) einem zweiten Trennschritt, wodurch die proteinspezifischen Peptidfragmente und die Markerverbindungen entlang der Y-Achse auf der Oberfläche eines zweiten Trennsubstrats wandern;

wobei (1) das erste und das zweite Trennsubstrat identisch sind oder (2) das erste und das zweite Trennsubstrat unterschiedlich sind;

wodurch aus dem Pool von Peptidfragmenten und den Markerverbindungen, die anfangs in der Assayprobe enthalten waren, eine zweidimensionale Anordnung von Proteinflecken entsteht;

(d) Bestimmen des experimentellen Lokalisierungswerts für jeden Peptidfleck, der in der zweidimensionalen Anordnung enthalten ist, wobei der experimentelle Lokalisierungswert aus den X- und Y-Koordinaten für jeden Peptidfleck, der in der zweidimensionalen Anordnung enthalten ist, in Bezug auf einen einzigen vorbestimmten Ursprung in der Anordnung besteht;

(e) Vergleichen des in Schritt (d) bestimmten experimentellen Lokalisierungswerts für jede Markerverbindung mit einem vorbestimmten Wert für den theoretischen Lokalisierungswert der Markerverbindung auf der zweidimensionalen Anordnung und dann Berechnen von zwei Korrekturfaktoren, nämlich:

(i) eines ersten Korrekturfaktors "Ce", der den Abstand entlang der X-Achse der zweidimensionalen Anordnung zwischen den experimentellen und theoretischen X-Koordinaten, die für die Markerverbindung bestimmt wurden, kennzeichnet; und

(ii) eines zweiten Korrekturfaktors "CrF", der den Abstand entlang der Y-Achse der zweidimensionalen Anordnung zwischen den experimentellen und theoretischen Y-Koordinaten, die für die Markerverbindung bestimmt wurden, kennzeichnet;

(f) Berechnen der korrigierten X- und Y-Koordinaten für jeden Peptidfleck, der den durch die Spaltung des unbekannten Proteins erzeugten Peptidfragmenten entspricht, auf der zweidimensionalen Anordnung durch Anwenden der Korrekturfaktoren "Ce" und "CrF" jeweils auf die in Schritt (d) bestimmten experimentellen X- und Y-Koordinaten des Peptidflecks, wodurch eine Menge von korrigierten Lokalisierungswerten für die Menge von Peptidflecken, die den Peptidfragmenten entsprechen, erhalten wird;

(g) Vergleichen der Menge von korrigierten Lokalisierungswerten, die am Ende von Schritt (f) aus der Menge von Peptidflecken erhalten werden, wobei die Menge von korrigierten Lokalisierungswerten das unbekannte Protein charakterisiert, mit entweder:

(i) einer vorher aufgebauten geordneten Sammlung von Mengen von theoretischen Lokalisierungswerten, wobei jede Menge von theoretischen Lokalisierungswerten, die in der geordneten Sammlung enthalten ist, die Menge von Peptidfragmenten charakterisiert, von der man erwartet, dass sie erzeugt wird, indem man ein gegebenes Protein mit einer bekannten Aminosäuresequenz derselben enzymatischen oder chemischen Spaltung unterzieht, wie sie in Schritt (a) des Verfahrens mit dem unbekannten Protein experimentell durchgeführt wurde; oder

(ii) aufeinanderfolgenden Mengen von theoretischen Lokalisierungswerten, wobei jede Menge von theoretischen Lokalisierungswerten die Menge von Peptidfragmenten charakterisiert, von der man erwartet, dass sie erzeugt wird, indem man ein gegebenes Protein mit einer bekannten Aminosäuresequenz derselben enzymatischen oder chemischen Spaltung unterzieht, wie sie in Schritt (a) des Verfahrens mit dem unbekannten Protein experimentell durchgeführt wurde, und wobei jede Menge von theoretischen Lokalisierungswerten erzeugt wird, unmittelbar bevor sie mit der Menge von korrigierten Lokalisierungswerten für die Menge von Proteinflecken, die der Menge von Peptidfragmenten entspricht, die in Schritt a) aus dem unbekannten Protein erzeugt wurden, verglichen wird;

(h) Auswählen aus den Mengen von theoretischen Lokalisierungswerten, die in der in Schritt (g-i) definierten geordneten Sammlung von Mengen enthalten sind, oder alternativ dazu aus den Mengen von theoretischen Lokalisierungswerten, die in Schritt (g-ii) definiert sind: (i) der Menge oder Mengen, die die größte Ähnlichkeit mit der am Ende von Schritt (f) für das zu identifizierende unbekannte Protein erhaltenen Menge von korrigierten Lokalisierungswerten aufweisen, oder (ii) der Menge oder Mengen, die mit dieser identisch sind, wodurch für das unbekannte Protein zugeordnet wird, dass es aus dem oder den Proteinen besteht, die der oder den ausgewählten Mengen entspricht.

2. Verfahren gemäß Anspruch 1, wobei die Proteinspaltung aus einer Enzymspaltung unter Verwendung eines Enzyms besteht, das aus der Gruppe ausgewählt ist, die aus Trypsin, Trypsin [lys-blockiert], Chymotrypsin, Clostipain, Prolin-Endopeptidase, Myxobacter-Protease, Armilaria-Protease, Staphylococcus-Protease und Endoproteinase Asp-N besteht.

3. Verfahren gemäß Anspruch 1, wobei die Proteinspaltung aus einer chemischen Spaltung unter Verwendung einer Verbindung besteht, die aus der Gruppe ausgewählt ist, die aus Bromcyan, 2-Nitro-5-thiocyanobenzoesäure und einer sauren Verbindung, die für die saure Hydrolyse bei pH 2,5 geeignet ist, besteht.

4. Verfahren gemäß Anspruch 1, wobei die wenigstens eine Peptidmarkerverbindung mit einem nachweisbaren Molekül markiert ist.

5. Verfahren gemäß Anspruch 1, wobei das unbekannte Protein oder die durch enzymatische oder chemische Spaltung davon abgeleiteten Peptidfragmente mit einem nachweisbaren Molekül markiert ist bzw. sind.

6. Verfahren gemäß Anspruch 1, wobei sowohl die wenigstens eine Markerverbindung als auch das unbekannte Protein oder die durch enzymatische oder chemische Spaltung davon abgeleiteten Peptidfragmente mit einem nachweisbaren Molekül markiert ist bzw. sind.

7. Verfahren gemäß Anspruch 6, wobei (i) die wenigstens eine Markerverbindung und (ii) das unbekannte Protein oder die durch enzymatische oder chemische Spaltung davon abgeleiteten Peptidfragmente jeweils mit zwei unterschiedlichen nachweisbaren Molekülen markiert sind.

8. Verfahren gemäß Anspruch 1, wobei in Schritt (c) der erste Trennschritt aus einer Hochspannungselektrophorese besteht und der zweite Trennschritt aus einer aufsteigenden Chromatographie besteht.

9. Verfahren gemäß Anspruch 1, wobei in Schritt (b) 2 bis 10 Markerpeptidverbindungen mit bekannten Aminosäuresequenzen eingebracht werden.

10. Verfahren gemäß Anspruch 1, wobei der vorbestimmte Wert für die theoretische Lokalisierung jeder Markerverbindung, wenn die Markerverbindung aus einem Markerprotein besteht, darin besteht, jeweils die theoretischen X- und Y-Koordinaten für ein Peptid bekannter Sequenz, wie der vorbestimmte Wert für jedes Markerpeptid, zu bestimmen, die wie folgt berechnet werden:

theoretischer X-Koordinatenwert:

(i) Bestimmen der Nettoladung des Markerpeptids mit der bekannten Aminosäuresequenz wie folgt:

(i-1) Berechnen der positiven Ladung des Markerpeptids gemäß der folgenden Formel (1):

$$\text{Nettoplus} = ([H^+]/Kd + [H^+]) + ([H^+]/pK1 + [H^+];$$

wobei $Kd = 10^{-PKr}$ ist und $pKr$ der pKa-Wert für die positiv geladenen Aminosäuren ist und $pK1$ der pKa-Wert der aminoterminalen Gruppe des Markerpeptids ist;
(i-2) Berechnen der negativen Ladung des Markerpeptids gemäß der folgenden Formel (2):

$$\text{Nettominus} = 1 - ([H^+]/Kd + [H^+]) + (1 - ([H^+]/pK2 + [H^+]));$$

wobei Kd = $10^{-pkr}$ ist und pKr der pKa-Wert für die negativ geladenen Aminosäuren ist und pK2 der pKa-Wert der carboxyterminalen Gruppe des Markerpeptids ist;

(i-3) Berechnen der Nettoladung des Markerpeptids gemäß der folgenden Formel (3):

$$\text{Nettoladung} = \text{Nettoplus} - \text{Nettominus};$$

(ii) Berechnen der elektrophoretischen Mobilität des Markerpeptids gemäß der folgenden Formel (4):

$$\text{Emobil} = \text{Nettoladung}/Mr^{(2.0/3.0)};$$

wobei Mr das in Dalton ausgedrückte Molekulargewicht des Markerpeptids ist;

theoretischer Y-Koordinatenwert:

(i) Berechnen des absoluten Y-Mobilitätswerts des Markerpeptids gemäß der folgenden Formel (5):

$$\text{AbsMob} = [rfaa_1 + rfaa_2 + ... + rfaa_n]/n,$$

wobei $rfaa_1$, $rfaa_2$, ..., $rfaa_n$ die jeweiligen Y-Achsen-Mobilitätswerte für jeden der in dem Markerprotein mit der bekannten Aminosäuresequenz enthaltenen Aminosäurereste ist und wobei "n" die Gesamtzahl der in dem Markerprotein enthaltenen Aminosäuren ist;

(ii) Berechnen eines korrigierten Werts für den absoluten Y-Mobilitätswert des Markerpeptids, wobei der korrigierte Wert (a) den Prozentsatz an Aminosäuren mit geringer Mobilität und (b) die Zahl der Aminosäuren mit hoher Mobilität in der Sequenz des Markerpeptids berücksichtigt, gemäß der folgenden Formel (6):

$$\text{Rf} = \text{AbsMob} \cdot \text{CF}$$

wobei CF der angewendete Korrekturfaktor ist.

11. Verfahren gemäß Anspruch 10, wobei der theoretische Wert für die absolute Y-Mobilität (Rf) für das Markerpeptid gemäß den folgenden Bedingungen berechnet wird:

wenn 53 < %lowRf < 99 und gmob > 0,36:

$$Rf(i,j) = \sum \frac{mob(i)}{size} * \left[ \frac{-0.9(\%lowRf)}{44} + \frac{23.15}{11} \right] + \sum \frac{highRf(j)}{size}$$

wenn 53 < %lowRf < 99:

$$Rf(i)=\sum \frac{mob(i)}{size}*\left[\frac{-0.9*(\%lowRf)}{44}+\frac{23.15}{11}\right]$$

wenn gmob > 0,36:

$$Rf(i,j)=\sum \frac{mob(i)}{size}+\sum \frac{highRf(j)}{size}$$

sonst:

$$Rf(i)=\sum \frac{mob(i)}{size}$$

wobei:

- Rf(i,j) und Rf(i) beide den Rf-Wert bedeuten, bei dem die obigen unterschiedlichen Bedingungen erfüllt sind;
- $\Sigma$[mob(i)/size] für AbsMob steht;
- %lowRf der Prozentsatz an Aminosäuren mit geringer Mobilität ist, die in der Sequenz des Markerpeptids enthalten ist, welcher verwendet wird, um den Korrekturfaktor zu berechnen, der den Prozentsatz an Aminosäuren mit geringer Mobilität berücksichtigt, wobei der Korrekturfaktor gemäß der obigen Formel in Abhängigkeit davon, welche Bedingung erfüllt ist, berechnet wird;
- gmob die allgemeine Mobilität des betreffenden Peptids bedeutet;
- size die Zahl der in dem Peptid enthaltenen Aminosäurereste bezeichnet; und
- $\Sigma$[highRf(j)/size] für den Korrekturfaktor steht, der die Zahl der Aminosäuren mit hoher Mobilität in der Sequenz des Markerpeptids berücksichtigt.

12. Verfahren gemäß Anspruch 1, wobei in Schritt (g) die (i) vorher aufgebaute geordnete Sammlung von Mengen von theoretischen Lokalisierungswerten oder die (ii) aufeinanderfolgenden Mengen von theoretischen Lokalisierungswerten, wobei jede Menge von theoretischen Lokalisierungswerten die Peptidfragmente charakterisiert, von denen man erwartet, dass sie erzeugt werden, indem man ein gegebenes Protein mit einer bekannten Aminosäuresequenz derselben enzymatischen oder chemischen Spaltung unterzieht, wie sie in Schritt (a) des Verfahrens mit dem unbekannten Protein experimentell durchgeführt wurde, oder alternativ dazu jede Menge von theoretischen Werten, die erzeugt wird, nach einem Verfahren erzeugt wird, das die folgenden Schritte umfasst:

g1) Erzeugen einer Menge von Untersequenzen aus der vollständigen Aminosäuresequenz eines bekannten Proteins, wobei jede Untersequenz der Aminosäuresequenz eines Peptidfragments entspricht, von dem man erwartet, dass es erzeugt wird, wenn das bekannte Protein derselben enzymatischen oder chemischen Spaltung unterzogen wird, wie sie in Schritt (a) des Verfahrens mit dem unbekannten Protein experimentell durchgeführt wurde;
g2) Berechnen des theoretischen Lokalisierungswerts für jede in Schritt g1) erzeugte Untersequenz, indem man eine identische Berechnung durchführt wie bei der Berechnung des theoretischen Lokalisierungswerts der Markerverbindungen, wodurch eine Menge von theoretischen Lokalisierungswerten, die der Menge von Untersequenzen entspricht, erzeugt wird, wobei die Menge von Untersequenzen dem bekannten Protein entspricht;
g3) Vergleichen der in Schritt (f) erhaltenen Menge von korrigierten Lokalisierungswerten des unbekannten Proteins mit jeweils jeder Menge von theoretischen Werten, die jedem bekannten Protein entsprechen, das in der geordneten Sammlung von Mengen von theoretischen Werten enthalten ist.

13. Verfahren gemäß Anspruch 12, wobei in Schritt g2) der theoretische Lokalisierungswert für die Untersequenz, die einen oder mehrere chemisch modifizierte Aminosäurereste aufweist, ebenfalls berechnet wird, wobei in Schritt (h) für das unbekannte (chemisch modifizierte) Protein zugeordnet wird, dass es aus dem oder den Proteinen besteht, die der oder den ausgewählten Mengen entspricht, die in der geordneten Sammlung von Mengen enthalten sind.

**14.** Verfahren gemäß Anspruch 1, wobei dann, wenn in Schritt h) keine Menge von theoretischen Werten ausgewählt wird, die Schritte g) und h) des Verfahrens ein zweites Mal mit Mengen von theoretischen Lokalisierungswerten durchgeführt werden, wobei jede Menge theoretische Lokalisierungswerte enthält, die den Peptidfragmenten entsprechen, von denen man erwartet, dass sie erzeugt werden, wenn die enzymatische oder chemische Spaltung, der das entsprechende bekannte Protein unterzogen wird, nicht vollständig ist.

**15.** Verfahren gemäß Anspruch 1, wobei dann, wenn in Schritt h) mehr als eine Menge von theoretischen Lokalisierungswerten ausgewählt wird, jeder der ausgewählten Mengen von theoretischen Lokalisierungswerten eine Ähnlichkeitspunktzahl zugeordnet wird.

**16.** Verfahren gemäß Anspruch 15, wobei in Schritt h) für das unbekannte Protein zugeordnet wird, dass es aus dem Protein besteht, das derjenigen ausgewählten Menge von theoretischen Lokalisierungswerten entspricht, der die höchste Ähnlichkeitspunktzahl zugeordnet wurde.

**17.** Verfahren gemäß Anspruch 1, wobei die Schritte d) bis h) durchgeführt werden, indem man Anweisungen einer Computersoftware ausführt, wobei die Computersoftware zuvor in den Speicher einer Computerapparatur geladen wurde.

**18.** Computerspeichermodul, der von einer Computerapparatur gelesen werden kann, wobei der Computerspeichermodul Anweisungen umfasst, die bewirken, dass die Computerapparatur die Schritte d) bis h) des Verfahrens gemäß Anspruch 1 durchführt, wenn es in die Computerapparatur geladen wird.

**19.** Computerprogramm, das Softwarecode umfasst, der geeignet ist, die Schritte e) bis h) des Verfahrens von Anspruch 1 durchzuführen.

**20.** Computerapparatur, die Einrichtungen zur Durchführung der Schritte e) bis h) des Verfahrens von Anspruch 1 umfasst.

**Revendications**

**1.** Procédé d'identification d'une protéine inconnue contenue dans un échantillon, basé sur les propriétés de migration sur un substrat des peptides générés à partir de ladite protéine inconnue, ledit procédé comprenant les étapes qui consistent à :

(a) soumettre ledit échantillon contenant ladite protéine inconnue à un clivage enzymatique ou chimique, par lequel un groupe de fragments peptidiques spécifiques de la protéine est obtenu;
(b) incorporer dans ledit groupe de fragments peptidiques spécifiques de la protéine au moins deux composés peptidiques de référence dont la séquence d'acides aminés est connue, moyennant quoi on obtient un échantillon d'analyse ;
(c) soumettre ledit échantillon d'analyse à une étape de séparation bidimensionnelle sur la surface d'un substrat de séparation, ladite étape de séparation bidimensionnelle consistant en :

(i) une première étape de séparation par laquelle les fragments peptidiques spécifiques de la protéine et les au moins deux composés de référence migrent selon l'axe des abscisses (X) sur la surface d'un premier substrat de séparation ; et
(ii) une seconde étape de séparation moyennant laquelle les fragments peptidiques spécifiques de la protéine et lesdits composés de référence migrent selon l'axe des ordonnées (Y) sur la surface d'un second substrat de séparation,
(1) le premier et le second substrat de séparation étant identiques ou
(2) le premier et le second substrat de séparation étant distincts,

moyennant quoi on forme une matrice bidimensionnelle de taches protéiques à partir du groupe de fragments peptidiques et des composés de référence qui étaient initialement contenus dans l'échantillon d'analyse ;
(d) déterminer la valeur de localisation expérimentale pour chaque tache peptidique contenue dans la matrice bidimensionnelle, ladite valeur de localisation expérimentale consistant en les coordonnées X et Y pour chaque tache peptidique contenue dans la matrice bidimensionnelle, par rapport à un point d'origine unique prédéterminé dans la matrice;

(e) comparer la valeur de localisation expérimentale déterminée à l'étape (d) pour chaque composé de référence à une valeur prédéterminée pour la valeur de localisation théorique dudit composé de référence sur la matrice bidimensionnelle, et calculer ensuite deux facteurs de correction, respectivement :

> (i) un premier facteur de correction "Ce" qui est représentatif de la distance, selon l'axe des abscisses (X) de la matrice bidimensionnelle, entre les coordonnées X expérimentale et théorique déterminées pour ledit composé de référence ; et
>
> (ii) un second facteur de correction "CrF" qui est représentatif de la distance, selon l'axe des ordonnées (Y) de la matrice bidimensionnelle, entre les coordonnées Y expérimentale et théorique déterminées pour ledit composé de référence ;

(f) calculer, pour chaque tache peptidique correspondant aux fragments peptidiques générés par le clivage de ladite protéine inconnue, les coordonnées X et Y corrigées de ladite tache peptidique sur la matrice bidimensionnelle en appliquant aux coordonnées X et Y expérimentales de ladite tache peptidique déterminées à l'étape (d) les facteurs de correction "Ce" et "CrF" déterminés à l'étape (e), respectivement, moyennant quoi on obtient un ensemble de valeurs de localisation corrigées pour l'ensemble des taches peptidiques correspondant aux fragments peptidiques ;

(g) comparer l'ensemble des valeurs de localisation corrigées obtenu à partir de l'ensemble des taches peptidiques à la fin de l'étape (f), ledit ensemble de valeurs de localisation corrigées caractérisant la protéine inconnue, soit à :

> (i) une collection ordonnée pré-construite d'ensembles de valeurs de localisation théoriques, dans laquelle chaque ensemble de valeurs de localisation théoriques contenu dans ladite collection ordonnée caractérise l'ensemble de fragments peptidiques que l'on s'attend à générer lorsque l'on soumet une protéine donnée ayant une séquence d'acides aminés connue au même clivage enzymatique ou chimique que celui réalisé expérimentalement sur la protéine inconnue à l'étape (a) du procédé ; soit à
>
> (ii) des ensembles successifs de valeurs de localisation théoriques, chaque ensemble de valeurs de localisation théoriques caractérisant l'ensemble de fragments peptidiques que l'on s'attend à générer lorsque l'on soumet une protéine donnée ayant une séquence d'acides aminés connue au même clivage enzymatique ou chimique que celui réalisé expérimentalement sur la protéine inconnue à l'étape (a) du procédé, et chaque ensemble de valeurs de localisation théoriques étant généré juste avant sa comparaison avec l'ensemble de valeurs de localisation corrigées pour l'ensemble de taches protéiques correspondant à l'ensemble de fragments peptidiques qui a été généré à partir de la protéine inconnue à l'étape a) ;

(h) sélectionner, parmi les ensembles de valeurs de localisation théoriques contenus dans la collection ordonnée d'ensembles définie à l'étape (g-i), ou en variante parmi les ensembles de valeurs de localisation théoriques définis à l'étape (g-ii), (i) le ou les ensembles qui révèlent la plus grande similitude avec, ou (ii) le ou les ensembles qui sont identiques à, l'ensemble de valeurs de localisation corrigées obtenu à la fin de l'étape (f) pour la protéine inconnue à identifier, moyennant quoi on attribue à ladite protéine inconnue qu'elle consiste en la ou les protéines correspondant audit ou auxdits ensembles sélectionnés.

**2.** Procédé selon la revendication 1, dans lequel le clivage de protéine consiste en un clivage enzymatique au moyen d'une enzyme choisie dans le groupe constitué par la trypsine, la trypsine [Lys bloquée], la chymotrypsine, la clostripaine, la proline endopeptidase, la protéase de myxobacter, la protéase d'armilaria, la protéase staphylococcique et l'endoprotéinase Asp-N.

**3.** Procédé selon la revendication 1, dans lequel le clivage de protéine consiste en un clivage chimique au moyen d'un composé choisi dans le groupe constitué par le bromure de cyanogène, l'acide 2-nitro-5-thiocyanobenzoïque et un composé acide approprié pour l'hydrolyse acide à un pH de 2,5.

**4.** Procédé selon la revendication 1, dans lequel l'au moins un composé de référence peptidique est marqué au moyen d'une molécule détectable.

**5.** Procédé selon la revendication 1, dans lequel la protéine inconnue, ou les fragments peptidiques dérivés de celle-ci par un clivage enzymatique ou chimique, est (sont) marquée (marqués) au moyen d'une molécule détectable.

**6.** Procédé selon la revendication 1, dans lequel à la fois l'au moins un composé de référence et la protéine inconnue ou les fragments peptidiques dérivés de celle-ci par un clivage enzymatique ou chimique sont marqués au moyen d'une molécule détectable.

7. Procédé selon la revendication 6, dans lequel (i) l'au moins un composé de référence et (ii) la protéine inconnue, ou les fragments peptidiques dérivés de celle-ci par un clivage enzymatique ou chimique, sont respectivement marqués au moyen de deux molécules détectables distinctes.

8. Procédé selon la revendication 1, dans lequel, à l'étape (c), la première étape de séparation consiste en une électrophorèse à haute tension et la seconde étape de séparation consiste en une chromatographie ascendante.

9. Procédé selon la revendication 1, dans lequel de 2 à 10 composés peptidiques de référence dont les séquences d'acides aminés sont connues sont incorporés à l'étape (b).

10. Procédé selon la revendication 1, dans lequel la valeur prédéterminée pour la localisation théorique pour chaque composé de référence, lorsque ledit composé de référence consiste en une protéine de référence, consiste à déterminer respectivement les coordonnées X et Y théoriques pour un peptide de séquence connue tel que la dite valeur prédéterminée pour chaque peptide de référence est calculée comme suit :

   Valeur de coordonnée X théorique :

   (i) Déterminer la charge totale du peptide de référence ayant une séquence d'acides aminés connue, comme suit :

   (i-1) Calculer la charge positive (netplus) du peptide de référence conformément à la formule (1) suivante :

$$netplus = ([H^+] \,/\, Kd + [H^+]) + ([H^+] \,/\, pK1 + [H^+]),$$

   dans laquelle Kd est $10^{x\,(-pKr)}$ et pKr est le pKa pour les acides aminés chargés positivement et pK1 est le pKa du groupe amino terminal du peptide de référence.
   (i-2) Calculer la charge négative (netminus) du peptide de référence conformément à la formule (2) suivante :

$$netminus = 1-([H^+] \,/\, Kd + [H^+]) + (1 - ([H^+] \,/\, pK2 + [H^+]))$$

   dans laquelle Kd est $10^{x\,(-pKr)}$ et pKr est le pKa pour les acides aminés chargés négativement et pK2 est le pKa du groupe carboxy terminal du peptide de référence.
   (i-3) Calculer la charge nette (netcharge) du peptide de référence conformément à la formule (3) suivante :

$$netcharge = netplus - netminus$$

   (ii) Calculer la mobilité électrophorétique du peptide de référence conformément à la formule (4) suivante :

$$Emobil. = netcharge/\, Mr^{\,(2,0/3,0)},$$

   dans laquelle Mr est la masse moléculaire du peptide de référence exprimée en daltons.
   Valeur de coordonnée Y théorique :

   (i) Calculer la valeur de mobilité Y absolue dudit peptide de référence conformément à la formule (5) suivante :

$$AbsMob = [rf\text{-}aa_1 + rfaa_2 + ... + rfaa_n]/n$$

dans laquelle $rf\text{-}aa_1$, $rfaa_2$, ..., $rfaa_n$ sont les valeurs de mobilité respectives selon l'axe des ordonnées pour chacun des résidus d'acide aminé contenus dans ladite protéine de référence ayant une séquence d'acides aminés connue et dans laquelle "n" est le nombre total d'acides aminés contenus dans ledit peptide de référence ;

(ii) Calculer la valeur corrigée de la valeur de mobilité Y absolue dudit peptide de référence, moyennant quoi la valeur corrigée tient compte (a) du pourcentage d'acides aminés de faible mobilité et (b) du nombre d'acides aminés de forte mobilité dans la séquence dudit peptide de référence, conformément à la formule (6) suivante :

$$Rf = AbsMob \cdot FC$$

dans laquelle FC est le facteur de correction appliqué.

**11.** Procédé selon la revendication 10, dans lequel la valeur théorique pour la mobilité Y absolue (Rf) pour ledit peptide de référence est calculée conformément aux conditions suivantes :

si 53 < % de faible Rf < 99 et gmob > 0,36 :

$$Rf(i,j) = \sum \frac{mob(i)}{taille} * \left[ \frac{-0,9 \,(\% \text{ de faible Rf})}{44} + \frac{23,15}{11} \right] + \sum \frac{Forte\ Rf(j)}{taille}$$

si 53 < % de faible Rf < 99 :

$$Rf(i) = \sum \frac{mob(i)}{taille} * \left[ \frac{-0,9 * (\% \text{ de faible Rf})}{44} + \frac{23,15}{11} \right]$$

si gmob > 0,36 :

$$Rf(i,j) = \sum \frac{mob(i)}{taille} + \sum \frac{forte\ Rf(j)}{taille}$$

sinon :

$$Rf(i) = \sum \frac{mob(i)}{taille}$$

dans lesquelles :

- Rf (i,j) et Rf (i) désignent tous deux la valeur de Rf dans laquelle les différentes conditions ci-dessus sont

remplies ;

- $\Sigma$ [mob (i) / taille] représente l'AbsMob ;

- le % de faible Rf est la quantité en pourcentage d'acides aminés de faible mobilité contenus dans la séquence dudit peptide de référence que l'on utilise pour calculer le facteur de correction qui tient compte du pourcentage d'acides aminés de faible mobilité et ledit facteur de correction étant calculé conformément à la formule ci-dessus, selon la condition qui est remplie ;

- gmob désigne la mobilité générale du peptide à l'étude ;

- taille indique le nombre de résidus d'acides aminés contenus dans le peptide ; et

- E [forte Rf (j) / taille] représente le facteur de correction qui tient compte du nombre d'acides aminés de forte mobilité dans la séquence dudit peptide de référence.

**12.** Procédé selon la revendication 1, dans lequel on génère, à l'étape (g), la (i) collection ordonnée pré-construite d'ensembles de valeurs de localisation théoriques ou les (ii) ensembles successifs de valeurs de localisation théoriques, chaque ensemble de valeurs de localisation théoriques caractérisant les fragments peptidiques que l'on s'attend à générer lorsque l'on soumet une protéine donnée ayant une séquence connue au même clivage enzymatique ou chimique que celui réalisé expérimentalement sur la protéine inconnue à l'étape (a) du procédé, ou en variante chaque ensemble de valeurs théoriques qui est généré, par un procédé qui comprend les étapes suivantes :

g1) génération, à partir de la séquence d'acides aminés complète d'une protéine connue, d'un ensemble de sous-séquences, dans lequel chaque sous-séquence correspond à la séquence d'acides aminés d'un fragment peptidique que l'on s'attend à générer lorsque l'on soumet ladite protéine connue au même clivage enzymatique ou chimique que celui réalisé expérimentalement sur la protéine inconnue à l'étape (a) du procédé ;

g2) calcul, pour chaque sous-séquence générée à l'étape g1), de la valeur de localisation théorique de ladite sous-séquence en réalisant un calcul identique à celui qui est réalisé pour calculer la valeur de localisation théorique des composés de référence, moyennant quoi on génère un ensemble de valeurs de localisation théoriques correspondant audit ensemble de sous-séquences, ledit ensemble de sous-séquences correspondant à ladite protéine connue ;

g3) comparaison de l'ensemble de valeurs de localisation corrigées de ladite protéine inconnue obtenu à l'étape (f) avec, respectivement, chaque ensemble de valeurs théoriques correspondant à toutes les protéines connues que contient la collection ordonnée d'ensembles de valeurs théoriques.

**13.** Procédé selon la revendication 12, dans lequel, à l'étape g2, on calcule également la valeur de localisation théorique de ladite sous-séquence ayant un ou plusieurs résidus d'acides aminés chimiquement modifiés, moyennant quoi, à l'étape (h), on attribue à ladite protéine inconnue (chimiquement modifiée) qu'elle consiste en la ou les protéines correspondant audit ou auxdits ensembles sélectionnés contenus dans la collection ordonnée d'ensembles.

**14.** Procédé selon la revendication 1, dans lequel, lorsque aucun ensemble de valeurs théoriques n'est sélectionné à l'étape h), alors les étapes g) et h) du procédé sont réalisées une seconde fois avec des ensembles de valeurs de localisation théoriques, chaque ensemble contenant des valeurs de localisation théoriques qui correspondent aux fragments peptidiques que l'on s'attend à générer si le clivage enzymatique ou chimique auquel on soumet la protéine connue correspondante n'est pas achevé.

**15.** Procédé selon la revendication 1, dans lequel, lorsque plus d'un ensemble de valeurs de localisation théoriques est sélectionné à l'étape h), alors une valeur de note de similitude est attribuée à chaque ensemble de valeurs de localisation théoriques sélectionné.

**16.** Procédé selon la revendication 15, dans lequel, à l'étape h), on attribue à la protéine inconnue qu'elle consiste en la protéine correspondant audit ensemble de valeurs de localisation théoriques sélectionné auquel a été attribué la valeur de note de similitude la plus élevée.

**17.** Procédé selon la revendication 1, dans lequel les étapes d) à h) sont réalisées en exécutant les instructions d'un logiciel informatique, lequel logiciel informatique ayant été précédemment chargé dans la mémoire d'un appareil de type ordinateur.

**18.** Support de mémoire informatique que l'on peut lire au moyen d'un appareil de type ordinateur, ledit support de mémoire informatique comprenant des instructions pour faire réaliser à l'appareil de type ordinateur les étapes d) à h) du procédé selon la revendication 1 lorsqu'elles sont chargées dans ledit appareil de type ordinateur.

**19.** Programme informatique comprenant un code de logiciel adapté pour réaliser les étapes e) à h) du procédé de la revendication 1.

**20.** Appareil de type ordinateur comprenant les moyens nécessaires pour réaliser les étapes e) à h) du procédé de la revendication 1.

```
┌─────────────────┐   ┌─────────────┐   ┌──────────────────────┐   ┌───────────────┐   ┌─────────────────────┐   ┌─────────────────┐
│ First protein   │   │ Second      │   │ Protein Visualization│   │               │   │ Analyse protein     │   │ Select proteins │
│ separation step.│──▶│ protein     │──▶│ (Silver staining,    │──▶│ Image stained │──▶│ output to           │──▶│ for excission   │
│ (Isolelectric   │   │ separation  │   │ fluorescence         │   │ gel           │   │ construct protein   │   │                 │
│ Focussing)      │   │ step        │   │ autoradiography)     │   │               │   │ Profile             │   │                 │
└─────────────────┘   │ (SDS-PAGE)  │   └──────────────────────┘   └───────────────┘   └─────────────────────┘   └─────────────────┘
                      └─────────────┘                                                                                      │
                                                                                                                          ▼
   ┌───────────────────┐   ┌──────────────────────┐   ┌───────────────┐   ┌───────────────┐   ┌─────────────────┐
   │ First peptide     │   │ pH selection and     │   │ Lyophilization│   │ Proteolysis   │   │ Excise selected │
   │ separation TLC    │◀──│ peptide marker       │◀──│               │◀──│               │◀──│ protein(s)      │
   │ at fixed pH       │   │ addition             │   │               │   │               │   │                 │
   └───────────────────┘   └──────────────────────┘   └───────────────┘   └───────────────┘   └─────────────────┘
             │
             ▼
   ┌───────────────────┐   ┌──────────────────────┐   ┌───────────────┐   ┌─────────────────────┐
   │ Second peptide    │   │ Peptide visualization│   │ Image TLC     │   │ Aquire peptide      │
   │ separation Solvent│──▶│ (fluorescence or     │──▶│ plate         │──▶│ position from TLC   │
   │ Chomatography     │   │ autoradiography      │   │               │   │ plate               │
   └───────────────────┘   └──────────────────────┘   └───────────────┘   └─────────────────────┘
                                                                                    │
                                                                                    ▼
   ┌───────────────────┐   ┌──────────────────────┐   ┌───────────────┐   ┌─────────────────────┐
   │ Create array of   │   │                      │   │ Calculate     │   │ Aquire peptide      │
   │ corrected peptide │◀──│ Correct Peptide      │◀──│ correction    │◀──│ marker position     │
   │ mobilities for rF │   │ mobilities           │   │ values        │   │                     │
   │ and emobil        │   └──────────────────────┘   └───────────────┘   └─────────────────────┘
   └───────────────────┘
       │      │    │
       ▼      │    ▼
   ┌────────┐ │  ┌──────────────────────┐   ┌─────────────────────┐
   │ Write  │ │  │ Compare peptide      │   │ Identify peptide    │
   │ resolve│ │  │ mobilities in both   │──▶│ profiles and        │
   │ file   │ │  │ emob and rF against  │   │ modification sites  │
   │containing│ │  │ resolution data base │   └─────────────────────┘
   │ array. │ │  │ of known protein     │
   └────────┘ │  │ sequences            │
              │  └──────────────────────┘
              ▼
   ┌──────────────────────┐   ┌─────────────────┐   ┌─────────────────────┐
   │ Calculate peptide    │   │ Compare to      │   │ Identify peptide    │
   │ mobility profiles for│──▶│ corrected       │──▶│ profiles and        │
   │ each protein in      │   │ peptide profile │   │ modification sites  │
   │ existing databases   │   └─────────────────┘   └─────────────────────┘
   └──────────────────────┘
```

FIGURE 1

FIGURE 2

Read image of peptide map

Is image RGB or TIFF

Correct image for tags
or reject image

Display image

Analyse image and calculate
peptide coordinates

Manual input of marker
peptide position and origin

Manual Confirmation of
peptide coordinates

Calculate local
correction factor

Apply correction factor to
peptide coordinate array
calculate peptide fingerprint
array.

Write image, peptide fingerprint arrays (observed and corrected)
and marker peptide position to a specific resolve
format file.

# FIGURE 3

Read image and annotate

Define peptide mobility area (4b)

Spot identification (4c)

Position Landmark peptides (4d)

Calculate conversion ratio (4e)

Calculate ratio areas (4f)

Create peptide mobility array.

4b
Non electrophoretic area (pH 1.9)
Non chromatographic area
Origin

4c
Low level filter for imperfections
Identify points of interest
Contour identification
Write peptide pixel array

4d

4e
X4
X3
X2
X1

4f
1   2   3   4   4'

FIGURE 4

FIGURE 5

1, Origin.
2, Upper chromatography boundary
3, Direction of electrophoresis
4, Chromatography

# FIGURE 6

FIGURE 7

FIGURE 8A

FIGURE 8B

FIGURE 9

FIGURE 10A

FIGURE 10B

FIGURE 10C

FIGURE 10D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0155721 A
- WO 0107920 A
- WO 9823950 A
- US 6064754 A
- US 20020098595 A
- US 5073963 A
- US 5138168 A

### Non-patent literature cited in the description

- Handbook of Fluorescent Probes. Molecular Probes, Inc,
- Molecular Probes Handbook of Fluorescent Probes
- **P. SOILLE.** Morphological Image Analysis, principle and applications. Springer, 1998
- **R. DETRICHE.** *The International Journal of Computer Vision,* 1987, vol. 1 (2), 167-187
- **BOYLE WJ ; VAN DER GEER P ; HUNTER T.** Phosphopeptide mapping and phosphoamino acid analysis by two-dimensional separation on thin layer cellulose plates. *Methods Enzymol.,* 1991, vol. 201, 110-49
- **GIRARD F ; STRAUSFELD U ; FERNANDEZ A ; LAMB NJ.** Cyclin A is required for the on set of DNA replication in mammalian fibroblasts. *Cell,* 1991, vol. 67, 1169-79
- **KITZMANN M ; VANDROMME M ; SCHAEFFER V ; CARNAC G ; LABBE JC ; LAMB N ; FERNANDEZ A.** cdl1- and cdk2-mediated phosphorylation of MyoD Ser200 in growing C2 myoblasts: role in modulating MyoD half-life and myogenic ac tivity. *Mol Cell Biol.,* 1999, vol. 19, 3167-76
- **LAMB NJ ; FERNANDEZ A ; FERAMISCO JR ; WELCH WJ.** Modulation of vimentin containing intermediate filament distribution and phosphorylation in living fibroblasts by the cAMP-dependent protein kinase. *J. Ce'll Biol.,* 1989, vol. 108, 2409-22
- **PEARSON WR.** Flexible sequence similarity searching with the FASTA3 program package. *Methods Mol Biol.,* 2000, vol. 132, 185-219
- **SOILLE, P.** Morphological Image analysis: Principles and applications. Springer Verlag, 1999
- **DEUTSCHER, M.P. ; ABELSON J.N.** Guide to Protein Purification. *Methods in Enzymology,* 1990, vol. 182, ISBN 0122135857


SEQUENCE LISTING

<110> Centre National de la Recherche Scientifique

<120> A.method for identifying unknown proteins or for identifying chemical modification events undergone by proteins in a sample

<130> L849-FR CNRS

<140>
<141>

<160> 24

<170> PatentIn Ver. 2.1

<210> 1

<211> 23
<212> PRT
<213> Homo sapiens

<400> 1

```
Thr Lys Ile Glu Lys Ile Glu Lys Ile Gly Glu Gly Thr Tyr Gly Val
 1               5                  10                  15
Val Tyr Lys Gly Arg His Cys
             20
```

<210> 2
<211> 9
<212> PRT
<213> Homo sapiens

<400> 2

```
Tyr Pro Tyr Asp Val Pro Asp Tyr Ala
 1               5
```

<210> 3
<211> 10
<212> PRT
<213> Homo sapiens

<400> 3

```
Glu Gln Lys Leu Ile Ser Glu Glu Asp Leu
 1               5                  10
```

<210> 4
<211> 4
<212> PRT
<213> Homo sapiens

<400> 4

```
Met Ser Thr Arg
 1
```

<210> 5
<211> 15
<212> PRT
<213> Homo sapiens

<400> 5

```
Met Phe Gly Gly Pro Gly Thr Ala Ser Arg Pro Ser Ser Ser Arg
 1               5                  10                  15
```

<210> 6

EP 1 577 823 B1

<211> 10
<212> PRT
<213> Homo sapiens

<400> 6

Leu Gly Asp Leu Tyr Glu Glu Glu Met Arg
1               5                   10

<210> 7
<211> 9
<212> PRT
<213> Homo sapiens

<400> 7

Asp Asn Leu Ala Glu Asp Ile Met Arg
1                 5

<210> 8
<211> 8
<212> PRT
<213> Homo sapiens

<400> 8

Leu Gln Glu Glu Met Leu Gln Arg
1                 5

<210> 9
<211> 3
<212> PRT
<213> Homo sapiens

<400> 9

Gln Met Arg
1

<210> 10
<211> 19
<212> PRT
<213> Homo sapiens

<400> 10

Glu Met Glu Glu Asn Phe Ala Val Glu Ala Ala Asn Tyr Gln Asp Thr
1               5                   10                  15

Ile Gly Arg

<210> 11
<111> 9

63

<212> PRT
<213> Homo sapiens

<400> 11

```
Leu Gln Asp Glu Ile Gln Asn Met Lys
1               5
```

<210> 12
<211> 5
<212> PRT
<213> Homo sapiens

<400> 12

```
Glu Glu Met Ala Arg
1               5
```

<210> 13
<211> 11
<212> PRT
<213> Homo sapiens

<400> 13

```
Met Ala Leu Asp Ile Glu Ile Ala Thr Tyr Arg
1               5                   10
```

<210> 14
<211> 466
<212> PRT
<213> Homo sapiens

<400> 14

```
Met Ser Thr Arg Ser Val Ser Ser Ser Ser Tyr Arg Arg Met Phe Gly
1               5                   10                  15

Gly Pro Gly Thr Ala Ser Arg Pro Ser Ser Ser Arg Ser Tyr Val Thr
                20                  25                  30

Thr Ser Thr Arg Thr Tyr Ser Leu Gly Ser Ala Leu Arg Pro Ser Thr
                35                  40                  45

Ser Arg Ser Leu Tyr Ala Ser Ser Pro Gly Gly Val Tyr Ala Thr Arg
        50                  55                  60
```

```
Ser Ser Ala Val Arg Leu Arg Ser Ser Val Pro Gly Val Arg Leu Leu
65                  70                  75                  80

Gln Asp Ser Val Asp Phe Ser Leu Ala Asp Ala Ile Asn Thr Glu Phe
                85                  90                  95

Lys Asn Thr Arg Thr Asn Glu Lys Val Glu Leu Gln Glu Leu Asn Asp
            100                 105                 110

Arg Phe Ala Asn Tyr Ile Asp Lys Val Arg Phe Leu Glu Gln Gln Asn
            115                 120                 125

Lys Ile Leu Leu Ala Glu Leu Glu Gln Leu Lys Gly Gln Gly Lys Ser
    130                 135                 140

Arg Leu Gly Asp Leu Tyr Glu Glu Glu Met Arg Glu Leu Arg Arg Gln
145                 150                 155                 160

Val Asp Gln Leu Thr Asn Asp Lys Ala Arg Val Glu Val Glu Arg Asp
                165                 170                 175

Asn Leu Ala Glu Asp Ile Met Arg Leu Arg Glu Lys Leu Gln Glu Glu
            180                 185                 190

Met Leu Gln Arg Glu Glu Ala Glu Asn Thr Leu Gln Ser Phe Arg Gln
    195                 200                 205

Asp Val Asp Asn Ala Ser Leu Ala Arg Leu Asp Leu Glu Arg Lys Val
    210                 215                 220

Glu Ser Leu Gln Glu Glu Ile Ala Phe Leu Lys Lys Leu His Glu Glu
225                 230                 235                 240

Glu Ile Gln Glu Leu Gln Ala Gln Ile Gln Glu Gln His Val Gln Ile
                245                 250                 255

Asp Val Asp Val Ser Lys Pro Asp Leu Thr Ala Ala Leu Arg Asp Val
            260                 265                 270

Arg Gln Gln Tyr Glu Ser Val Ala Ala Lys Asn Leu Gln Glu Ala Glu
    275                 280                 285

Glu Trp Tyr Lys Ser Lys Phe Ala Asp Leu Ser Glu Ala Ala Asn Arg
    290                 295                 300

Asn Asn Asp Ala Leu Arg Gln Ala Lys Gln Glu Ser Thr Glu Tyr Arg
305                 310                 315                 320

Arg Gln Val Gln Ser Leu Thr Cys Glu Val Asp Ala Leu Lys Gly Thr
                325                 330                 335

Asn Glu Ser Leu Glu Arg Gln Met Arg Glu Met Glu Glu Asn Phe Ala
            340                 345                 350

Val Glu Ala Ala Asn Tyr Gln Asp Thr Ile Gly Arg Leu Gln Asp Glu
            355                 360                 365

Ile Gln Asn Met Lys Glu Glu Met Ala Arg His Leu Arg Glu Tyr Gln
    370                 375                 380
```

Asp Leu Leu Asn Val Lys Met Ala Leu Asp Ile Glu Ile Ala Thr Tyr
385                 390             395                 400

Arg Lys Leu Leu Glu Gly Glu Glu Ser Arg Ile Ser Leu Pro Leu Pro
            405                 410             415

Asn Phe Ser Ser Leu Asn Leu Arg Glu Thr Asn Leu Asp Ser Leu Pro
            420                 425                 430

Leu Val Asp Thr His Ser Lys Arg Thr Phe Leu Ile Lys Thr Val Glu
            435                 440                 445

Thr Arg Asp Gly Gln Val Ile Asn Glu Thr Ser Gln His His Asp Asp
    450                 455                 460

Leu Glu
465


<210> 15
<211> 19
<212> PRT
<213> Homo sapiens

<400> 15


Leu Gln Glu Glu Met Leu Gln Arg Glu Glu Ala Glu Asn Thr Leu Gln
    1               5               10                  15

Ser Phe Arg


<210> 16
<211> 22
<212> PRT
<213> Homo sapiens

<400> 16


Gln Met Arg Glu Met Glu Glu Asn Phe Ala Val Glu Ala Ala Asn Tyr
    1               5               10                  15

Gln Asp Thr Ile Gly Arg
            20


<210> 17
<211> 23
<212> PRT
<213> Homo sapiens

<400> 17

```
Met Phe Gly Gly Pro Gly Thr Ala Ser Arg Pro Ser Ser Ser Arg Ser
 1               5                   10                   15

Tyr Val Thr Thr Ser Thr Arg
                20
```

<210> 18
<211> 12
<212> PRT
<213> Homo sapiens

<400> 18

```
Ser Arg Leu Gly Asp Leu Tyr Glu Glu Glu Met Arg
 1               5                   10
```

<210> 19
<211> 14
<212> PRT
<213> Homo sapiens

<400> 19

```
Val Glu Val Glu Arg Asp Asn Leu Ala Glu Asp Ile Met Arg
 1               5                   10
```

<210> 20
<211> 20
<212> PRT
<213> Homo sapiens

<400> 20

```
Glu Tyr Gln Asp Leu Leu Asn Val Lys Met Ala Leu Asp Ile Glu Ile
 1               5                   10                   15

Ala Thr Tyr Arg
            20
```

<210> 21
<211> 16
<212> PRT
<213> Homo sapiens

<400> 21

```
Arg Met Phe Gly Gly Pro Gly Thr Ala Ser Arg Pro Ser Ser Ser Arg
 1               5                   10                   15
```

<210> 22

<211> 28
<212> PRT
<213> Homo sapiens

<400> 22

Glu Met Glu Glu Asn Phe Ala Val Glu Ala Ala Asn Tyr Gln Asp Thr
1               5                   10                  15

Ile Gly Arg Leu Gln Asp Glu Ile Gln Asn Met Lys
            20                  25

<210> 23
<211> 12
<212> PRT
<213> Homo sapiens

<400> 23

Met Ser Thr Arg Ser Val Ser Ser Ser Ser Tyr Arg
1               5                   10

<210> 24
<211> 11
<212> PRT
<213> Homo sapiens

<400> 24

Gly Thr Asn Glu Ser Leu Glu Arg Gln Met Arg
1               5                   10

1

1

SEQUENCE LISTING

<110> Centre National de la Recherche Scientifique

<120> A method for identifying unknown proteins or for identifying chemical modification events undergone by proteins in a sample

<130> L849-FR CNRS

<140>
<141>

<160> 24

<170> PatentIn Ver. 2.1

<210> 1
<211> 23
<212> PRT
<213> Homo sapiens

<400> 1

```
Thr Lys Ile Glu Lys Ile Glu Lys Ile Gly Glu Gly Thr Tyr Gly Val
 1               5                   10                  15

Val Tyr Lys Gly Arg His Cys
             20
```

<210> 2
<211> 9
<212> PRT
<213> Homo sapiens

<400> 2

```
Tyr Pro Tyr Asp Val Pro Asp Tyr Ala
 1               5
```

<210> 3
<211> 10
<212> PRT
<213> Homo sapiens

<400> 3

```
Glu Gln Lys Leu Ile Ser Glu Glu Asp Leu
 1               5                   10
```

<210> 4
<211> 4
<212> PRT
<213> Homo sapiens

<400> 4

```
Met Ser Thr Arg
 1
```

<210> 5
<211> 15
<212> PRT
<213> Homo sapiens

<400> 5

```
Met Phe Gly Gly Pro Gly Thr Ala Ser Arg Pro Ser Ser Ser Arg
 1               5                   10                  15
```

<210> 6
<211> 10
<212> PRT
<213> Homo sapiens

<400> 6

```
Leu Gly Asp Leu Tyr Glu Glu Glu Met Arg
 1               5                   10
```

<210> 7
<211> 9
<212> PRT
<213> Homo sapiens

<400> 7

```
Asp Asn Leu Ala Glu Asp Ile Met Arg
 1               5
```

<210> 8
<211> 8
<212> PRT
<213> Homo sapiens

<400> 8

```
Leu Gln Glu Glu Met Leu Gln Arg
 1               5
```

<210> 9
<211> 3
<212> PRT
<213> Homo sapiens

<400> 9

```
Gln Met Arg
 1
```

<210> 10
<211> 19
<212> PRT
<213> Homo sapiens

<400> 10

Glu Met Glu Glu Asn Phe Ala Val Glu Ala Ala Asn Tyr Gln Asp Thr
1                   5                   10                  15

Ile Gly Arg


<210> 11
<211> 9
<212> PRT
<213> Homo sapiens


<400> 11


Leu Gln Asp Glu Ile Gln Asn Met Lys
1                   5


<210> 12
<211> 5
<212> PRT
<213> Homo sapiens


<400> 12


Glu Glu Met Ala Arg
1                   5


<210> 13
<211> 11
<212> PRT
<213> Homo sapiens


<400> 13


Met Ala Leu Asp Ile Glu Ile Ala Thr Tyr Arg
1                   5                   10


<210> 14
<211> 466
<212> PRT
<213> Homo sapiens


<400> 14

```
Met Ser Thr Arg Ser Val Ser Ser Ser Ser Tyr Arg Arg Met Phe Gly
 1               5                 10                15

Gly Pro Gly Thr Ala Ser Arg Pro Ser Ser Ser Arg Ser Tyr Val Thr
             20                 25                 30

Thr Ser Thr Arg Thr Tyr Ser Leu Gly Ser Ala Leu Arg Pro Ser Thr
         35                 40                 45

Ser Arg Ser Leu Tyr Ala Ser Ser Pro Gly Gly Val Tyr Ala Thr Arg
     50                 55                 60
```

```
Ser Ser Ala Val Arg Leu Arg Ser Ser Val Pro Gly Val Arg Leu Leu
65              70                  75                  80

Gln Asp Ser Val Asp Phe Ser Leu Ala Asp Ala Ile Asn Thr Glu Phe
                85                  90                  95

Lys Asn Thr Arg Thr Asn Glu Lys Val Glu Leu Gln Glu Leu Asn Asp
            100                 105                 110

Arg Phe Ala Asn Tyr Ile Asp Lys Val Arg Phe Leu Glu Gln Gln Asn
            115                 120                 125

Lys Ile Leu Leu Ala Glu Leu Glu Gln Leu Lys Gly Gln Gly Lys Ser
        130                 135                 140

Arg Leu Gly Asp Leu Tyr Glu Glu Glu Met Arg Glu Leu Arg Arg Gln
145                 150                 155                 160

Val Asp Gln Leu Thr Asn Asp Lys Ala Arg Val Glu Val Glu Arg Asp
            165                 170                 175

Asn Leu Ala Glu Asp Ile Met Arg Leu Arg Glu Lys Leu Gln Glu Glu
            180                 185                 190

Met Leu Gln Arg Glu Glu Ala Glu Asn Thr Leu Gln Ser Phe Arg Gln
        195                 200                 205

Asp Val Asp Asn Ala Ser Leu Ala Arg Leu Asp Leu Glu Arg Lys Val
    210                 215                 220

Glu Ser Leu Gln Glu Glu Ile Ala Phe Leu Lys Lys Leu His Glu Glu
225                 230                 235                 240

Glu Ile Gln Glu Leu Gln Ala Gln Ile Gln Glu Gln His Val Gln Ile
            245                 250                 255

Asp Val Asp Val Ser Lys Pro Asp Leu Thr Ala Ala Leu Arg Asp Val
            260                 265                 270

Arg Gln Gln Tyr Glu Ser Val Ala Ala Lys Asn Leu Gln Glu Ala Glu
        275                 280                 285

Glu Trp Tyr Lys Ser Lys Phe Ala Asp Leu Ser Glu Ala Ala Asn Arg
    290                 295                 300

Asn Asn Asp Ala Leu Arg Gln Ala Lys Gln Glu Ser Thr Glu Tyr Arg
305                 310                 315                 320

Arg Gln Val Gln Ser Leu Thr Cys Glu Val Asp Ala Leu Lys Gly Thr
            325                 330                     335

Asn Glu Ser Leu Glu Arg Gln Met Arg Glu Met Glu Glu Asn Phe Ala
        340                 345                 350

Val Glu Ala Ala Asn Tyr Gln Asp Thr Ile Gly Arg Leu Gln Asp Glu
        355                 360                 365

Ile Gln Asn Met Lys Glu Glu Met Ala Arg His Leu Arg Glu Tyr Gln
    370                 375                 380
```

73

```
Asp Leu Leu Asn Val Lys Met Ala Leu Asp Ile Glu Ile Ala Thr Tyr
385                 390             395                 400

Arg Lys Leu Leu Glu Gly Glu Glu Ser Arg Ile Ser Leu Pro Leu Pro
                405             410             415

Asn Phe Ser Ser Leu Asn Leu Arg Glu Thr Asn Leu Asp Ser Leu Pro
            420             425             430

Leu Val Asp Thr His Ser Lys Arg Thr Phe Leu Ile Lys Thr Val Glu
            435             440             445

Thr Arg Asp Gly Gln Val Ile Asn Glu Thr Ser Gln His His Asp Asp
    450             455             460

Leu Glu
465
```

<210> 15
<211> 19
<212> PRT
<213> Homo sapiens

<400> 15

```
Leu Gln Glu Glu Met Leu Gln Arg Glu Glu Ala Glu Asn Thr Leu Gln
1               5               10                  15

Ser Phe Arg
```

<210> 16
<211> 22
<212> PRT
<213> Homo sapiens

<400> 16

```
Gln Met Arg Glu Met Glu Glu Asn Phe Ala Val Glu Ala Ala Asn Tyr
1               5               10                  15

Gln Asp Thr Ile Gly Arg
                20
```

<210> 17
<211> 23
<212> PRT
<213> Homo sapiens

<400> 17

```
Met Phe Gly Gly Pro Gly Thr Ala Ser Arg Pro Ser Ser Ser Arg Ser
 1               5                  10                  15

Tyr Val Thr Thr Ser Thr Arg
                20
```

<210> 18
<211> 12
<212> PRT
<213> Homo sapiens

<400> 18

```
Ser Arg Leu Gly Asp Leu Tyr Glu Glu Glu Met Arg
 1               5                  10
```

<210> 19
<211> 14
<212> PRT
<213> Homo sapiens

<400> 19

```
Val Glu Val Glu Arg Asp Asn Leu Ala Glu Asp Ile Met Arg
 1               5                  10
```

<210> 20
<211> 20
<212> PRT
<213> Homo sapiens

<400> 20

```
Glu Tyr Gln Asp Leu Leu Asn Val Lys Met Ala Leu Asp Ile Glu Ile
 1               5                  10                  15

Ala Thr Tyr Arg
            20
```

<210> 21
<211> 16
<212> PRT
<213> Homo sapiens

<400> 21

```
Arg Met Phe Gly Gly Pro Gly Thr Ala Ser Arg Pro Ser Ser Ser Arg
 1               5                  10                  15
```

<210> 22
<211> 28
<212> PRT
<213> Homo sapiens

<400> 22

Glu Met Glu Glu Asn Phe Ala Val Glu Ala Ala Asn Tyr Gln Asp Thr
1               5               10                  15

Ile Gly Arg Leu Gln Asp Glu Ile Gln Asn Met Lys
        20              25


<210> 23
<211> 12
<212> PRT
<213> Homo sapiens

<400> 23

Met Ser Thr Arg Ser Val Ser Ser Ser Ser Tyr Arg
1               5                   10


<210> 24
<211> 11
<212> PRT
<213> Homo sapiens

<400> 24

Gly Thr Asn Glu Ser Leu Glu Arg Gln Met Arg
1               5                   10


1


1